# EUROPEAN PATENT SPECIFICATION

(11) **EP 1 296 998 B1**
(45) Date of publication and mention of the grant of the patent: **21.09.2011**
(21) Application number: 01937697.9
(22) Date of filing: 23.05.2001
(51) Int. Cl.: C07H 21/00, C12N 15/11, C12N 15/63, C12N 15/85, C12Q 1/68

(54) **HCV VARIANTS**
VARIANTEN DES HEPATITISVIRUS
VARIANTS DU VIRUS DE L'HEPATITE C

(30) Priority: 23.05.2000 US 576989
(43) Date of publication of application: 02.04.2003
(73) Proprietor: Washington University, St. Louis, MO 63130 (US)
(72) Inventor: RICE, Charles M., III, University City, MO 63130 (US); BLIGHT, Keril, J., St. Louis, MO 63108 (US)
(74) Representative: Brasnett, Adrian Hugh
(86) International application number: PCT/US2001/016822
(87) International publication number: WO 2001/089364

(56) References cited:
- LOHMANN V ET AL: "REPLICATION OF SUBGENOMIC HEPATITIS C VIRUS RNAS IN A HEPATOMA CELLLINE" SCIENCE, AMERICAN ASSOCIATION FOR THE ADVANCEMENT OF SCIENCE, US, WASHINGTON, DC, vol. 285, 2 July 1999 (1999-07-02), pages 110-113, XP000960693 ISSN: 0036-8075
- BLIGHT ET AL.: 'Efficient initiation of HCV RNA replication in cell culture' SCIENCE vol. 290, 08 December 2000, pages 1972 - 1974, XP002951271

## Description

### (1) Field of the Invention

The invention relates to materials and methodologies relating to the production and use of hepatitis C virus (HCV) variants. More specifically, HCV variants are provided that are useful for diagnostic, therapeutic, vaccines and other uses.

### (2) Description of the Related Art

### Brief general overview of hepatitis C virus

After the development of diagnostic tests for hepatitis A virus and hepatitis B virus, an additional agent, which could be experimentally transmitted to chimpanzees [Alter et al., Lancet 1, 459-463 (1978); Hollinger et al., Intervirology 10, 60-68 (1978); Tabor et al., Lancet 1, 463-466 (1978)], became recognized as the major cause of transfusion-acquired hepatitis. cDNA clones corresponding to the causative non-A non-B (NANB) hepatitis agent, called hepatitis C virus (HCV), were reported in 1989 [Choo et al., Science 244, 359-362 (1989)]. This breakthrough has led to rapid advances in diagnostics, and in our understanding of the epidemiology, pathogenesis and molecular virology of HCV (For review, see Houghton et al., Curr Stud Hematol Blood Transfus 61, 1-11 (1994); Houghton (1996), pp. 1035-1058 in FIELDS VIROLOGY, Fields et al., Eds., Raven Press, Philadelphia; Major et al., Hepatology 25, 1527-1538 (1997); Reed and Rice, pp. 1-37 in HEPATITIS C VIRUS, Reesink, Ed., Karger, Basel; Hagedorn and Rice (1999), THE HEPATITIS C VIRUSES, Springer, Berlin). Evidence of HCV infection is found throughout the world, and the prevalence of HCV-specific antibodies ranges from 0.4-2% in most countries to more than 14% in Egypt [Hibbs et al., J. Inf. Dis. 168, 789-790 (1993)]. Besides transmission via blood or blood products, or less frequently by sexual and congenital routes, sporadic cases, not associated with known risk factors, occur and account for more than 40% of HCV cases [Alter et al., J. Am. Med. Assoc. 264, 2231-2235 (1990); Mast and Alter, Semin. Virol. 4, 273-283 (1993)]. Infections are usually chronic [Alter et al., N. Eng. J. Med. 327, 1899-1905 (1992)], and clinical outcomes range from an inapparent carrier state to acute hepatitis, chronic active hepatitis, and cirrhosis which is strongly associated with the development of hepatocellular carcinoma.

Although interferon (IFN)-α has been shown to be useful for the treatment of a minority of patients with chronic HCV infections [Davis et al., N. Engl. J. Med. 321, 1501-1506 (1989); DiBisceglie et al., New Engl. J. Med. 321, 1506-1510 (1989)] and subunit vaccines show some promise in the chimpanzee model [Choo et al., Proc. Natl. Acad. Sci. USA 91, 1294-1298 (1994)], future efforts are needed to develop more effective therapies and vaccines (See, e.g., Tsambiras et al., 1999, Hepatitis C: Hope on the Horizon, Hepatitis C Symposium of 37th Annual Meeting of the Infectious Diseases Society of America, reviewed at http://www.medscape.com/medscape/cno/1999/IDSA/Story.cfm?story_id=913). The considerable diversity observed among different HCV isolates [for review, see Bukh et al., Sem. Liver Dis. 15,41-63 (1995); Fanning et al., 2000, Medscape Gastroenterology 2:mgi6558.fann], the emergence of genetic variants in chronically infected individuals [Enomoto et al., J. Hepatol. 17, 415-416 (1993); Hijikata et al., Biochem. Biophys. Res. Comm. 175, 220-228 (1991); Kato et al., Biochem. Biophys. Res. Comm. 189, 119-127 (1992); Kato et al., J. Virol. 67, 3923-3930 (1993); Kurosaki et al., Hepatology 18, 1293-1299 (1993); Lesniewski et al., J. Med. Virol. 40, 150-156 (1993); Ogata et al., Proc. Natl. Acad. Sci. USA 88, 3392-3396 (1991); Weiner et al., Virology 180, 842-848 (1991); Weiner et al., Proc. Natl. Acad. Sci. USA 89, 3468-3472 (1992)], and the lack of protective immunity elicited after HCV infection [Farci et al., Science 258, 135-140 (1992); Prince et al., J. Infect. Dis. 165, 438-443 (1992)] present major challenges towards these goals.

### Molecular Biology of HCV

*Classification.* Based on its genome structure and virion properties, HCV has been classified as a separate genus in the flavivirus family, which includes two other genera: the flaviviruses (*e.g*., yellow fever (YF) virus) and the animal pestiviruses (*e.g*., bovine viral diarrhea virus (BVDV) and classical swine fever virus (CSFV)) [Francki et al., Arch. Virol. Suppl. 2, 223 (1991)]. All members of this family have enveloped virions that contain a positive-strand RNA genome encoding all known virus-specific proteins via translation of a single long open reading frame (ORF).

*Structure and physical properties of the virion.* Studies on the structure and physical properties of the HCV virion have been hampered by the lack of a cell culture system able to support efficient virus replication and the typically low titers of infectious virus present in serum. The size of infectious virus, based on filtration experiments, is between 30-80 nm [Bradley et al., Gastroenterology 88, 773-779 (1985); He et al., J. Infect. Dis. 156, 636-640 (1987); Yuasa et al., J. Gen. Virol. 72, 2021-2024 (1991)]. Initial measurements of the buoyant density of infectious material in sucrose yielded a range of values, with the majority present in a low density pool of < 1.1 g/ml [Bradley et al., J. Med. Virol. 34, 206-208 (1991)]. Subsequent studies have used RT/PCR to detect HCV-specific RNA as an indirect measure of potentially infectious virus present in sera from chronically infected humans or experimentally infected chimpanzees. From these studies, it has become increasingly clear that considerable heterogeneity exists between different clinical samples, and that many factors can affect the behavior of particles containing HCV RNA [Hijikata et al., J. Virol. 67, 1953-1958 (1993); Thomssen et al., Med. Microbiol. Immunol. 181, 293-300 (1992)]. Such factors include association with immunoglobulins [Hijikata *et al.,* (1993) *supra*] or low density lipoprotein [Thomssen *et al.,* 1992, *supra*; Thomssen et al., Med. Microbiol. Immunol. 182, 329-334 (1993)]. In highly infectious acute phase chimpanzee serum, HCV-specific RNA is usually detected in fractions of low buoyant density (1.03-1.1 g/ml) [Carrick et al., J. Virol. Meth. 39, 279-289 (1992); Hijikata *et al.,* (1993) *supra*]. In other samples, the presence of HCV antibodies and formation of immune complexes correlate with particles of higher density and lower infectivity [Hijikata *et al.,* (1993) *supra*]*.* Treatment of particles with chloroform, which destroys infectivity [Bradley et al., J. Infect. Dis. 148,254-265 (1983); Feinstone et al., Infect. Immun. 41, 816-821 (1983)], or with nonionic detergents, produced RNA containing particles of higher density (1.17-1.25 g/ml) believed to represent HCV nucleocapsids [Hijikata *et al.,* (1993) *supra*; Kanto et al., Hepatology 19, 296-302 (1994); Miyamoto et al., J. Gen Virol. 73,715-718 (1992)].

There have been reports of negative-sense HCV-specific RNAs in sera and plasma [see Fong et al., Journal of Clinical Investigation 88:1058-60 (1991)]. However, it seems unlikely that such RNAs are essential components of infectious particles since some sera with high infectivity can have low or undetectable levels of negative-strand RNA [Shimizu et al., Proc. Natl. Acad. Sci. USA 90: 6037-6041 (1993)].

The virion protein composition has not been rigorously determined, but HCV structural proteins include a basic C protein and two membrane glycoproteins, E1 and E2.

*HCV replication.* Early events in HCV replication are poorly understood. A hepatocyte receptor may be CD81, which binds the E2 envelope glycoprotein (Peleri et al., 1998, Science 282:938-41). The association of some HCV particles with beta-lipoprotein and immunoglobulins raises the possibility that these host molecules may modulate virus uptake and tissue tropism.

Studies examining HCV replication have been largely restricted to human patients or experimentally inoculated chimpanzees. In the chimpanzee model, HCV RNA is detected in the serum as early as three days post-inoculation and persists through the peak of serum alanine aminotransferase (ALT) levels (an indicator of liver damage) [Shimizu et al., Proc. Natl. Acad. Sci. USA 87: 6441-6444 (1990)]. The onset of viremia is followed by the appearance of indirect hallmarks of HCV infection of the liver. These include the appearance of a cytoplasmic antigen [Shimizu *et al.,* (1990) *supra*] and ultrastructural changes in hepatocytes such as the formation of microtubular aggregates for which HCV previously was referred to as the chloroform-sensitive "tubule forming agent" or "TFA" [reviewed by Bradley, Prog. Med. Virol. 37: 101-135 (1990)]. As shown by the appearance of viral antigens [Blight et al., Amer. J. Path. 143: 1568-1573 (1993); Hiramatsu et al., Hepatology 16: 306-311 (1992); Krawczynski et al., Gastroenterology 103: 622-629 (1992); Yamada et al., Digest. Dis. Sci. 38: 882-887 (1993)] and the detection of positive and negative sense RNAs [Fong *et al.,* (1991) *supra*; Gunji et al., Arch. Virol. 134: 293-302 (1994); Haruna et al., J. Hepatol. 18: 96-100 (1993); Lamas et al., J. Hepatol. 16: 219-223 (1992); Nouri Aria et al., J. Clin. Inves. 91; 2226-34 (1993); Sherker et al., J. Med. Virol. 39: 91-96 (1993); Takehara et al., Hepatology 15: 387-390 (1992); Tanaka et al., Liver 13: 203-208 (1993)], hepatocytes appear to be a major site of HCV replication, particularly during acute infection [Negro et al., Proc. Natl. Acad. Sci. USA 89: 2247-2251 (1992)]. In later stages of HCV infection the appearance of HCV-specific antibodies, the persistence or resolution of viremia, and the severity of liver disease, vary greatly both in the chimpanzee model and in human patients (Fanning et al., *supra*). Although some liver damage may occur as a direct consequence of HCV infection and cytopathogenicity, the emerging consensus is that host immune responses, in particular virus-specific cytotoxic T lymphocytes, may play a more dominant role in mediating cellular damage.

It has been speculated that HCV may also replicate in extra-hepatic reservoir(s). In some cases, RT/PCR or *in situ* hybridization has shown an association of HCV RNA with peripheral blood mononuclear cells including T-cells, B-cells, and monocytes [reviewed in Blight and Gowans, Viral Hepatitis Rev. 1: 143-155 (1995)]. Such tissue tropism could be relevant to the establishment of chronic infections and might also play a role in the association between HCV infection and certain immunological abnormalities such as mixed cryoglobulinemia [reviewed by Ferri et al., Eur. J. Clin. Invest. 23: 399-405 (1993)], glomerulonephritis, and rare non-Hodgkin's B-lymphomas [Ferri *et al.,* (1993) *supra*; Kagawa et al., Lancet 341: 316-317 (1993)]. However, the detection of circulating negative strand RNA in serum, the difficulty in obtaining truly strand-specific RT/PCR [Gunji *et al*., (1994) *supra*], and the low numbers of apparently infected cells have made it difficult to obtain unambiguous evidence for replication in these tissues in vivo.

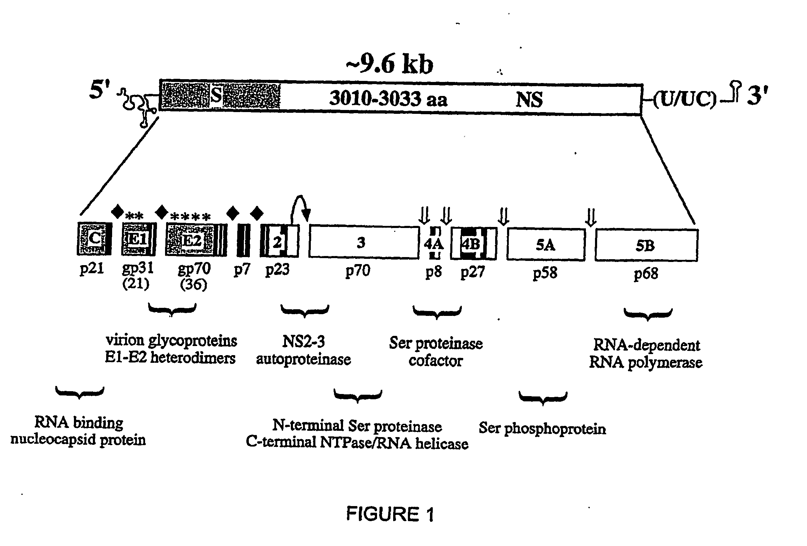

*Genome structure.* Full-length or nearly full-length genome sequences of numerous HCV isolates have been reported [see, e.g., Lin et al., J. Virol. 68: 5063-5073 (1994a); Okamoto et al., J. Gen. Virol. 75: 629-635 (1994); Sakamoto et al., J. Gen. Virol. 75: 1761-1768 (1994); Trowbridge et al, Arch Virol. 143:501-511 (1998); Chamberlain et al, J. Gen. Virol. 78:1341-1347 (1997); and citations within Davis, Am. J. Med. 27:21S-26S]. HCV genome RNAs are ~9.6 kilobases (kb) in length (Figure 1) and consist of a 5' nontranslated region (5' NTR), a polyprotein coding region consisting of a single long open reading frame (ORF), and a 3' NTR. The 5' NTR is 341-344 bases long and highly conserved. The length of the long ORF varies slightly among isolates, encoding polyproteins of about 3010 to about 3033 amino acids.

The 3' NTR can be divided into three domains. The first (most 5') domain shows considerable diversity both in composition and length (28-42 bases). Recent work by Yanagi et al. [Proc. Natl. Acad. Sci. USA 96:2291-2295(1999)] demonstrate that this region is not necessary for virus replication. The second domain is consists of a variable length polypyrimidine region of poly(A) (in at least HCV-1, type 1a [Han et al., Proc. Natl. Acad. Sci. USA 88:1711-1715 (1991)]) or poly(U-UC) (see Chen et al., Virology 188:102-113 (1992); Okamoto et al., J. Gen. Virol. 72:2697-2704 (1991); Tokita et al., J. Gen. Virol. 66:1476-83 (1994)]. The third domain, at the extreme 3' end of the genome, is a highly conserved, novel RNA element of about 98 nucleotides, which is necessary for efficient initiation of viral RNA replication [see, e.g., U.S. Patent No. 5,874,565 and U.S. Patent No.6127116; Kolykhalov et al., J. Virol. 70: 3363-3371 (1996); Tanaka et al., Biochem. Biophys. Res. Comm. 215: 744-749 (1996); Tanaka et al., J. Virol. 70:3307-12 (1996); Yamada et al., Virology 223:255-261 (1996); Cheng et al. J. Virol. 73:7044-7049]. This domain and the polypyrimidine regions appear to be critical for infectivity in vivo [Yanagi et al., Proc. Natl. Acad Sci. USA 96:2291-2295 (1999)].

*Translation and proteolytic processing.* The highly conserved 5' NTR sequence contains multiple short AUG-initiated ORFs and shows significant homology with the 5' NTR region of pestiviruses [Bukh et al., Proc. Natl. Acad. Sci. USA 89: 4942-4946 (1992); Han *et al.,* (1991) *supra*]. A series of stem-loop structures that interact with host factors are present. These structures interact with host factors to initiate polyprotein synthesis through an internal ribosome entry site (IRES) allowing efficient translation initiation at the first AUG of the long ORF [Honda et al., J. Virol 73:4941-4951 (1999); Tang et al., J. Virol. 73:2359-2364(1999); Psaridi et al., FEBS Lett. 453:49-53 (1999)]. Some of the predicted features of the HCV and pestivirus IRES elements are similar to one another [Brown *et al.,* (1992) *supra*]. The ability of this element to function as an IRES suggests that HCV genome RNAs may lack a 5' cap structure.

The organization and processing of the HCV polyprotein (Figure 1) appears to be most similar to that of the pestiviruses. At least 10 polypeptides have been identified and the order of these cleavage products in the polyprotein is NH2-C-E1-E2-p7-NS2-NS3-NS4A-NS4B-NS5A-NS5B-COOH. As shown in Figure 1, proteolytic processing is mediated by host signal peptidase and two HCV-encoded proteinases, the NS2-3 autoproteinase and the NS3-4A serine proteinase [see Rice, In "Fields Virology" (B. N. Fields, D. M. Knipe and P. M. Howley, Eds.), Vol. pp. 931-960. Raven Press, New York (1996); Shimotohno et al., J. Hepatol. 22: 87-92 (1995) for reviews]. C is a basic protein that serves as the viral core or capsid protein; E1 and E2 are virion envelope glycoproteins; p7 is a hydrophobic protein of unknown function that is inefficiently cleaved from the E2 glycoprotein [Lin *et al.,* (1994a) *supra*; Mizushima et al., J. Virol. 68: 6215-6222 (1994); Selby et al., Virology 204: 114-122 (1994)]. NS2-NS5B are nonstructural (NS) proteins which function in viral RNA replication complexes. Their functions have been identified as follows: NS2 is a metalloprotease; NS3 is a protease/helicase that contains motifs characteristic of RNA helicases and that has been shown to possess an RNA-stimulated NTPase activity [Suzich et al., J. Virol. 67, 6152-6158 (1993)]; NS4A is a co-factor for NS3; NS4B is of unknown function; NS5A interacts with cellular factors to transcriptionally modulate cellular genes and promote cell growth [Ghosh et al., J. Biol. Chem. 275:7184-7188] and provide IFNα resistance; and NS5B is a replicase that contains the GDD motif characteristic of the RNA-dependent RNA polymerases of other positive-strand RNA viruses.

*Virion assembly and release.* This process has not been examined directly, but the lack of complex glycans, the ER localization of expressed HCV glycoproteins [Dubuisson et al., J. Virol. 68: 6147-6160 (1994); Ralston et al., J. Virol. 67: 6753-6761 (1993)] and the absence of these proteins on the cell surface [Dubuisson *et al.,* (1994) *supra*; Spaete et al., Virology 188: 819-830 (1992)] suggest that initial virion morphogenesis may occur by budding into intracellular vesicles. Thus far, efficient particle formation and release has not been observed in transient expression assays, suggesting that essential viral or host factors are absent or blocked. HCV virion formation and release may be inefficient, since a substantial fraction of the virus remains cell-associated, as found for the pestiviruses. Extracellular HCV particles partially purified from human plasma contain complex N-linked glycans, although these carbohydrate moieties were not shown to be specifically associated with E1 or E2 [Sato et al., Virology 196: 354-357 (1993)]. Complex glycans associated with glycoproteins on released virions would suggest transit through the trans-Golgi and movement of virions through the host secretory pathway. If this is correct, intracellular sequestration of HCV glycoproteins and virion formation might then play a role in the establishment of chronic infections by minimizing immune surveillance and preventing lysis of virus-infected cells via antibody and complement.

*Genetic variability.* As for all positive-strand RNA viruses, the RNA-dependent RNA polymerase of HCV (NS5B) is believed to lack a 3'-5' exonuclease proof reading activity for removal of misincorporated bases. Replication is therefore error-prone, leading to a "quasi-species" virus population consisting of a large number of variants [Martell et al., J. Virol. 66: 3225-3229 (1992); Martell et al., J. Virol. 68: 3425-3436 (1994)]. This variability is apparent at multiple levels. First, in a chronically infected individual, changes in the virus population occur over time [Ogata *et al.,* (1991) *supra*; Okamoto et al., Virology 190: 894-899 (1992)]; and these changes may have important consequences for disease. A particularly interesting example is the N-terminal 30 residue segment of the E2 glycoprotein, which exhibits a much higher degree of variability than the rest of the polyprotein [for examples, see Higashi et al., Virology 197, 659-668. 1993; Hijikata *et al.,* (1991) *supra*; *Weiner et al.,* (1991) *supra*]. There is accumulating evidence that this hypervariable region, called hypervariable region 1 (HVR1), perhaps analogous to the V3 domain of HIV-1 gp120, may be under immune selection by circulating HCV-specific antibodies [Kato *et al.,* (1993) *supra*; Taniguchi et al., Virology 195: 297-301 (1993); Weiner *et al.,* (1992) *supra.* In this model, antibodies directed against this portion of E2 may contribute to virus neutralization and thus drive the selection of variants with substitutions that permit escape from neutralization. This plasticity suggests that a specific amino acid sequence in the E2 hypervariable region is not essential for other functions of the protein such as virion attachment, penetration, or assembly. Genetic evolution of HVR1 within the first 4 months of infection has been correlated with the ability of a particular strain of the virus to cause chronic infection [Farci et al., Science 288:339-344 (2000)].

Genetic variability may also contribute to the spectrum of different responses observed after IFN-α treatment of chronically infected patients. Diminished serum ALT levels and improved liver histology, which usually correlates with a decrease in the level of circulating HCV RNA, is seen in ~40% of those treated [Greiser-Wilke et al., J. Gen. Virol. 72: 2015-2019 (1991)]. After treatment, approximately 70% of the responders relapse. In some cases, after a transient loss of circulating viral RNA, renewed viremia is observed during or after the course of treatment. While this might suggest the existence or generation of IFN-resistant HCV genotypes or variants, further work is needed to determine the relative contributions of virus genotype and host-specific differences in immune response.

Sequence comparisons of different HCV isolates around the world have also revealed enormous genetic diversity [reviewed in Bukh *et al.,* (1995) *supra*]. Because of the lack of biologically relevant serological assays such as cross-neutralization tests, HCV types (designated by numbers), subtypes (designated by letters), and isolates are currently grouped on the basis of nucleotide or amino acid sequence similarity. Worldwide, HCV has been classified into six major genotypes and more than 50 subtypes [Purcell, Hepatology 26:11 S-14S (1997)]. Those of greatest importance in the U.S. are genotype 1, subtypes 1a and 1b (see below and Bukh *et al.,* (1995) *supra* for a discussion of genotype prevalence and distribution). Amino acid sequence similarity between the most divergent genotypes can be a little as ~50%, depending upon the protein being compared. This diversity has important biological implications, particularly for diagnosis, vaccine design, and therapy.

*HCV RNA replication.* By analogy with other flaviviruses, replication of the positive-sense HCV virion RNA is thought to occur via a minus-strand intermediate. This strategy can be described briefly as follows: (i) uncoating of the incoming virus particle releases the genomic plus-strand, which is translated to produce a single long polyprotein that is probably processed co- and post-translationally to produce individual structural and nonstructural proteins; (ii) the nonstructural proteins form a replication complex that utilizes the virion RNA as template for the synthesis of minus strands; (iii) these minus strands in turn serve as templates for synthesis of plus strands, which can be used for additional translation of viral protein, minus strand synthesis, or packaging into progeny virions. Very few details about HCV replication process are available, due to the lack of a good experimental system for virus propagation. Detailed analyses of authentic HCV replication and other steps in the viral life cycle would be greatly facilitated by the development of an efficient system for HCV replication in cell culture.

Many attempts have been made to infect cultured cells with serum collected from HCV-infected individuals, and low levels of replication have been reported in a number of cells types infected by this method, including B-cell [Bertolini et al., Res. Virol. 144: 281-285 (1993); Nakajima et al., J. Virol. 70: 9925-9 (1996); Valli et al., Res. Virol. 146:285-288 (1995)]. T-cell (Kato et al., Biochem. Biophys. Res. Commun. 206:863-9 (1996); Mizutani et al., Biochem. Biophys. Res. Comm. 227:822-826; Mizutani et al., J. Virol. 70: 7219-7223 (1996); Nakajima *et al.,* (1996) *supra*; Shimizu and Yoshikura, J Virol, 68: 8406-8408 (1994); Shimizu et al., Proc. Natl. Acad. Sci USA, 89: 5477-5481 (1992); Shimizu et al., Proc. Natl. Acad. Sci. USA, 90: 6037-6041 (1993)], and hepatocyte [Kato et al., Jpn. J. Cancer Res., 87: 787-92 (1996); Tagawa, J. Gastoenterol. and Hepatol., 10: 523-527 (1995)] cell lines, as well as peripheral blood monocular cells (PBMCs) [Cribier et al., J. Gen. Virol., 76: 2485-2491 (1995)], and primary cultures of human fetal hepatocytes [Carloni et al., Arch. Virol. Suppl. 8: 31-39 (1993); Cnbier et al., (1995) *supra*; Iacovacci et al., Res. Virol., 144: 275-279 (1993)] or hepatocytes from adult chimpanzees [Lanford et al., *Virology 202*: 606-14 (1994)]. HCV replication has also been detected in primary hepatocytes derived from a human HCV patient that were infected with the virus in vivo prior to cultivation [Ito et al., J. Gen. Virol. 77: 1043-1054 (1996)] and in the human hepatoma cell line Huh7 following transfection with RNA transcribed in vitro from an HCV-1 cDNA clone [Yoo et al., J. Virol., 69: 32-38 (1995)]. The reported observation of replication in cells transfected with RNA derived from the HCV-1 clone was puzzling, since this clone lacks the required terminal 3'NTR sequence downstream of the homopolymer tract (see below), and because a number of unusual observations were reported (see the background section of Application U.S. Patent No.6127116. The most well-characterized cell-culture systems for HCV replication utilize a B-cell line (Daudi) or T-cell lines persistently infected with retroviruses (HPB-Ma or MT-2) [Kato et al., (1995) *supra;* Mizutani et al., Biochem Biophys Res. Comm., 227: 822-826 (1996a); Mizutani et al., (1996) *supra*; Nakajima et al., (1996) *supra*; Shimizu and Yoshikura, (1994) *supra*]; Shimizu, Proc. Natl. Acad. Sci. USA, 90: 6037-6041 (1993)]. HPBMa is infected with an amphotropic murine leukemia virus pseudotype of murine sarcoma virus, while MT-2 is infected with human T-cell lymphotropic virus type I (HTLV-1). Clones (HPBMa 10-2 and MT-2C) that support HCV replication more efficiently than the uncloned population have been isolated for the two T-cell lines HPBMa and MT-2 [Mizutani et al. J. Virol. (1996) *supra*; Shimizu et al., (1993) *supra*]*.* However, the maximum levels of RNA replication obtained in these lines or in the Daudi lines after degradation of the input RNA is still only about 5 x 10⁴ RNA molecules per 10⁶ cells (Mizutani et al., (1996) *supra*; Mizutani et al., (1996) *supra*] or 10⁴ RNA molecules per ml of culture medium [Nakajima et al., (1996) *supra*]*.* Although the level of replication is low, long-term infections of up to 198 days in one system [Mizutani et al., Biochem. Biophys. Res. Comm. 227: 822-826 (1996a)] and more than a year in another system [Nakajima et al., (1996) *supra*] have been documented, and infectious virus production has been demonstrated by serial cell-free or cell-mediated passage of the virus to naive cells.

However, efficient replication of an HCV clone comprising the essential conserved terminal 3' NTR sequence had not been observed until the work described in U.S. Patent No.6127116 also reported in Kolykhalov et al., Science 277:570 (1997), which describes an infectious clone of an isolate of the H strain (type 1a). HCV clones of other subtypes are now known. See, e.g., Yanagi et al., Virology 262:250-263 (1999) and Yanagi et al., virology 244:161-172 (1998). While RNA transcripts of these clones are able to infect chimpanzees, cell cultures with these clones only support replication of the virus poorly if at all.

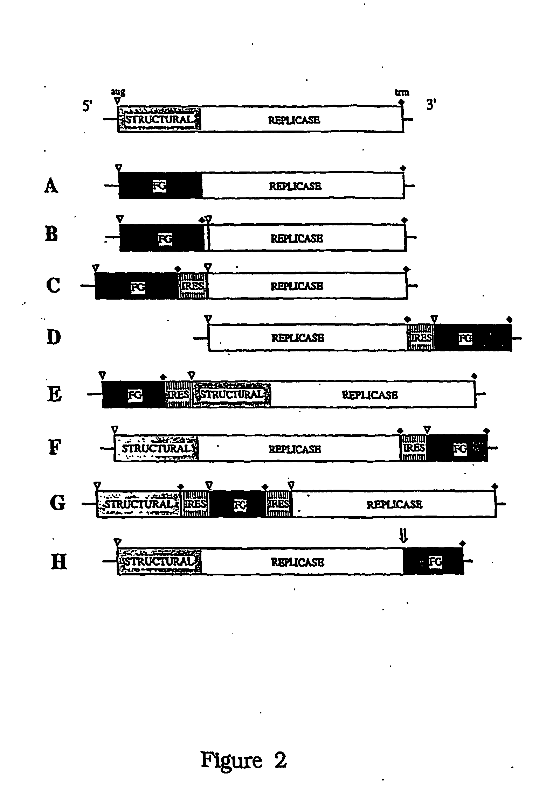

As described in U.S. Patent No.6127116 (see, e.g., Figure 2 therein) many variations of a functional clone are possible. These include full length or partial sequences where a foreign gene is inserted. The foreign gene can include, e.g., a reporter gene such as β-galactosidase or luciferase, or a gene encoding a selectable marker such as *neo, DHFR,* or *tk.* In a specific example disclosed therein, the neo gene is operably linked to an internal ribosome entry site (IRES), in order for infected cells to be selected by neomycin or G418 resistance. In this way, presence of replicating HCV RNA in essentially all surviving cells is assured. Additionally, the HCV polyprotein coding region of these clones can be deficient in some or all of the structural genes C, E1 and E2. Thus, replicons can be created without the production of virions. By combining the structural gene-deficient construct with a selectable marker such as *neo,* an efficiently replicating replicon system can be created that can be used to study HCV replication and for other purposes.

Examples of the replicons disclosed in U.S. Patent No.6127116 is provided in Lohmann et al., Science 285:110-113 (1999). In that work, DNA clones of HCV replicons of genotype 1, subtype 1b were constructed. Features of those replicons that are not wild-type HCV features are: a polyprotein coding region lacking the genes encoding the HCV structural proteins; an EMCV IRES immediately 5' to the polyprotein region; and a *neo* gene immediately 3' to the 5' NTR (and the HCV IRES), where the 5' end of the HCV C protein gene is fused to the 5' end of the *neo* gene. When Huh-7 cells were transfected with RNA transcripts of these clones, 6 to >60 G418-resistant colonies arose per experiment. Although the number of cells treated was not specified, about 10⁶ - 10⁷ cells are normally treated in experiments of this type. Therefore, it is believed that the transfection efficiency, as measured by G418-resistant colonies/total treated, was less than 01% in those studies.

Controls in the Lohmann et al. work included in-frame deletions of the active site of the NS5B polymerase. Although care was taken to remove template DNA from the control transcripts, several G418-resistant control colonies arose. Still, the number of G418-resistant control colonies that arose was much less than the colonies arising from the cells transfected with the replicons containing the wild-type NS5B.

When the G418-resistant colonies were subpassaged, most could not be maintained. Out of more than 303 G418-resistant colonies from non-control replicon treatments, 9 (<3%) could be subpassaged to establish stable cell lines. Replicons established in infected cell lines were sequenced. Although each replicon had a number of amino acid substitutions, the substitutions were scattered throughout the polyprotein coding region. Therefore, there were no mutations that were consistently in one area of the polyprotein coding region, and it was concluded that the establishment of the nine cell lines was not due to adaptive mutations in those replicons. This contention was experimentally tested by transfection/reconstitution experiments that did not provide evidence for adaptive changes.

Despite the advances described above, more efficient HCV-infected cell systems are needed for the production of concentrated virus stocks, structural analysis of virion components, evaluation of putative antiviral therapies including vaccines and antiviral compounds, and improved analyses of intracellular viral processes, including RNA replication. Thus, there is a need for various types of HCV clones that can be used for any of the above purposes. There is also a need to characterize HCV with respect to regions of the genome that might contribute to more efficient *in vitro or in vivo* replication and virion production.

### Summary of the Invention

Thus, a primary object of the present invention has been to provide DNA encoding non-naturally occurring HCV that is capable of replication.

A related object of the invention is to provide genomic RNA from the above DNA. Still another object of the invention is to provide attenuated HCV DNA or genomic RNA suitable for vaccine development, which can invade a cell and replicate but cannot propagate infectious virus.

Another object of the invention is to provide *in vitro* and *in vivo* models of HCV infection and RNA replication for testing anti-HCV (or antiviral) drugs, for evaluating drug resistance, and for testing attenuated HCV viral vaccines.

An additional object of the invention is to provide replicating HCV replicons. These replicons do not encode structural proteins but may encode a foreign protein such as a reporter gene or a selectable marker.

Still another object of the invention is to provide adaptive replicons, with increased 5 ability to establish replication in continuous or primary cell lines.

Briefly, therefore, the inventors have succeeded in discovering methods of creating replicating HCV variants, including variants with adaptive mutations in HCV that improve their ability to establish RNA replication in culture to create continuous cell lines. These HCV variants and the cell lines that harbor them are useful for studying replication and other 10 HCV characteristics. The cell lines are also useful for developing vaccines and for testing compounds for antiviral properties.

Thus the present invention is directed to a polynucleotide comprising a non-naturally occurring HCV sequence that is capable of productive replication in a host cell, or is capable of being transcribed into a non-naturally occurring HCV sequence 15 that is capable of productive replication in a host cell. The HCV sequence comprises, from 5' to 3' on the positive-sense nucleic acid, a functional 5' non-translated region (5'NTR); one or more protein coding regions, including at least one polyprotein coding region that is capable of replicating HCV RNA; and a functional HCV 3' non-translated region (3'NTR). wherein the polyprotein coding region comprises an NS5A gene comprising an adaptive mutation (i) encoding an amino acid sequence charge selected the group consisting of Ser (1179) to Ile, Arg (1164) to Gly, Ala (1174) to Ser, Ser (1172) to Cys, Ser (1172) Pro, of SEQ ID NO:3; or (ii) comprising a deletion of nucleotides corresponding to nucleotides 5345 to 5485 of SEQ ID NO:6 In preferred embodiments of these polynucleotides, the 5' NTR is an HCV 5' NTR, the polynucleotide comprises at least one IRES selected from the group consisting of a viral IRES, a cellular IRES, and an artificial IRES, and the polyprotein coding region is an HCV polyprotein coding region.

The above polynucleotides comprise an adaptive mutation. The adaptive mutation can be such that the polynucleotide has a transfection efficiency into mammalian cells of greater than 0.01%; more preferably greater than 0.1%; even more preferably, greater than 1%; still more preferably greater than 5%, may be about 6%. The adaptive mutations can be such that the polynucleotide is capable of replication in a non-hepatic cell, for example HeLa cells. The adaptive mutations can also cause the polynucleotide to have attenuated virulence, wherein the HCV is impaired in its ability to cause disease, establish chronic infections; trigger autoimmune responses, and transform cells.

In the above described adaptive mutants, the polyprotein region comprises an NSSA gene that is not a wild-type NS5A gene and comprises a mutation. The mutation is preferably within 50 nucleotides of an ISDR or includes the ISDR; more preferably the mutation is within 20 nt of the ISDR, or includes the ISDR. The adaptive mutations are those that encode an amino acid sequence change selected from the group consisting of Ser (1179) to Ile, Arg (1164) to Gly, Ala(1174) to Ser, Ser(1172) to Cys, and Ser(1172) to Pro of SEQ ID NO:3; or include a deletion of nucleotides 5345 to 5485 of SEQ ID NO:6.

In some embodiments of the invention polynucleotides, the HCV polyprotein coding region encodes all HCV structural and nonstructural proteins. In other embodiments, the polyprotein coding region is incapable of making infectious HCV particles, making the HCV variant a replicon. Preferably the inability to make HCV particles is due to a deletion in the structural protein coding region. Some embodiments of these replicons further comprise a foreign gene operably linked to a first IRES and the HCV polyprotein coding region operably linked to a second IRES. Preferably, the replicon comprises a genotype 1 HCV sequence, most preferably subtype 1b. Preferred foreign genes in these replicons are selectable markers or reporter genes. In other preferred replicon embodiments, the first IRES is an HCV IRES, the foreign gene is a neo gene, and the second IRES is a EMCV IRES. Examples of the above replicons include SEQ ID NO:5, SEQ ID NO:6, SEQ ID NO:22 and SEQ ID NO:25. The above replicons also preferably comprise an adaptive mutation, including any of the adaptive phenotypes previously described, including increased transfection efficiency, replication in a non-hepatic cell including HeLa cells, and attenuated virulence, and further comprising any of the adaptive mutations previously described, such as the various NS5A mutations and deletions previously described.

The polynucleotides of the present invention can be in the form of RNA or DNA. Preferred embodiments of the polynucleotides are SEQ ID NOs:5-13 and 22-25, the complements thereof, and the RNA equivalents of the sequences or their complements. In certain embodiments, the polynucleotides are capable of productive infection in a chimpanzee upon intrahepatic injection.

The present invention is also directed to expression vectors comprising DNA forms of any of the above polynucleotides, operably associated with a promoter. Additionally, the invention is directed to cells comprising the above expression vectors as well as host cells comprising any of the polynucleotides described above. The host cells are preferably mammalian cells, more preferably human cells. The host cells are preferably hepatocytes, T-cells, B-cells, or foreskin fibroblasts; most preferably hepatocytes. Certain adaptive mutants can also replicate in HeLa cells. The host cells can be within a non-human mammal capable of supporting transfection and replication of the HCV RNA, and infection when the HCV RNA encodes a virus particle. A preferred non-human mammal is a chimpanzee.

Also described are methods for identifying a cell line that is permissive for RNA replication with HCV. The method includes the steps of contacting a cell in tissue culture with an infectious amount of the above-described polynucleotides, and detecting replication of HCV variants in cells of the cell line.

Also described is a method for producing a cell line comprising replicating HCV. The method includes the steps of (a) transcribing the above-described expression vector to synthesize HCV RNA; (b) transfecting a cell with the HCV RNA; and (c) culturing the cell.

Additionally, described a vaccine. The vaccine includes any of the above-described polynucleotides, in a pharmaceutically acceptable carrier. In a related description is a method of inducing immunoprotection to HCV in a primate. The method includes administering the vaccine to the primate.

In a further description is a method of testing a compound for inhibiting HCV replication. The method includes the steps of (a) treating the above described host cells with the compound; and (b) evaluating the treated host cell for reduced replication, wherein reduced HCV replication indicates the ability of the compound to inhibit replication.

In an additional description is a method of testing a compound for inhibiting HCV infection. The method comprises treating a host cell with the compound before, during or after infecting the host cell with any of the invention polynucleotides.

In a still further description is an HCV variant that has (a) transfection efficiency greater than 0.01%, as determined by replication-dependent neomycin resistance, or (b) greater ability of initial colonies of cells transfected with the variant to survive subpassage than wild-type HCV genotype 1, subtype 1b. The HCV variant also has, from 5' to 3' on the positive-sense nucleic acid, a functional HCV 5' non-translated region (5'NTR) comprising an extreme 5'-terminal conserved sequence; an HCV polyprotein coding region; and a functional HCV 3' non-translated region (3'NTR) comprising a variable region, a polypyrimidine region, and an extreme 3'-terminal conserved sequence. Preferably the transfection efficiency is greater than 0.1%; in more preferably embodiments, greater than 1%; in still more preferably, greater than 5%. Most preferably, the transfection efficiency is about 6%.

The variants can have any of the characteristics of the polynucleotides described above and comprise the NS5A mutation or deletion described for the polynucleotides above.

Among the several advantages achieved by the present invention are the provision of polynucleotides comprising non-naturally occurring HCV sequences; the provision of HCV variants that have a transfection efficiency and ability to survive subpassage greater than HCV forms that have wild-type polyprotein coding regions; the provision of expression vectors comprising the above polynucleotides and HCV variants; the provision of cells and host cells comprising the above expression vectors, the provision of methods for identifying a cell line that is permissive for RNA replication with HCV; the provision of vaccines comprising the above polynucleotides in a pharmaceutically acceptable carrier; the provision of methods for inducing immunoprotection to HCV in a primate; and the provision of methods for testing a compound for inhibiting HCV replication.

### Brief Description of the Drawings

FIGURE 1. *HCV genome structure, polyprotein processing, and protein features.* At the top is depicted the viral genome with the structural and nonstructural protein coding regions, and the 5'and 3' NTRs, and the putative 3' secondary structure. Boxes below the genome indicate proteins generated by the proteolytic processing cascade. Putative structural proteins are indicated by shaded boxes and the nonstructural proteins by open boxes. Contiguous stretches of uncharged amino acids are shown by black bars. Asterisks denote proteins with N-linked glycans but do not necessarily indicate the position or number of sites utilized. Cleavage sites shown are for host signalase (◆), the NS2-3 proteinase (curved arrow), an the NS3-4A serine protease (⇓).
FIGURE 2. *Strategies for expression of heterologous RNAs and proteins using HCV vectors.* At the top is a diagram of the positive-polarity RNA virus HCV, which expresses mature viral proteins by translation of a single long ORF and proteolytic processing. The regions of the polyprotein encoding the structural proteins (STRUCTURAL) and the nonstructural proteins (REPLICASE) are indicated as lightly-shaded and open boxes, respectively. Below are shown a number of proposed replication-competent "replicon" expression constructs. The first four constructs (A-D) lack structural genes and would therefore require a helper system to enable packaging into infectious virions. Constructs E-G would not require helper functions for replication or packaging. Darkly shaded boxes indicate heterologous or foreign gene sequences (FG). Translation initiation (aug) and termination signals (trm) are indicated by open triangles and solid diamonds, respectively. Internal ribosomes entry sites (IRES) are shown as boxes with vertical stripes. Constructs A and H illustrate the expression of a heterologous product as an in-frame fusion with the HCV polyprotein. Such protein fusion junctions can be engineered such that processing is mediated either by host or viral proteinases (indicated by the arrow).

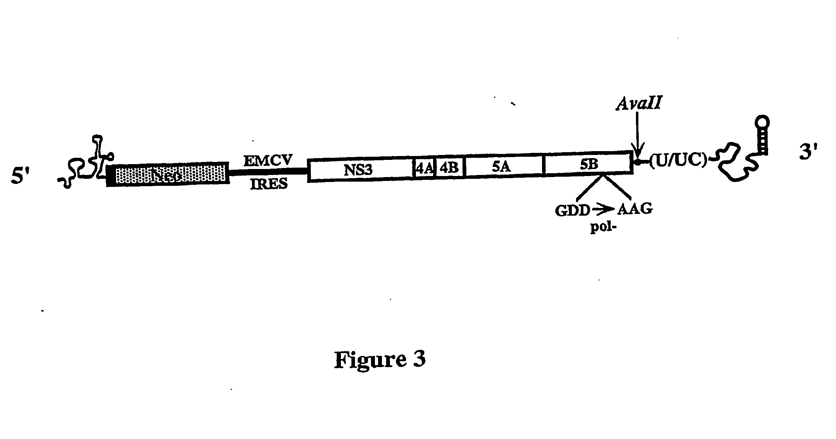

FIGURE 3. *Structure of HCVrep1bBartMan.* Two versions of this infectious replicon were constructed as described in Example 1. The first, HCVrep1bBartMan/AvaII, has a AvaII restriction site in the variable domain of the 3' NTR that is not present in the 3' NTR of wild-type HCV subtype 1b. The second variant, HCVrep1bBartMan/Δ2U's, has 32, rather than the wild-type 34, U's in the longest stretch of contiguous U's in the polypyrimidine domain of the 3' NTR. The "GDD→AGG" designation shows the inactivating mutation in the non-replicating replicons that were used as polymerase-minus controls in Example 1.

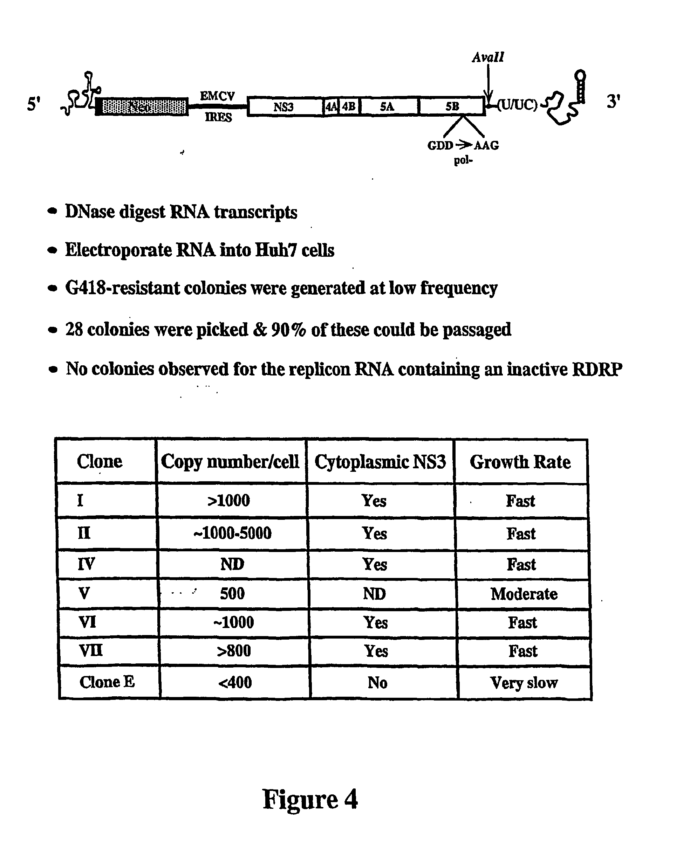

FIGURE 4. *Generation of G418-resistant cell clones.* At the top is a diagram of the HCVrep1bBartMan replicons as described in Figure 3. The middle text summarizes the steps used to isolate the adaptive mutants, which are further described in Example 1. The bottom chart summarizes several characteristics of some of the replicons isolated as described in the Example.

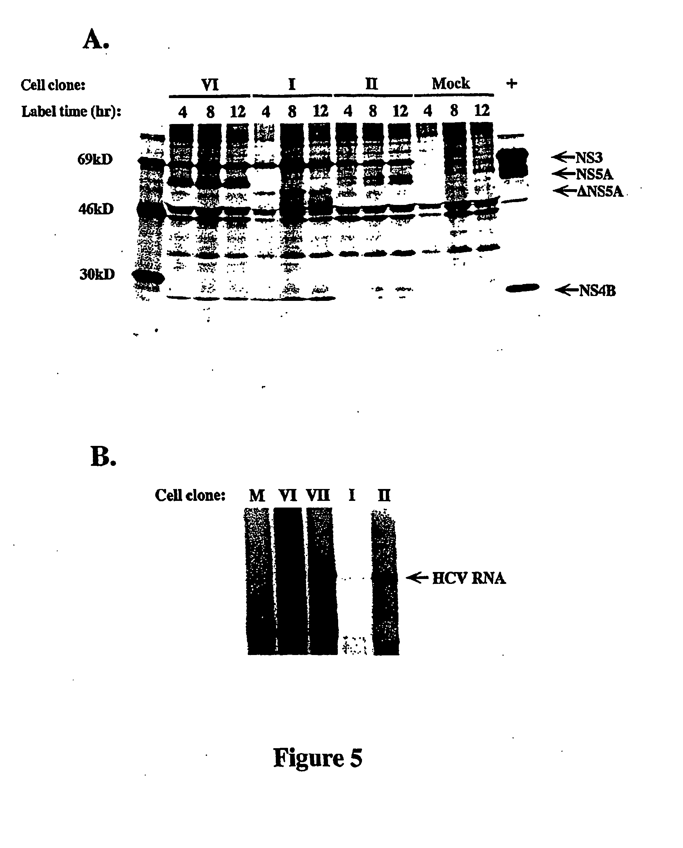

FIGURE 5. *Synthesis of HCV-specific RNA and proteins.* Figure 5A illustrates actinomycin D-resistant RNA replication of four adaptive replicons as further described in the Example. Figure 5B illustrates the immunoprecipitation of ³⁵S-labeled HCV-specific proteins of three adaptive replicons as further described in Example 1.

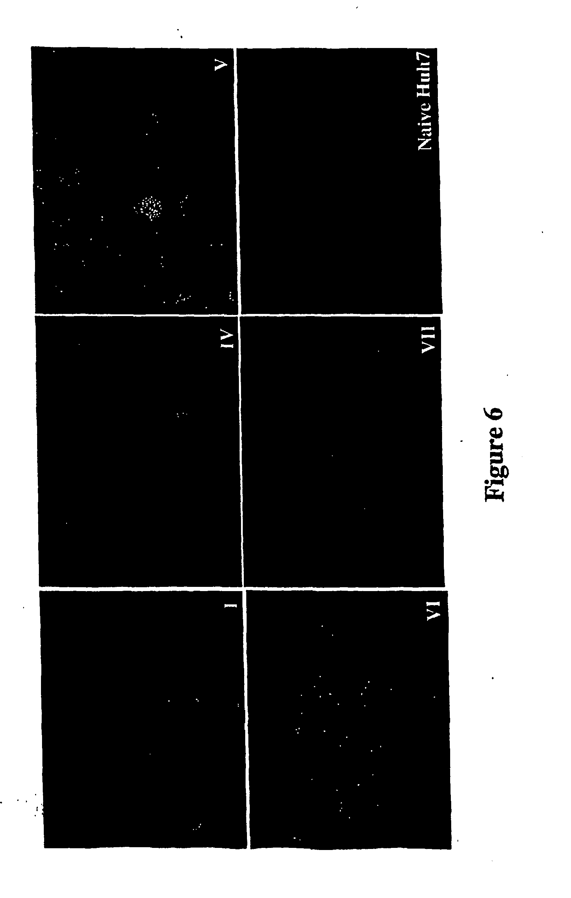

FIGURE 6. *Detection of NS3 in G418-resistant cell clones.* Monolayers of cells transfected with various replicons as indicated were immunostained with an anti-NS3 antibody. Patterns of staining were similar to cells stained from an infected liver.

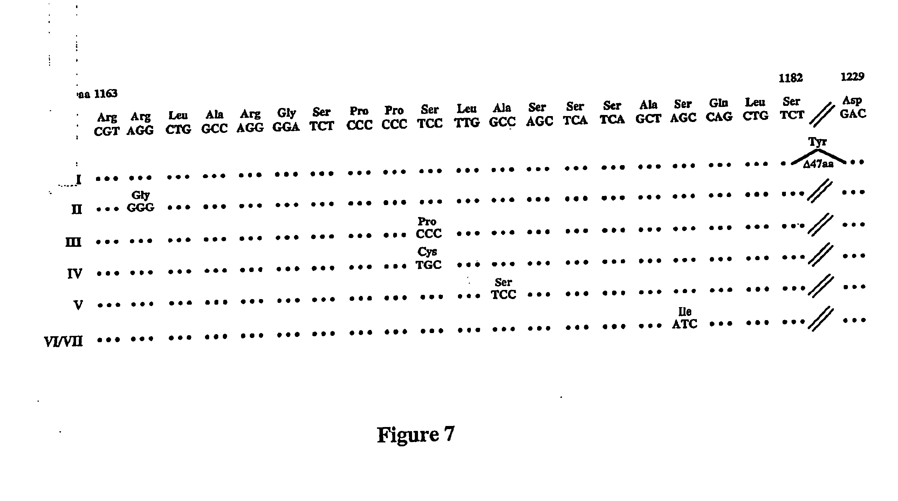

FIGURE 7. *Nucleotide and amino acid changes in the NS5A coding region of HCV.* Nucleotide and amino acid changes in a portion of the NS5A coding region of seven adaptive clones are indicated.

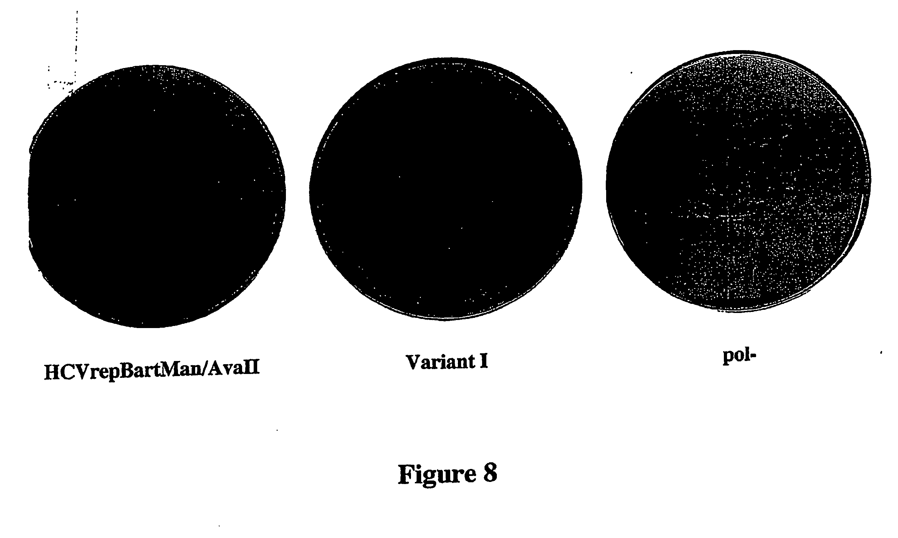

FIGURE 8. *G418-resistant colonies generated after electroporation of replicon RNAs into Huh7 cells.* The ability of an adaptive replicon (Replicon I) to establish colonies after transfection into Huh7 cells (middle) is compared to the original replicon HCVrepBartMan/AvaII (left) and the same adaptive replicon, but with an inactivating mutation in the polymerase gene (right).

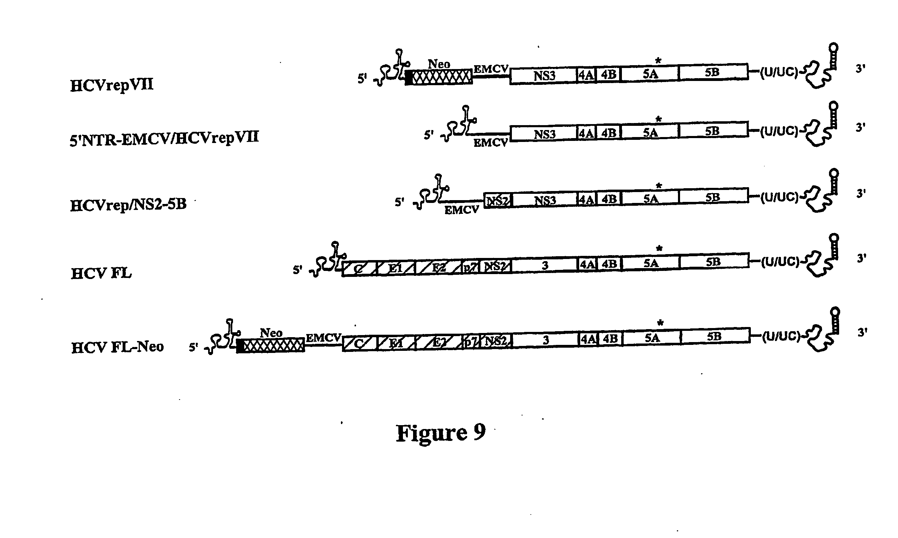

FIGURE 9. *Structures of HCV replicons and full-length HCV RNAs.* The adaptive replicon 5'NTR-EMCV has the 5'NTR fused directly to the EMCV IRES upstream of NS3. Another adaptive replicon, HCVrep/NS2-5B has the non-structural protein, NS2, upstream of NS3. A full-length HCV cDNA clone, HCV FL, was assembled. Also, a bicistronic derivative, HCV FL-neo, was assembled where the 5'NTR is fused to the neomycin phosphotransferase gene and the EMCV IRES is upstream of the HCV open reading frame. In both full-length clones, the open reading frame comprises the structural and non-structural regions, from capsid to NS5B. In addition, all of the replicons and full-length HCV RNAs comprise the mutation coding for Ser to Ile substitution at position 1179 of SEQ ID NO:3, in NS5A.

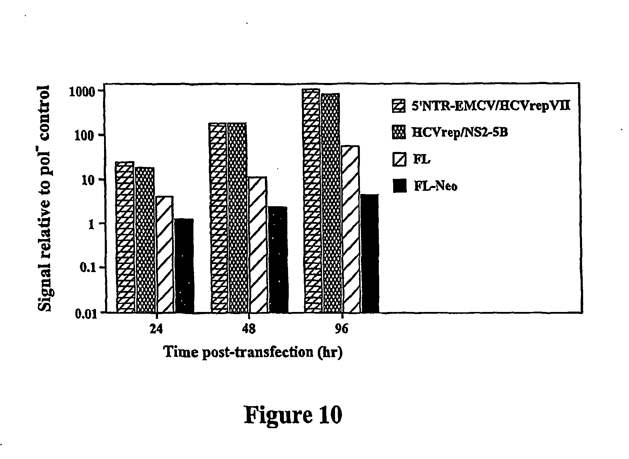

FIGURE 10. *RNA replication of replicons and full-length HCV RNAs.* The HCV replicons and full-length HCV RNAs shown in FIGURE 9 are replication competent.

### Detailed Description of the Invention

### Definitions

Various terms are used herein, which have the following definitions:
As used herein, "HCV polyprotein coding region" means the portion of a hepatitis C virus that codes for the polyprotein open reading frame (ORF). This ORF may encode proteins that are the same or different than wild-type HCV proteins. The ORF may also encode only some of the functional proteins encoded by a wild-type polyprotein coding region. The proteins encoded therein may also be from different isolates of HCV, and non-HCV proteins may also be encoded therein.
The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.
The phrase "therapeutically effective amount" is used herein to mean an amount sufficient to reduce by at least about 15 percent, preferably by at least 50 percent, more preferably by at least 90 percent, and most preferably prevent, a clinically significant deficit in the activity, function and response of the host. Alternatively, a therapeutically effective amount is sufficient to cause an improvement in a clinically significant condition in the host.
The term "adjuvant" refers to a compound or mixture that enhances the immune response to an antigen. An adjuvant can serve as a tissue depot that slowly releases the antigen and also as a lymphoid system activator that non-specifically enhances the immune response (Hood et al., Immunology, Second Ed., 1984, Benjamin/Cummings: Menlo Park, California, p. 384). Often, a primary challenge with an antigen alone, in the absence of an adjuvant, will fail to elicit a humoral or cellular immune response. Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, dinitrophenol, and potentially useful human adjuvants such as BCG *(bacille Calmette-Guerin)* and *Corynebacterium parvum.* Preferably, the adjuvant is pharmaceutically acceptable.

In a specific embodiment, the term "about" or "approximately" means within 20%, preferably within 10%, and more preferably within 5% of a given value or range.

The term "virus infection" as used herein, refers to the usual way that wild-type virus particles become established in host cells. This generally includes binding to the host cell, uptake, delivery to the cytosol or nucleus, and initiation of replication.

The term "transfection" as used herein, refers to the infection of a cell with a polynucleotide. The polynucleotide can be DNA or RNA. A preferred method of transfecting a cell with an HCV polynucleotide is with replication competent RNA. Delivery to permissive cells can be facilitated by electroporation, charged liposomes, high salt, DE dextran, etc. Replication competent RNAs can also be launched in cells after transfection of DNA such as plasmids or DNA viruses that have been appropriately engineered to provide transcription initiation and termination signals. The transfected RNAs can represent full-length genome RNAs capable of initating a complete replication cycle (including production of progeny virus), or they may be defective lacking one or more RNA elements or proteins essential for virion production but not RNA replication. The latter RNAs, which are lacking in the ability to produce a virion, will be referred to generally herein as "replication competent RNAs", "RNA replicons" or "replicons".

As used herein, the term "subpassage" connotes the transfer of a colony from one vessel of media to another vessel of media. Examples of vessels of media include dishes, bottles or test tubes with solid or liquid growth media. Unless otherwise indicated, "subpassage" means the transfer of a colony of HCV-transfected cells from a vessel of media where the newly transfected cells were plated to a vessel of media where the colony is isolated.

The term "authentic" is used herein to refer to an HCV polynucleotide, whether a DNA or RNA, that provides for replication and production of functional HCV proteins, or components thereof. The authentic HCV polynucleotides of the present invention are capable of replication and may be infectious, *e.g*., in a chimpanzee model or in tissue culture, to form viral particles (*i.e*., "virions"). An authentic HCV polynucleotide of the present invention may also be a "replicon", such that it is incapable of producing the full complement of structural proteins to make a replication competent infectious virion. However, such replicons are capable of RNA replication. Thus, the authentic HCV polynucleotides exemplified in the present application contains all of the virus-encoded information, whether in RNA elements or encoded proteins, necessary for initiation of an HCV RNA replication cycle. The authentic HCV polynucleotides of the invention include modifications described herein, *e.g*., by site-directed mutagenesis or by culture adaptation, producing a defective or attenuated derivative, or an adaptive variant. Alternatively, sequences from other genotypes or isolates can be substituted for the homologous sequence of the specific embodiments described herein. For example, an authentic HCV nucleic acid of the invention may comprise the adaptive mutations disclosed herein, *e.g*., on a recipient plasmid, engineered into the polyprotein coding region of a functional clone from another isolate or genotype (either a consensus region or one obtained by very high fidelity cloning). In addition, the HCV polynucleotide of the present invention can include a foreign gene, such as a gene encoding a selectable marker or a reporter protein.

### General Description

The practice of the present invention will employ, unless otherwise indicated, conventional techniques of cell culture, molecular biology, microbiology, recombinant DNA, and immunology, which are within the skill of the art. Such techniques are explained fully in the literature. See, e.g., Ausubel et al. (ed.) (1993) "Current protocols in molecular biology. Green Publishing Associates, New York; Ausubel et al. (1995), "Short Protocols in Molecular Biology", John Wiley and Sons; Joseph Sambrook et al. (1989), "Molecular Cloning, A Laboratory Manual", second ed., Cold Spring Harbor Laboratory Press; the series, METHODS IN ENZYMOLOGY (Academic Press, Inc.); Animal Cell Culture [R.I. Freshney, ed. (1986)]; Lau, ed. (1999), HEPATITIS C PROTOCOLS, Humana Press, New York; and Immobilized Cells And Enzymes [IRL Press, (1986)]; .

The present invention is directed to variants of hepatitis C virus (HCV) and methods for producing the variants. As used herein, an HCV variant is a non-naturally occurring HCV sequence that is capable of productive replication in a host cell. The genetic sequence of these variants may comprise insertions, deletions, or base mutations from wild type HCV sequences. As further discussed *infra,* the variants may be produced by genetic engineering, by methods known to the skilled artisan (see, e.g., U.S. Patent No. 6127116, Lohmann et al., Science 285:110-113(1999)). Alternatively, as further discussed *infra*, the variants may also be produced by culture selection methods, or a combination of culture selection and genetic engineering.

The variants are in the form of DNA or RNA and can be incorporated into any useful form of those compounds, for example in extrachromosomal DNA that replicates in a microorganism such as *E*. *coli* or yeast. Included among these are plasmids, phage, BACs, YACs, etc. RNA and virions comprising the variant are also envisioned as within the scope of the invention. The variants of the present invention can also be in the form of cassettes for insertion into a DNA cloning vector. The HCV RNAs are envisioned to be complementary to any HCV DNA disclosed herein. An infectious HCV RNA is a positive strand RNA created from the negative strand template of the HCV DNA clone of the invention.

The variants of the present invention are not narrowly limited to any particular virus subtype. Thus, any particular component of the variant, or the entire variant, may be from any HCV subtype. Preferred subtypes are 1a and 1b, due to the widespread occurrence, as well as the large amount of knowledge available for those two subtypes. However, the use of any other genotype or subtype, as would be considered within the skill of the art, is envisioned as within the scope of the invention. These subtypes include, but are not limited to, any subtypes within genotypes HCV-1, HCV-2, HCV-3, HCV-4, HCV-5, and HCV-6. Moreover, since HCV lacks proofreading activity, the virus itself readily mutates, forming mutant "quasi-species" of HCV that are also contemplated as useful for the present invention. Such mutations are easily identified by sequencing isolates from a subject as detailed herein or in U.S. Patent No.6127116. It would be expected that the methods and compositions disclosed herein are useful for any known subtype or quasi-species, or any subtype or quasi-species not now known but that is discovered in the future.

The HCV variants of the invention include a 5'-NTR conserved sequence, which generally comprises the 5'-terminal sequence GCCAGCC, and which may have additional bases upstream of this conserved sequence without affecting functional activity of the HCV nucleic acid. In a preferred embodiment, the 5'-GCCAGCC includes from 0 to about 10 additional upstream bases; more preferably it includes from 0 to about 5 upstream bases; more preferably still it includes 0, one, or two upstream bases. In specific embodiments, the extreme 5'-terminal sequence may be GCCAGCC; GGCCAGCC; UGCCAGCC; AGCCAGCC; AAGCCAGCC; GAGCCAGCC; GUGCCAGCC; or GCGCCAGCC, wherein the sequence GCCAGCC is the 5'-terminus of SEQ ID NO:1. However, the scope of the HCV variants of the invention encompasses any functional HCV 5'NTR, whether now known or later discovered.

The HCV variants of the invention also include a 3'NTR that comprises a polypyrimidine region as is known in wild-type HCV. These polypyrimidine regions are known to comprise, on the positive-strand HCV RNA, a poly(U)/poly(UC) tract or a poly(A) tract. However, the polypyrimidine region of the present invention may also include other polypyrimidine tracts that are not now known but are later found to be functional in infectious HCV. As is known in the art, the polypyrimidine tract may be of variable length: both short (about 75 bases) and long (133 bases) are effective, although an HCV clone containing a long poly(U/UC) tract is found to be highly infectious. Longer tracts may be found in naturally occurring HCV isolates. Thus, an authentic HCV nucleic acid of the invention may have a variable length polypyrimidine tract.

The 3'NTR also comprises, at its extreme 3' end, the highly conserved RNA element of about 98 nucleotides known in the art, and as described in, e.g., U.S. PatentNo. 5,874,565, U.S. Patent No.6127116, and U.S. Patent No. 5,837,463. In a specific aspect, the 3'-NTR extreme terminus is RNA homologous to a DNA having the sequence
5'-TGGTGGCTCCATCTTAGCCCTAGTCACGGCTAGCTGTGAAAGGTCCGTGAGCC GCATGACTGCAGAGAGTGCTGATACTGGCCTCTCTGCTGATCATGT-3' (SEQ ID NO:2). However, the scope of the invention is meant to encompass HCV variants with any HCV 3' NTR that allows virus replication, whether the sequence is now known or later discovered. Included are 3'NTRs that do not comprise a variable region.

The HCV variants of the present invention also include a polyprotein coding region sufficient to allow replication of the HCV RNA. Thus, the polyprotein coding region may be deficient in functional genes encoding the full complement of the HCV structural genes C, E1 and E2. In addition, the polyprotein coding region may comprise deletions, insertions, or mutations that do not occur in wild-type HCV strains. Further, the polyprotein coding region may be chimeric, such that some of the genes encoded therein are from analogous regions of another virus, as discussed *infra.*

The HCV variants encompassed by the present invention include variants that do not produce virus particles. These variants, which may be termed "replicons", lack the ability to produce a fully functional complement of the structural proteins C, E1 and E2. The inability to produce the functional structural protein component of the HCV virus may be conferred by deletion of the genes encoding one, two, or all three of these proteins. Alternatively, a deletion of a small portion of the coding sequence of one of the structural proteins, or a mutation in a critical region of the coding sequence, or an insertion into the coding sequence could lead to an HCV that cannot produce virions. In the latter case, the insertion can be any sequence that disrupts the ability of the structural protein from becoming part of a virion, and can include functional sequences, such as those that encode a reporter gene (such as β-galactosidase) or those that confers selectability to the cell harboring the replicon (such as *neo).* The above manipulations are entirely within the skill of the art. See, e.g., Lohmann et al., *supra* and Example 1. As discussed *infra,* such variants are useful for studying replication of the HCV virus, among other things.

The variants of the present invention can also comprise an alteration in the coding sequence of the polyprotein coding region that does not affect the production of functional virions or replicons. These alterations can be such that the amino acid sequence of the mature protein is not changed from the wild-type sequence, due to the degeneracy of the genetic code. Such alterations can be useful, e.g., when they introduce or remove a restriction site, such that the size of HCV fragments produced by digestion with a restriction enzyme is altered. This provides a distinguishing characteristic of that variant, which can be used, e.g., to identify a particular infectious isolate in a multiple infection animal model, or to provide convenient sites for subsequent engineering. Any technique for mutagenesis known in the art can be used, including but not limited to *in vitro* site-directed mutagenesis [Hutchinson, C., et al., 1978, J. Biol. Chem. 253:6551; Zoller and Smith, 1984, DNA 3:479-488; Oliphant et al., 1986, Gene 44:177; Hutchinson et al., 1986, Proc. Natl. Acad. Sci. U.S.A. 83:710], use of TAB® linkers (Pharmacia), etc. PCR techniques are preferred for site directed mutagenesis [see Higuchi, 1989, "Using PCR to Engineer DNA", in PCR Technology: Principles and Applications for DNA Amplification, H. Erlich, ed., Stockton Press, Chapter 6, pp. 61-70].

Alterations in the polyprotein coding sequence can also introduce conservative amino acid substitutions in the HCV-encoded proteins. Conservative amino acid substitutions refer to the interchangeability of residues having similar side chains. Conservatively substituted amino acids can be grouped according to the chemical properties of their side chains. For example, one grouping of amino acids includes those amino acids have neutral and hydrophobic side chains (A, V, L, I, P, W, F, and M); another grouping is those amino acids having neutral and polar side chains (G, S, T, Y, C, N, and Q); another grouping is those amino acids having basic side chains (K, R, and H); another grouping is those amino acids having acidic side chains (D and E); another grouping is those amino acids having aliphatic side chains (G, A, V, L, and I); another grouping is those amino acids having aliphatichydroxyl side chains (S and T); another grouping is those amino acids having amine-containing side chains (N, Q, K, R, and H); another grouping is those amino acids having aromatic side chains (F, Y, and W); and another grouping is those amino acids having sulfur-containing side chains (C and M). Preferred conservative amino acid substitutions are: R-K; E-D, Y-F, L-M; V-I, and Q-H. Conservative amino acid substitutions, when conferred on the structural proteins, can alter antigenic epitopes, and thus the immune reactivity of the virus. Those substitutions could also alter the function of the non-structural proteins, such that the virus reproduces at a different rate or is altered in its ability to replicate in cell culture or in an organism. See, e.g., Example 1, where replicon IV is adaptive to cell culture conditions due to the conservative amino acid substitution Ser → Cys in the NS5A protein.

Alterations in the polyprotein coding region could also introduce nonconservative amino acid substitutions in one or more of the proteins encoded therein. Nonconservative substitutions would be expected to alter protein function more drastically than conservative substitutions, and would thus be more likely than conservative substitutions to alter phenotypic characteristics of the virus such as replication rate, adaptation to cell culture or in vivo culture, and displayed antigenic determinants. Examples are several adaptive mutations in the NS5A coding region described in the , *infra.*

In some embodiments of the invention, the polyprotein coding region has a consensus sequence derived from more than one HCV isolate. For example, an authentic HCV nucleic acid of the invention may comprise a 5' and 3' sequence from any one subtype of the virus and a polyprotein region from any other subtype. Alternatively, only one of the proteins encoded in the polyprotein might be from another viral subtype. In this way, the effect of a particular protein in conferring characteristics of a particular strain (e.g., reduced virulence, increased replication rate etc.) can be studied.

Chimeras with other viruses, such as with bovine viral diarrhea virus, or another flavivirus, are also envisioned. See, e.g., PCT/US99/08850. In these embodiments, components of the functional clones can be used to construct chimeric viruses for assay of HCV gene functions and inhibitors thereof [Filocamo et al., J. Virol. 71: 1417-1427 (1997); Hahm et al., Virology 226: 318-326 (1996); Lu and Wimmer, Proc Natl Acad Sci USA 93:1412-7 (1996)]. In one such extension of the invention, functional HCV elements such as the 5' IRES, proteases, RNA helicase, polymerase, or 3' NTR are used to create chimeric derivatives of BVDV whose productive replication is dependent on one or more of these HCV elements. Such BVDV/HCV chimeras can then be used to screen for and evaluate antiviral strategies against these functional components.

Chimeras where a gene encoding a structural or nonstructural protein from a closely related virus such as GB virus B replaces the corresponding HCV gene would also be expected to be functional. See, e.g., Butkiewicz et al., 2000, J. Virol. 74, 4291-4301*.*

Other alterations in the polyprotein coding region contemplated by the present invention include deletions or insertions in the sequence. Such alterations may also alter replication rate, adaptation to various growth conditions, or antigenic determinants. A preferred example of a useful deletion includes the 47 amino acid deletion and replacement of Ser 1182 to Asp 1229 of SEQ ID NO:3 with Tyr, which is an adaptive mutation in the NS5A that provides greater transfection efficiency than HCVs with wild-type NS5A. See Example 1.

Insertions into the polyprotein coding region can be of any length and into any area of the region, provided the modified HCV is still able to replicate. Preferably, the insertion is engineered in frame with the rest of the polyprotein coding region, to allow correct translation of the polyprotein region downstream from the insertion.

Insertions into the polyprotein coding region could introduce a gene encoding a heterologous protein. The choice of heterologous protein is not narrowly limited and can include a protein that is therapeutic to the infected host or cell, or a protein that is harvested and purified for another purpose. Particularly useful heterologous genes include those used for detection of the variant (i.e., reporter genes), or for selection of cells having the variant. Nonlimiting examples of reporter genes useful in the present invention include β-galactosidase, β-glucuronidase, firefly or bacterial luciferase, green fluorescent protein (GFP) and humanized derivatives thereof, cell surface markers, and secreted markers. Such products are either assayed directly or may activate the expression or activity of additional reporters. Nonlimiting examples of selectable markers for mammalian cells include, but are not limited to, the genes encoding dihydrofolate reductase (DHFR; methotrexate resistance), thymidine kinase (*tk*; methotrexate resistance), puromycin acetyl transferase *(pac;* puromycin resistance), neomycin resistance (*neo*; resistance to neomycin or G418), mycophenolic acid resistance (gpt), hygromycin resistance, blasticidin resistance, and resistance to zeocin. Other selectable markers can be used in different hosts such as yeast (*ura*3*, his*3*, leu*2*, trp*1)*.*

The present invention also encompasses HCV variants that have alterations in the noncoding regions of the virus. For example, the foreign gene discussed above can also be inserted into a noncoding region of the virus, provided the region with the insert continues to be sufficiently functional to allow replication. To provide for translation of a foreign gene inserted into a noncoding region, the foreign gene must be operatively linked to translational start signals, preferably an internal ribosome entry site (IRES) derived from cellular or viral mRNAs [Jang et al., Enzyme 44: 292-309 (1991); Macejak and Sarnow, Nature 353: 90-94 1991); Molla et al., Nature 356: 255-257 (1992)]. In essence, this strategy creates a second cistron in the variant, separate from the polyprotein coding region cistron. A preferred IRES is the encephalomyocarditis virus (EMCV) IRES.

The foreign gene can also be inserted into the 3' NTR or the 5' NTR. In the 3' NTR, the foreign gene/IRES cassette is preferably inserted into the most 5', variable domain. However, insertions are also envisioned for other regions of the 3'NTR, such as at the junction of the variable region and the polypyrimidine region, or within the polypyrimidine region. In the 5'NTR, the foreign gene is preferably inserted into the area just adjacent (3' to) the internal HCV IRES. In these variants, the foreign gene is engineered to be operably linked to the HCV IRES. Where this is the case, it is preferred that the second IRES (e.g., an EMCV IRES) is engineered just 5' to the polyprotein coding region, to be operably linked to that region. See Example and Lohmann et al., *supra.*

Some of the above strategies for functional expression of heterologous genes have been previously described. See Bredenbeek and Rice, (1992) *supra* for review; see, also Figure 2, which is also Figure 2 of U.S. Patent No.6127116

Additionally, noncoding region alterations such as mutations, deletions or insertions that do not encode a foreign protein are within the scope of the invention. For example, mutations, deletions of insertions in the variable or polypyrimidine regions of the 3'NTR, including deletions of the entire variable region, or in the 5' NTR region, that create or destroy restriction sites or make the variant otherwise identifiable can be used advantageously to create a "tagged" variant. See, e.g., Example, where a mutation in the variable region of the 3' NTR created an easily identifiable *Ava*II restriction site, and where a deletion in the polypyrimidine region created another identifiable variant.

The polyprotein coding sequence comprises adaptive mutations and can comprise mutants with desirable functional adaptations such as attenuated variants. These improved variants can be superior in any desired characteristic. Nonlimiting examples of characteristics that can be improved by the present methods include more rapid or more accurate replication in vivo or in culture, improved transfection efficiency, improved ability to establish subpassaged cell lines, ability to infect a host or a host cell line, virulence, and attenuation of disease symptoms.

Such HCV variants may be adaptive, *e.g*., by selection for propagation in animals or in vitro. See, e.g., Example. Alternatively, the variants can be engineered by design to comprise the functional adaptation. See, e.g., Example, where a deletion was designed that had increased transfection efficiency and ability to be subpassaged to create a stable cell line, supporting persistent HCV replication.

Non-functional HCV clones, *e.g*., that are incapable of genuine replication, that fail to produce HCV proteins, that do not produce HCV RNA as detected by Northern analysis, or that fail to infect susceptible animals or cell lines *in vitro,* can be corrected using components of the variants of the present invention. By comparing a variant of an authentic HCV nucleic acid sequence of the invention, with the sequence of the non-functional HCV clone, defects in the non-functional clone can be identified and corrected, and the corrected, replicating variant could have characteristics like the variant, such as an adaptive mutation, etc. All of the methods for modifying nucleic acid sequences available to one of skill in the art to effect modifications in the non-functional HCV genome, including but not limited to site-directed mutagenesis, substitution of the functional sequence from an authentic HCV variant for the homologous sequence in the non-functional clone, etc.

*Adaptation of HCV for more improved cell culture characteristics.* Replication and transfection efficiency and stability of virions and replicons that have wild-type polyprotein replication in cell culture is inefficient. That is, cells transfected with, e.g., RNA transcripts of clones of these strains replicate slowly in culture and the transfected cells are difficult to maintain. Additionally, transfection efficiency is poor. That is, very few cells that are transfected with the RNA replicon are able to support HCV replication. See, e.g., Example 1 and Lohmann et al., *supra,* where less than 0.01% of Huh-7 cells transfected with RNA transcripts of replicons that have a wild-type (genotype 1, subtype 1b) nonstructural polyprotein coding region grew into colonies on the petri dish where the transfectants were plated. Furthermore, a low percentage of colonies that arose from the original plating (<3%) could be subpassaged onto another dish of media to form an isolated stable cell line supporting HCV replication.

"Transfection efficiency" is defined by determining the percent of cells having replicating HCV RNA that continue to translate proteins encoded by the transfected nucleic acids. The easiest way to measure this is by determining the percentage of cells that exhibit a characteristic conferred by the HCV RNA. See, e.g., Example 1, where replicons comprising a *neo* gene conferred G418 resistance to the transfected cells, and where the cells were G418 resistant after dividing and forming colonies on the dish where the transfected cells were plated. In that example, G418 resistance would not persist sufficiently for colonies to form unless the HCV RNA was able to replicate and partition into the dividing cells while continuing to replicate and translate the *neo* gene to confer G418 resistance. Transfection efficiency is thus replication dependent, in that the transfected HCV must replicate, transcribe, and translate the measured characteristic (here, G418 resistance). In the context of the *neo* selectable marker, this method of determining transfection efficiency is termed "replication-dependent neomycin resistance". This is the preferred way of measuring transfection efficiency because it only measures transcription from HCV that established itself sufficiently to replicate and partition into dividing cells to form a colony.

Another disadvantageous cell culture characteristic of HCV nucleic acid that has wild-type nonstructural polyprotein genes is that only a low percentage of colonies that form after transfection and selection are able to continue to be maintained upon subpassage as continuous cell lines harboring replicating RNA. This was <3% in Lohmann et al., as discussed *supra.*

Disadvantageous characteristics of HCV having wild-type nonstructural polyprotein genes can be reduced by utilizing certain adaptive mutations and deletions in the NS5A coding region or elsewhere as disclosed herein. Preferred mutations comprise alterations in the encoded amino acid sequence in a region of the NS5A that is just 5' to the coding region of the "interferon sensitivity-determining region" (ISDR). Specifically, various mutations within about 50 nucleotides 5' to the ISDR, more preferably within about 20 nucleotides of the ISDR, where the encoded amino acid sequence is altered, have the effect of adapting an HCV to have higher transfection efficiency and increased ability to withstand subpassage to establish a cell line harboring persistent HCV replication. Specific mutations having this effect include Ser to Ile at amino acid 1179 of SEQ ID NO:3 (subtype 1b nonstructural polyprotein region), conferred, for example, by the mutation g to t at position 5336 of SEQ ID NO:6, embodied in SEQ ID NO:8 (nucleotide[nt]) and SEQ ID NO:16 (amino acid[aa]); Arg to Gly at amino acid 1164 of SEQ ID NO:3, conferred, for example, by the mutation from a to g at position 5289 of SEQ ID NO:6, embodied in SEQ ID NO:9 (nt) and SEQ ID NO:17 (aa); Ala to Ser at amino acid 1174 of SEQ ID NO:3, conferred, for example, by the mutation from g to t at position 5320 of SEQ ID NO:6, embodied in SEQ ID NO:10 (nt) and the NS5A amino acid sequence of SEQ ID NO:19; Ser to Cys at amino acid 1172 of SEQ ID NO:3, conferred, for example, by the mutation c to g at position 5315 of SEQ ID NO:6, embodied in the NS5A gene SEQ ID NO:11 and the NS5A amino acid sequence of SEQ ID NO:20; and Ser to Pro at amino acid 1172 of SEQ ID NO:3, conferred, for example by the mutation t to c at position 5314 of SEQ ID NO:6, embodied in the NS5A gene SEQ ID NO:12 and the NS5A amino acid SEQ ID NO:21. The adaptive effect of these mutations is surprising since this region of HCV is normally conserved among HCV isolates. Additionally, deletions within the ISDR, including deletions of the entire ISDR and various flanking sequences, cause this adaptive effect. Among these deletions is the substitution of the ISDR and flanking sequence comprising amino acids 1182 to 1229 of SEQ ID NO:3 with a tyrosine, conferred, for example, by the deletion of nt 5345-5485 of SEQ ID NO:6, and embodied in SEQ ID NO:7 (nt) and the NS5A amino acid SEQ ID NO:14.

HCV variants comprising mutations adaptive to cell culture may also be attenuated, that is impaired in its ability to cause disease, establish chronic infections, trigger autoimmune responses, and transform cells.

The present invention also discloses methods for selecting for adaptive HCV variants. These methods comprise the use of an HCV virion or preferably a replicon, which further comprises a dominant selectable marker such as a *neo* gene. Cells are transfected with these variants. The transfectants are plated into selection media, such as G418 when the *neo* gene is utilized in the variant. Colonies that arise to exhibit resistance to the selectable marker are subpassaged into fresh selection media. HCV in colonies that withstand subpassage to establish a cell line harboring HCV replication can be isolated and used to transfect additional cells. Any of these colonies that show increased transfection efficiency or other desirable characteristics, such as the ability to withstand subpassage, are adaptive variants, where the adaptive nature of the variant is conferred by at least one mutation or deletion. Selected areas of the HCV in these adaptive variants are sequenced. Preferably, at least the NS5A is sequenced. More preferably, the entire polyprotein coding region is sequenced. Any mutations in these variants can be further evaluated to determine the adaptive nature of the mutations. That evaluation preferably involves recreating the mutation in an otherwise wild-type coding region and determining if the recreated HCV mutant exhibits the adaptive phenotype of the original mutant.

Adaptive mutations could also be manifested, but are not restricted to: (i) altering the tropism of HCV RNA replication; (ii) altering viral products responsible for deleterious effects on host cells; (iii) increasing or decreasing HCV RNA replication efficiency; (iv) increasing or decreasing HCV RNA packaging efficiency and/or assembly and release of HCV particles; (v) altering cell tropism at the level of receptor binding and entry. Thus, the engineered dominant selectable marker, whose expression is dependent upon productive HCV RNA replication, can be used to select for adaptive mutations in either the HCV replication machinery or the transfected host cell, or both. In addition, dominant selectable markers can be used to select for mutations in the HCV replication machinery that allow higher levels of RNA replication or particle formation. In one example, engineered HCV derivatives expressing a mutant form of DHFR can be used to confer resistance to methotrexate (MTX). As a dominant selectable marker, mutant DHFR is inefficient since nearly stoichiometric amounts are required for MTX resistance. By successively increasing concentrations of MTX in the medium, increased quantities of DHFR will be required for continued survival of cells harboring the replicating HCV RNA. This selection scheme, or similar ones based on this concept, can result in the selection of mutations in the HCV RNA replication machinery allowing higher levels of HCV RNA replication and RNA accumulation. Similar selections can be applied for mutations allowing production of higher yields of HCV particles in cell culture or for mutant HCV particles with altered cell tropism. Such selection schemes involve harvesting HCV particles from culture supernatants or after cell disruption and selecting for MTX-resistant transducing particles by reinfection of naive cells.

Methods similar to the above can be used to establish adaptive variants with variations in characteristics such as the increased or decreased ability to cause infection, the ability to cause infection in a host that wild-type strains are unable to infect, or cells of such a host.

The invention also provides host cell lines transfected with any of the HCV DNA (or HCV RNA) as set forth above. Examples of host cells include, but are by no means limited to, the group consisting of a bacterial cell, a yeast cell, an insect cell, and a mammalian cell. Preferably, the host cell is capable of providing for expression of functional HCV RNA replicase, virions or virus particle proteins.

In a related aspect, as briefly described above, the invention provides a vector for gene therapy or a gene vaccine (also termed herein a genetic vaccine), in which a heterologous protein is inserted into the HCV nucleic acid under conditions that permit expression of the heterologous protein. These vaccines can be either DNA or RNA. In particular, the invention provides an infectious hepatitis C virus (HCV) DNA vector comprising from 5' to 3' on the positive-sense DNA, a promoter; an HCV 5'-non-translated region (NTR) containing the extreme 5'-terminal sequence GCCAGCC; an HCV polyprotein coding region comprising a coding region for a heterologous gene; and a 3' non-translated region (NTR). Preferably, the promoter is selected from the group consisting of bacteriophage T3, T7, and SP6.

In the embodiments of the invention where the functional HCV nucleic acid is DNA, it may further comprise a promoter operatively associated with the 5' NTR. For example, but not by way of limitation, the promoter may be selected from the group consisting of bacteriophage T7, T3, and SP6. However, any suitable promoter for transcription of HCV genomic RNA corresponding to the HCV DNA can be used, depending on the specific transcription system employed. For example, for nuclear transcription (*e.g*., in an animal transgenic for HCV), an endogenous or viral promoter, such as CMV, may be used. Additionally, these promoter-driven HCV DNAs can be incorporated into an extrachromosomally replicating DNA such as a plasmid or a phage.

Various uses of the invention variants are envisioned herein. Uses relevant to therapy and vaccine development include: (i) the generation of defined HCV virus stocks to develop *in vitro* and *in vivo* assays for virus neutralization, attachment, penetration and entry; (ii) structure/function studies on HCV proteins and RNA elements and identification of new antiviral targets; (iii) a systematic survey of cell culture systems and conditions to identify those that support wild-type and variant HCV RNA replication and particle release; (iv) production of adaptive HCV variants capable of more efficient replication in cell culture; (v) production of HCV variants with altered tissue or species tropism; (vi) establishment of alternative animal models for inhibitor evaluation including those supporting HCV variant replication; (vii) development of cell-free HCV replication assays; (viii) production of immunogenic HCV particles for vaccination; (ix) engineering of attenuated HCV derivatives as possible vaccine candidates; (x) engineering of attenuated or defective HCV derivatives for expression of heterologous gene products for gene therapy and vaccine applications; (xi) utilization of the HCV glycoproteins for targeted delivery of therapeutic agents to the liver or other cell types with appropriate receptors.

Described is a method for infecting an animal with HCV variants, where the method comprises administering an infectious dose of HCV variant RNA prepared by transcription of infectious HCV variant DNA. This extends to a non-human animal infected with HCV variants or transfected with HCV variant RNA or DNA. Similarly, described is a method for propagating infectious HCV variants *in vitro* comprising culturing a cell line contacted with an infectious amount of HCV variant RNA prepared by transcription of the infectious HCV DNA, as well as an *in vitro* cell line infected with HCV variants. In a specific embodiment, the cell line is a hepatocyte cell line transfected or infected with an HCV variant in which an IRES-antibiotic resistance cassette has been engineered to provide for selection. The variant may also comprise the adaptive mutations described above.

In accordance with gene therapy (genetic vaccine) also described is a method for transducing an animal capable of HCV RNA replication with a heterologous gene, comprising administering an amount of an HCV variant RNA prepared by transcription of the HCV variant DNA vector.

Also described is a method for producing HCV particle proteins comprising culturing a host expression cell line transfected with an HCV variant of the invention under conditions that permit expression of HCV particle proteins; and isolating HCV particle proteins from the cell culture. In a specific embodiment, such an expression cell line may be a cell selected from the group consisting of a bacterial cell, a yeast cell, an insect cell, and a mammalian cell.

Also described is an HCV virion comprising an HCV variant RNA genome. Such virions can be used in an HCV vaccine, preferably after attenuation, e.g., by heat or chemical treatment, or through selection of attenuated variants by the methods described above.

The *in vivo* and *in vitro* HCV variants of the invention permits controlled screening for anti-HCV agents (i.e., drugs for treatment of HCV), as well as for evaluation of drug resistance. An *in vivo* method for screening for agents capable of modulating HCV replication may comprise administering a candidate agent to an animal containing an HCV variant, and testing for an increase or decrease in a level of HCV variant infection, replication or activity compared to a level of HCV variant infection, replication or activity in the animal prior to administration of the candidate agent; wherein a decrease in the level of HCV variant infection, replication or activity compared to the level of HCV variant infection, replication or activity in the animal prior to administration of the candidate agent is indicative of the ability of the agent to inhibit HCV variant infection, replication or activity. Testing for the level of HCV variant infection or replication can involve measuring the viral titer (e.g., RNA levels) in a serum or tissue sample from the animal; testing for the level of HCV variant activity can involve measuring liver enzymes. Alternatively, an *in vitro* method for screening for agents capable of modulating HCV replication can comprise contacting a cell line supporting a replicating HCV variant with a candidate agent; and thereafter testing for an increase or decrease in a level of HCV variant replication or activity compared to a level of HCV variant replication or activity in a control cell line or in the cell line prior to administration of the candidate agent, wherein a decrease in the level of HCV variant replication or activity compared to the level of HCV variant replication or activity in a control cell line or in the cell line prior to administration of the candidate agent is indicative of the ability of the agent to inhibit HCV variant replication or activity. In a specific embodiment, testing for the level of HCV variant replication *in vitro* may involve measuring the HCV titer, (*e.g*., RNA levels) in the cell culture; testing for the level of HCV activity *in vitro* may involve measuring HCV replication.

In addition to the specific HCV variant DNA clones and related HCV variant RNAs, the invention is directed to a method for preparing an HCV variant DNA clone that is capable of replication in a host or host cell line, comprising joining from 5' to 3' on the positive-sense DNA a promoter; an HCV 5' non-translated region (NTR) an HCV polyprotein coding region; and a 3' non-translated region (NTR), where at least one of these regions is not a naturally occurring region. Preferably, the promoter is selected from the group consisting of bacteriophage T7, T3, and SP6. In a specific embodiment, the extreme 5'-terminal sequence is homologous to SEQ ID NO:1, *e.g*., the 5'-terminal sequence may be selected from the group consisting of GCCAGCC; GGCCAGCC; UGCCAGCC; AGCCAGCC; AAGCCAGCC; GAGCCAGCC; GUGCCAGCC; and GCGCCAGCC, wherein the sequence GCCAGCC is the 5'-terminus of SEQ ID NO:1.

The 3'-NTR poly-U for use in the method of preparing an HCV variant DNA clone may include a long poly-U region. Similarly, the 3'-NTR extreme terminus may be RNA homologous to a DNA having the sequence
5'-TGGTGGCTCCATCTTAGCCCTAGTCACGGCTAGCTGTGAAAGGTCCGTGAGCC GCATGACTGCAGAGAGTGCTGATACTGGCCTCTCTGCTGATCATGT-3' (SEQ ID NO:2); in a specific embodiment, the 3'-NTR extreme terminus has the foregoing sequence.

*Components of functional HCV variant DNA clones.* Components of the functional HCV variant DNA described in this invention can be used to develop cell-free, cell culture, and animal-based screening assays for known or newly identified HCV antiviral targets as described *infra.* For each selected target, it is preferred that the HCV variant used has the wild-type form of the target. Examples of known or suspected targets and assays include [see Houghton, In "Fields Virology" (B. N. Fields, D. M. Knipe and P. M. Howley, Eds.), Vol. pp. 1035-1058. Raven Press, New York (1996); Rice, (1996) *supra;* Rice et al., Antiviral Therapy 1, Suppl. 4, 11-17 (1997); Shimotohno, Hepatology 21,:887-8 (1995) for reviews], but are not limited to, the following:

The highly conserved 5' NTR, which contains elements essential for translation of the incoming HCV genome RNA, is one target. It is also likely that this sequence, or its complement, contains RNA elements important for RNA replication and/or packaging. Potential therapeutic strategies include: antisense oligonucleotides *(supra);* trans-acting ribozymes *(supra);* RNA decoys; small molecule compounds interfering with the function of this element (these could act by binding to the RNA element itself or to cognate viral or cellular factors required for activity).

Another target is the HCV C (capsid or core) protein, which is highly conserved and is associated with the following functions: RNA binding and specific encapsidation of HCV genome RNA; transcriptional modulation of cellular [Ray et al., Virus Res. 37: 209-220 (1995)] and other viral [Shih et al., J. Virol. 69: 1160-1171 (1995); Shih et al., J. Virol. 67: 5823-5832 (1993)] genes; binding of cellular helicase [You et al., J. Virol. 73:2841-2853 (1999)]; cellular transformation [Ray et al., J. Virol. 70: 4438-4443 (1996a); Ray et al., J. Biol. Chem. 272:10983-10986(1997)]; prevention of apoptosis [Ray et al., Virol. 226: 176-182 (1996b)]; modulation of host immune response through binding to members of the TNF receptor superfamily [Matsumoto et al., J. Virol. 71: 1301-1309 (1997)].

The E1, E2, and perhaps the E2-p7 glycoproteins that form the components of the virion envelope are targets for potentially neutralizing antibodies. Key steps where intervention can be targeted include: signal peptidase mediated cleavage of these precursors from the polyprotein [Lin *et al.,* (1994a) *supra*]*;* ER assembly of the E1E2 glycoprotein complex and association of these proteins with cellular chaperones and folding machinery [Dubuisson *et al.,* (1994) *supra;* Dubuisson and Rice, J. Virol. 70: 778-786 (1996)]; assembly of virus particles including interactions between the nucleocapsid and virion envelope; transport and release of virus particles; the association of virus particles with host components such as VLDL *[Hijikata et al.,* (1993) *supra;* Thomssen *et al.,* (1992) *supra;* Thomssen et al., Med. Microbiol. Immunol. 182: 329-334 (1993)] which may play a role in evasion of immune surveillance or in binding and entry of cells expressing the LDL receptor; conserved and variable determinants in the virion which are targets for neutralization by antibodies or which bind to antibodies and facilitate immune-enhanced infection of cells via interaction with cognate Fc receptors; conserved and variable determinants in the virion important for receptor binding and entry; virion determinants participating in entry, fusion with cellular membranes, and uncoating the incoming viral nucleocapsid.

The NS2-3 autoprotease, which is required for cleavage at the 2/3 site is a further target.

The NS3 serine protease and NS4A cofactor which form a complex and mediate four cleavages in the HCV polyprotein [see Rice, (1997) *supra* for review) is yet another suitable target. Targets include the serine protease activity itself; the tetrahedral Zn²⁺ coordination site in the C-terminal domain of the serine protease; the NS3-NS4A cofactor interaction; the membrane association of NS4A; stabilization of NS3 by NS4A; transforming potential of the NS3 protease region [Sakamuro et al., J Virol 69: 3893-6 (1995)].

The NS3 RNA-stimulated NTPase *[Suzich et al.,* (1993) *supra],* RNA helicase [Jin and Peterson, Arch Biochem Biophys 323: 47-53 (1995); Kim et al., Biochem. Biophys. Res. Commun. 215: 160-6 (1995)], and RNA binding [Kanai et al., FEBS Lett 376: 221-4 (1995)] activities; the NS4A protein as a component of the RNA replication complex is another potential target.

The NS5A protein, another replication component, represents another target. This protein is phosphorylated predominantly on serine residues [Tanji et al., J. Virol. 69: 3980-3986 (1995)]. Transcription modulating, cell growth promoting, and apoptosis inhibiting activities of NS5A [Ghosh et al., J. Biol. Chem. 275:7184-7188 (2000)] can be targeted. Other characteristics of NS5A that could be targets for therapy include the kinase responsible for NS5A phosphorylation and its interaction with NS5A, and the interaction with NS5A and other components of the HCV replication complex.

The NS5B RNA-dependent RNA polymerase, which is the enzyme responsible for the actual synthesis of HCV positive and negative-strand RNAs, is another target. Specific aspects of its activity include the polymerase activity itself [Behrens et al., EMBO J. 15: 12-22 (1996)]; interactions of NS5B with other replicase components, including the HCV RNAs; steps involved in the initiation of negative- and positive-strand RNA synthesis; phosphorylation of NS5B [Hwang et al., Virology 227:438 (1997)].

Other targets include structural or nonstructural protein functions important for HCV RNA replication and/or modulation of host cell function. Possible hydrophobic protein components capable of forming channels important for viral entry, egress or modulation of host cell gene expression may be targeted.

The 3' NTR, especially the highly conserved elements (poly (U/UC) tract; 98-base terminal sequence) can be targeted. Therapeutic approaches parallel those described for the 5' NTR, except that this portion of the genome is likely to play a key role in the initiation of negative-strand synthesis. It may also be involved in other aspects of HCV RNA replication, including translation, RNA stability, or packaging.

The functional HCV variants of the present invention may encode all of the viral proteins and RNA elements required for RNA packaging. These elements can be targeted for development of antiviral compounds. Electrophoretic mobility shift, UV cross-linking, filter binding, and three-hybrid [SenGupta et al., Proc. Natl. Acad. Sci. USA 93: 8496-8501 (1996)] assays can be used to define the protein and RNA elements important for HCV RNA packaging and to establish assays to screen for inhibitors of this process. Such inhibitors might include small molecules or RNA decoys produced by selection *in vitro* [Gold *et al.,* (1995) *supra*]*.*

Complex libraries of the variants of the present invention can be prepared using PCR shuffling, or by incorporating randomized sequences, such as are generated in "peptide display" libraries. Using the "phage method" [Scott and Smith, 1990, Science 249:386-390 (1990); Cwirla, et al., Proc. Natl. Acad. Sci USA., 87:6378-6382 (1990); Devlin et al., Science, 249:404-406 (1990)], very large libraries can be constructed (10⁶-10⁸ chemical entities). Clones from such libraries can be used to generate other variants or chimeras, e.g., using various HCV subtypes. Such variants can be generated by methods known in the art, without undue experimentation.

A clone that includes a primer and run-off sequence can be used directly for production of functional HCV variant RNA. A large number of vector-host systems known in the art may be used. Examples of vectors include, but are not limited to, *E. coli,* bacteriophages such as lambda derivatives, or plasmids such as pBR322 derivatives or pUC plasmid derivatives, *e.g*., pGEX vectors, pmal-c, pFLAG, pTET, etc. As is well known, the insertion into a cloning vector can, for example, be accomplished by ligating the DNA fragment into a cloning vector that has complementary cohesive termini. However, if the complementary restriction sites used to fragment the DNA are not present in the cloning vector, the ends of the DNA molecules may be enzymatically modified. Alternatively, any site desired could be produced by ligating nucleotide sequences (linkers) onto the DNA termini; these ligated linkers may comprise specific chemically synthesized oligonucleotides encoding restriction endonuclease recognition sequences. Recombinant molecules can be introduced into host cells via transformation, transfection, infection, electroporation, etc., so that many copies of the gene sequence are generated.

### Expression of HCV RNA and Polypeptides

The HCV variant DNA, which codes for HCV variant RNA and HCV proteins, particularly HCV RNA replicase or virion proteins, can be inserted into an appropriate expression vector, *i.e*., a vector which contains the necessary elements for the transcription and translation of the inserted protein-coding sequence. Such elements are termed herein a "promoter." Thus, the HCV variant DNA of the invention is operationally (or operably) associated with a promoter in an expression vector of the invention. An expression vector also preferably includes a replication origin. The necessary transcriptional and translational signals can be provided on a recombinant expression vector. In a preferred embodiment for *in vitro* synthesis of functional RNAs, the T7, T3, or SP6 promoter is used.

Potential host-vector systems include but are not limited to mammalian cell systems infected with virus recombinant (*e.g*., vaccinia virus, adenovirus, Sindbis virus, Semliki Forest virus, etc.); insect cell systems infected with recombinant viruses (*e.g*., baculovirus); microorganisms such as yeast containing yeast vectors; plant cells; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

The cell into which the recombinant vector comprising the HCV variant DNA clone has been introduced is cultured in an appropriate cell culture medium under conditions that provide for expression of HCV RNA or such HCV proteins by the cell. Any of the methods previously described for the insertion of DNA fragments into a cloning vector may be used to construct expression vectors containing a gene consisting of appropriate transcriptional/translational control signals and the protein coding sequences. These methods may include *in vitro* recombinant DNA and synthetic techniques and *in vivo* recombination (genetic recombination).

Expression of HCV variant RNA or protein may be controlled by any promoter/enhancer element known in the art, but these regulatory elements must be functional in the host selected for expression. Promoters which may be used to control expression include, but are not limited to, the SV40 early promoter region (Benoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad. Sci. U.S.A. 80:21-25); promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58), alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94), myelin basic protein gene control region which is active in oligodendrocyte cells in the brain (Readhead et al., 1987, Cell 48:703-712), myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

A wide variety of host/expression vector combinations may be employed in expressing the DNA sequences of this invention. Useful expression vectors, for example, may consist of segments of chromosomal, non-chromosomal and synthetic DNA sequences. Suitable vectors include derivatives of SV40 and known bacterial plasmids, *e.g., E. coli* plasmids col E1, pCR1, pBR322, pMal-C2, pET, pGEX [Smith et al., 1988, Gene 67:31-40], pMB9 and their derivatives, plasmids such as RP4; phage DNAS, e.g., the numerous derivatives of phage λ, *e.g*., NM989, and other phage DNA, *e.g.,* M13 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like known in the art.

In addition to the preferred sequencing analysis, expression vectors containing an HCV variant DNA clone of the invention can be identified by four general approaches: (a) PCR amplification of the desired plasmid DNA or specific mRNA, (b) nucleic acid hybridization, (c) presence or absence of selection marker gene functions, (d) analysis with appropriate restriction endonucleases and (e) expression of inserted sequences. In the first approach, the nucleic acids can be amplified by PCR to provide for detection of the amplified product. In the second approach, the presence of nucleic acids in an expression vector can be detected by nucleic acid hybridization using probes comprising sequences that are homologous to the HCV variant DNA. In the third approach, the recombinant vector/host system can be identified and selected based upon the presence or absence of certain "selection marker" gene functions (*e.g*., β-galactosidase activity, thymidine kinase activity, resistance to antibiotics, transformation phenotype, occlusion body formation in baculovirus, etc.) caused by the insertion of foreign genes in the vector. In the fourth approach, recombinant expression vectors are identified by digestion with appropriate restriction enzymes. In the fifth approach, recombinant expression vectors can be identified by assaying for the activity, biochemical, or immunological characteristics of the gene product expressed by the recombinant, *e.g*., HCV RNA, HCV virions, or HCV viral proteins.

For example, in a baculovirus expression systems, both non-fusion transfer vectors, such as but not limited to pVL941 (BamHI cloning site; Summers), pVL1393 *(Bam*HI*, Sma*I*, Xba*I*, Eco*R1*, NotI, Xma*III*, Bgl*II*, and Pst*I cloning site; Invitrogen), pVL1392 *(Bgl*II*, Pst*I*, Not*I*, Xma*III*, Eco*RI*, Xba*I*, Sma*I*,* and *Bam*HI cloning site; Summers and Invitrogen), and pBlue*Bac*III (*Bam*HI*, Bgl*II*, Pst*I*, Nco*I*,* and *Hind*III cloning site, with blue/white recombinant screening possible; Invitrogen), and fusion transfer vectors, such as but not limited to pAc700 *(Bam*HI and *Kpn*I cloning site, in which the *Bam*HI recognition site begins with the initiation codon; Summers), pAc701 and pAc702 (same as pAc700, with different reading frames), pAc360 (*Bam*HI cloning site 36 base pairs downstream of a polyhedrin initiation codon; Invitrogen(195)), and pBlueBacHisA, B, C (three different reading frames, with *Bam*HI*, Bgl*II*, Pst*I*, Nco*I*,* and *Hind*III cloning site, an N-terminal peptide for ProBond purification, and blue/white recombinant screening of plaques; Invitrogen) can be used.

Examples of mammalian expression vectors contemplated for use in the invention include vectors with inducible promoters, such as the dihydrofolate reductase (DHFR) promoter, *e.g*., any expression vector with a *DHFR* expression vector, or a DHFR/methotrexate co-amplification vector, such as pED *(Pst*I*, Sal*I*, Sba*I*, Sma*I*,* and *Eco*RI cloning site, with the vector expressing both the cloned gene and DHFR); *[see* Kaufman, Current Protocols in Molecular Biology, 16.12 (1991)]. Alternatively, a glutamine synthetase/methionine sulfoximine co-amplification vector, such as pEE14 *(Hind*III*, Xba*I*, Sma*I*, Sba*I*, Eco*RI*,* and *Bcl*I cloning site, in which the vector expresses glutamine synthase and the cloned gene; Celltech). In another embodiment, a vector that directs episomal expression under control of Epstein Barr Virus (EBV) can be used, such as pREP4 (*Bam*HI*, Sfi*I*, Xho*I*, Not*I*, Nhe*I*, Hind*III*, Nhe*I*,* PvuII, and *Kpn*I cloning site, constitutive RSV-LTR promoter, hygromycin selectable marker; Invitrogen), pCEP4 *(Bam*HI*, Sfi*I*, Xho*I*, Not*I*, Nhe*I*, Hind*III*, Nhe*I*, Pvu*II*,* and *Kpn*I cloning site, constitutive hCMV immediate early gene, hygromycin selectable marker; Invitrogen), pMEP4 *(Kpn*I*, Pvu*I*, Nhe*I*, Hind*III*, Not*I*, Xho*I*, Sfi*I*, Bam*HI cloning site, inducible methallothionein IIa gene promoter, hygromycin selectable marker: Invitrogen), pREP8 *(Bam*HI*, Xho*I*, Not*I*, Hind*III*, Nhe*I*,* and *Kpn*I cloning site, RSV-LTR promoter, histidinol selectable marker; Invitrogen), pREP9 *(KpnI, NheI, Hind*III*, Not*I*, Xho*I*, Sfi*I*,* and BamHI cloning site, RSV-LTR promoter, G418 selectable marker; Invitrogen), and pEBVHis (RSV-LTR promoter, hygromycin selectable marker, N-terminal peptide purifiable via ProBond resin and cleaved by enterokinase; Invitrogen). Regulatable mammalian expression vectors, can be used, such as Tet and rTet [Gossen and Bujard, Proc. Natl. Acad. Sci. USA 89:5547-51 (1992); Gossen et al., Science 268:1766-1769 (1995)]. Selectable mammalian expression vectors for use in the invention include pRc/CMV *(Hind*III*, BstX*I*, Not*I*, Sba*I*,* and *Apa*I cloning site, G418 selection; Invitrogen), pRc/RSV *(Hind*III*, Spe*I*, BstX*I*, Not*I*, Xba*I cloning site, G418 selection; Invitrogen), and others. Vaccinia virus mammalian expression vectors *[see,* Kaufman (1991) *supra]* for use according to the invention include but are not limited to pSC11 *(SmaI* cloning site, TK- and β-gal selection), pMJ601 *(Sal*I*, Sma*I*, Afl*I*, Nar*I*, Bsp*MII*, Bam*HI*, Apa*I*, Nhe*I*, Sac*II*, Kpn*I*,* and HindIII cloning site; TK- and β-gal selection), and pTKgptF1S *(EcoRI, PstI, SalI, AccI, Hind*II*, Sba*I*, Bam*HI*,* and Hpa cloning site, TK or XPRT selection).

Examples of yeast expression systems include the non-fusion pYES2 vector (*Xba*I, *Sph*I*, Sho*I*, Not*I*, Gst*XI*, Eco*RI*, Bst*XI*, Bam*HI*, Sac*I*, Kpn*I*,* and *Hind*III cloning sit; Invitrogen) or the fusion pYESHisA, B, C *(Xba*I*, Sph*I*, Sho*I*, Not*I*, Bst*XI*, Eco*RI*, Bam*HI*, Sac*I*, Kpn*I*,* and *Hind*III cloning site, N-terminal peptide purified with ProBond resin and cleaved with enterokinase; Invitrogen), to mention just two, can be employed according to the invention.

In addition, a host cell strain may be chosen that modulates the expression of the inserted sequences, or modifies and processes the gene product in the specific fashion desired. Different host cells have characteristic and specific mechanisms for the translational and post-translational processing and modification (*e.g*., glycosylation, cleavage [*e.g*., of signal sequence]) of proteins. Expression in yeast can produce a glycosylated product. Expression in eukaryotic cells can increase the likelihood of "native" glycosylation and folding of an HCV protein. Moreover, expression in mammalian cells can provide a tool for reconstituting, or constituting, native HCV virions or virus particle proteins.

A variety of transfection methods, useful for other RNA virus studies, can be utilized herein without undue experimentation. Examples include microinjection, cell fusion, calcium-phosphate cationic liposomes such as lipofectin [Rice et al., New Biol. 1:285-296 (1989); see "HCV-based Gene Expression Vectors", infra], DE-dextran *[*Rice et al., J. Virol. 61: 3809-3819 (1987)], and electroporation [Bredenbeek et al., J. Virol. 67: 6439-6446 (1993*);* Liljeström et al., J. Virol. 65: 4107-4113 (1991)]. Scrape loading [Kumar et al., Biochem. Mol. Biol. Int. 32: 1059-1066 (1994)] and ballistic methods [Burkholder et al., J. Immunol. Meth. 165: 149-156 (1993)] may also be considered for cell types refractory to transfection by these other methods. A DNA vector transporter may be considered [see, *e.g.,* Wu et al., 1992, J. Biol. Chem. 267:963-967; Wu and Wu, 1988, J. Biol. Chem. 263:14621-14624; *Hartmut et al.,* Canadian Patent Application No. 2,012,311, filed March 15, 1990].

### In Vitro Transfection With HCV Variants

*Identification of cell lines supporting HCV replication.* An important aspect of the invention is a method it provides for developing new and more effective anti-HCV therapy by conferring the ability to evaluate the efficacy of different therapeutic strategies using an authentic and standardized *in vitro* HCV variant replication system. Such assays are invaluable before moving on to trials using rare and valuable experimental animals, such as the chimpanzee, or HCV-infected human patients. The adaptive variants of the invention are particularly useful for this work because their growth in culture and their ability to withstand subpassage is superior to wild-type strains. Also, the replicons disclosed herein are useful because replication can be evaluated without the confounding effects of the structural proteins.

The HCV variant infectious clone technology can also be used to establish *in vitro* and *in vivo* systems for analysis of HCV replication and packaging. These include, but are not restricted to, (i) identification or selection of permissive cell types (for RNA replication, virion assembly and release); (ii) investigation of cell culture parameters (*e.g*., varying culture conditions, cell activation, etc.) or selection of adaptive mutations that increase the efficiency of HCV replication in cell cultures; and (iii) definition of conditions for efficient production of infectious HCV variant particles (either released into the culture supernatant or obtained after cell disruption). These and other readily apparent extensions of the invention have broad utility for HCV therapeutic, vaccine, and diagnostic development.

General approaches for identifying permissive cell types are outlined below. Optimal methods for RNA transfection (see also, *supra)* vary with cell type and are determined using RNA reporter constructs. These include, for example, the bicistronic replicons disclosed *supra* and in the Examples, and bicistronic virus [Wang *et al., J. Virol.* **67**: 3338-44 (1993)] with the structure 5'-CAT-HCV IRES-LUC-3'. These HCV variants are used both to optimize transfection conditions (using, e.g., by measuring β-galactosidase or CAT [chloramphenicol acetyltransferase] activity to determine transfection efficiency) and to determine if the cell type is permissive for HCV IRES-mediated translation (e.g., by measuring LUC; luciferase activity). For actual HCV RNA transfection experiments, cotransfection with a 5' capped luciferase reporter RNA [Wang *et al.,* (1993) *supra]* provides an internal standard for productive transfection and translation. Examples of cell types potentially permissive for HCV replication include, but are not restricted to, primary human cells (*e.g*., hepatocytes, T-cells, B-cells, foreskin fibroblasts) as well as continuous human cell lines (*e.g*., HepG2, Huh7, HUT78, HPB-Ma, MT-2, MT-2C, and other HTLV-1 and HTLV-II infected T-cell lines, Namalawa, Daudi, EBV-transformed LCLs). In addition, cell lines of other species, especially those which are readily transfected with RNA and permissive for replication of flaviviruses or pestiviruses (*e.g*., SW-13, Vero, BHK-21, COS, PK-15, MBCK, etc.), can be tested. Cells are transfected using a method as described *supra.*

For replication assays, RNA transcripts are prepared using the HCV variant and the corresponding non-functional, *e.g*., ΔGDD (see Examples) derivative as a negative control, for persistence of HCV RNA and antigen in the absence of productive replication. Template DNA (which complicates later analyses) is removed by repeated cycles of DNaseI treatment and acid phenol extraction followed by purification by either gel electrophoresis or gel filtration, to preferably achieve less than one molecule of amplifiable DNA per 10⁹ molecules of transcript RNA. DNA-free RNA transcripts are mixed with LUC reporter RNA and used to transfect cell cultures using optimal conditions determined above. After recovery of the cells, RNaseA is added to the media to digest excess input RNA and the cultures incubated for various periods of time. An early timepoint (∼1 day post-transfection) will be harvested and analyzed for LUC activity (to verify productive transfection) and positive-strand RNA levels in the cells and supernatant (as a baseline). Samples are collected periodically for 2-3 weeks and assayed for positive-strand RNA levels by QC-RT/PCR *[see* Kolykhalov *et al.,* (1996) *supra].* Cell types showing a clear and reproducible difference between the intact infectious transcript and the non-functional derivative, *e.g*., ΔGDD deletion, control can be subjected to more thorough analyses to verify authentic replication. Such assays include measurement of negative-sense HCV RNA accumulation by QC-RT/PCR [Gunji *et al.,* (1994) *supra;* Lanford et al., Virology 202: 606-14 (1994)], Northern-blot hybridization, or metabolic labeling [Yoo *et al.,* (1995) *supra]* and single cell methods, such as *in situ* hybridization [ISH; Gowans et al., In "Nucleic Acid Probes" (R. H. Symons, Eds.), Vol. pp. 139-158. CRC Press, Boca Raton. (1989)], *in situ* PCR [followed by ISH to detect only HCV-specific amplification products; Haase et al., Proc. Natl. Acad. Sci. USA 87: 4971-4975 (1990)], and immunohistochemistry.

*HCV particles for studying virus-receptor interactions.* In combination with the identification of cell lines that are permissive for HCV replication, defined HCV variant stocks can be used to evaluate the interaction of the HCV with cellular receptors. Assays can be set up which measure binding of the virus to susceptible cells or productive infection, and then used to screen for inhibitors of these processes.

*Identification of cell lines for characterization of HCV receptors.* Cell lines permissive for HCV RNA replication, as assayed by RNA transfection, can be screened for their ability to be infected by the virus using the HCV variants of the present invention. Cell lines permissive for RNA replication but which cannot be infected by the homologous virus may lack one or more host receptors required for HCV binding and entry. Such cells provide valuable tools for (i) functional identification and molecular cloning of HCV receptors and co-receptors; (ii) characterization of virus-receptor interactions; and (iii) developing assays to screen for compounds or biologics (*e.g*., antibodies, SELEX RNAs [Bartel and Szostak, In "RNA-protein interactions" (K. Nagai and I. W. Mattaj, Eds.), Vol. pp. 82-102. IRL Press, Oxford (1995); Gold et al., Annu. Rev. Biochem. 64: 763-797 (1995)], etc.) that inhibit these interactions. Once defined in this manner, these HCV receptors serve not only as therapeutic targets but may also be expressed in transgenic animals rendering them susceptible to HCV infection *[Koike et al., Dev Biol Stand* **78**: 101-7 (1993); Ren and Racaniello, *J Virol* **66***:* 296-304 (1992)]. Such transgenic animal models supporting HCV replication and spread have important applications for evaluating anti-HCV drugs.

The ability to manipulate the HCV glycoprotein structure may also be used to create HCV variants with altered receptor specificity. In one example, HCV glycoproteins can be modified to express a heterologous binding domain for a known cell surface receptor. The approach should allow the engineering of HCV derivatives with altered tropism and perhaps extend infection to non-chimeric small animal models.

*Alternative approaches for identifying permissive cell lines.* As previously discussed, and as exemplified in the Examples, functional HCV variants can be engineered that comprise selectable markers for HCV replication. For instance, genes encoding dominant selectable markers can be expressed as part of the HCV polyprotein, or as separate cistrons located in permissive regions of the HCV RNA genome.

### Animal Models for HCV Infection and Replication

In addition to chimpanzees, the present invention permits development of alternative animal models for studying HCV replication and evaluating novel therapeutics. Using clones of the authentic HCV variants described in this invention as starting material, multiple approaches can be envisioned for establishing alternative animal models for HCV replication. In one manifestation, the variants could be used to inoculate immunodeficient mice harboring human tissues capable of supporting HCV replication. An example of this art is the SCID:Hu mouse, where mice with a severe combined immunodeficiency are engrafted with various human (or chimpanzee) tissues, which could include, but are not limited to, fetal liver, adult liver, spleen, or peripheral blood mononuclear cells. Besides SCID mice, normal irradiated mice can serve as recipients for engraftment of human or chimpanzee tissues. These chimeric animals would then be substrates for HCV replication after either *ex vivo* or *in vivo* infection with defined virus-containing inocula.

In another manifestation, adaptive mutations allowing HCV replication in alternative species may produce variants that are permissive for replication in these animals. For instance, adaptation of HCV for replication and spread in either continuous rodent cell lines or primary tissues (such as hepatocytes) could enable the virus to replicate in small rodent models. Alternatively, complex libraries of HCV variants created by DNA shuffling [Stemmer, Proc. Natl. Acad. Sci. USA 91:10747 (1994)] or other methods known in the art can be created and used for inoculation of potentially susceptible animals. Such animals could be either immunocompetent or immunodeficient, as described above.

The functional activity of HCV variants can be evaluated transgenically. In this respect, a transgenic mouse model can be used *[see, e.g.,* Wilmut *et al., Experientia* **47**:905 (1991)]. The HCV RNA or DNA clone can be used to prepare transgenic vectors, including viral vectors, plasmid or cosmid clones (or phage clones). Cosmids may be introduced into transgenic mice using published procedures [Jaenisch, *Science,* **240**:1468-1474 (1988)]. In the preparation of transgenic mice, embryonic stem cells are obtained from blastocyst embryos [Joyner, In Gene Targeting: A Practical Approach. The Practical Approach Series, Rickwood, D., and Hames, B. D., Eds., IRL Press: Oxford (1993)] and transfected with HCV variant DNA or RNA. Transfected cells are injected into early embryos, e.g., mouse embryos, as described [Hammer et al., Nature 315:680 (1985); Joyner, *supra].* Various techniques for preparation of transgenic animals have been described [U.S. Patent No. 5,530,177, issued June 25, 1996; U.S. Patent No. 5,898,604, issued December 31, 1996]. Of particular interest are transgenic animal models in which the phenotypic or pathogenic effects of a transgene are studied. For example, the effects of a rat phosphoenolpyruvate carboxykinase-bovine growth hormone fusion gene has been studied in pigs [Wieghart et al., J. Reprod. Fert., Suppl. 41:89-96 (1996)]. Transgenic mice that express of a gene encoding a human amyloid precursor protein associated with Alzheimer's disease are used to study this disease and other disorders [International Patent Publication WO 96/06927, published March 7, 1996; Quon et al., Nature 352:239 (1991)]. Transgenic mice have also been created for the hepatitis delta agent [Polo et al., J. Virol. 69:5203 (1995)] and for hepatitis B virus [Chisari, Curr. Top. Microbiol. Immunol. 206:149 (1996)], and replication occurs in these engineered animals.

Thus, the functional HCV variants described here, or parts thereof, can be used to create transgenic models relevant to HCV replication and pathogenesis. In one example, transgenic animals harboring the entire genome of an HCV variant can be created. Appropriate constructs for transgenic expression of the entire HCV variant genome in a transgenic mouse of the invention could include a nuclear promoter engineered to produce transcripts with the appropriate 5' terminus, the full-length HCV variant cDNA sequence, a cis-cleaving delta ribozyme [Ball, J. Virol. 66: 2335-2345 (1992); Pattnaik et al., Cell 69: 1011-1020 (1992)] to produce an authentic 3' terminus, followed possibly by signals that promote proper nuclear processing and transport to the cytoplasm (where HCV RNA replication occurs). Besides the entire HCV variant genome, animals can be engineered to express individual or various combinations of HCV proteins and RNA elements. For example, animals engineered to express an HCV gene product or reporter gene under the control of the HCV IRES can be used to evaluate therapies directed against this specific RNA target. Similar animal models can be envisioned for most known HCV targets.

Such alternative animal models are useful for (i) studying the effects of different antiviral agents on replication of HCV variants, including replicons, in a whole animal system; (ii) examining potential direct cytotoxic effects of HCV gene products on hepatocytes and other cell types, defining the underlying mechanisms involved, and identifying and testing strategies for therapeutic intervention; and (iii) studying immune-mediated mechanisms of cell and tissue damage relevant to HCV pathogenesis and identifying and testing strategies for interfering with these processes.

### Selection and Analysis of Drug-Resistant Variants

Cell lines and animal models supporting HCV replication can be used to examine the emergence of HCV variants with resistance to existing and novel therapeutics. Like all RNA viruses, the HCV replicase is presumed to lack proofreading activity and RNA replication is therefore error prone, giving rise to a high level of variation [Bukh *et al.,* (1995) *supra*]*.* The variability manifests itself in the infected patient over time and in the considerable diversity observed between different isolates. The emergence of drug-resistant variants is likely to be an important consideration in the design and evaluation of HCV mono and combination therapies. HCV replication systems of the invention can be used to study the emergence of variants under various therapeutic formulations. These might include monotherapy or various combination therapies (*e.g*., IFN-α, ribavirin, and new antiviral compounds). Resistant mutants can then be used to define the molecular and structural basis of resistance and to evaluate new therapeutic formulations, or in screening assays for effective anti-HCV drugs *(infra).*

### Screening For Anti-HCV Agents

HCV-permissive cell lines or animal models (preferably rodent models) comprising adaptive HCV variants can be used to screen for novel inhibitors or to evaluate candidate anti-HCV therapies. Such therapies include, but would not be limited to, (i) antisense oligonucleotides or ribozymes targeted to conserved HCV RNA targets; (ii) injectable compounds capable of inhibiting HCV replication; and (iii) orally bioavailable compounds capable of inhibiting HCV replication. Targets for such formulations include, but are not restricted to, (i) conserved HCV RNA elements important for RNA replication and RNA packaging; (ii) HCV-encoded enzymes; (iii) protein-protein and protein-RNA interactions important for HCV RNA replication, virus assembly, virus release, viral receptor binding, viral entry, and initiation of viral RNA replication; (iv) virus-host interactions modulating the ability of HCV to establish chronic infections; (v) virus-host interactions modulating the severity of liver damage, including factors affecting apoptosis and hepatotoxicity; (vi) virus-host interactions leading to the development of more severe clinical outcomes including cirrhosis and hepatocellular carcinoma; and (vii) virus-host interactions resulting in other, less frequent, HCV-associated human diseases.

*Evaluation of antisense and ribozyme therapies.* The present invention extends to the preparation of antisense nucleotides and ribozymes that may be tested for the ability to interfere with HCV replication. This approach utilizes antisense nucleic acid and ribozymes to block translation of a specific mRNA, either by masking that mRNA with an antisense nucleic acid or cleaving it with a ribozyme.

Antisense nucleic acids are DNA or RNA molecules that are complementary to at least a portion of a specific mRNA molecule. Reviews of antisense technology include: Baertschi, Mol. Cell. Endocrinol. 101:R15-R24 (1994); Crooke et al., Annu. Rev. Pharmacol. Toxicol. 36:107-129 (1996); Alama et al., Pharmacol. Res. 36:171-178; and Boyer et al., J. Hepatol. 32(1 Suppl):98-112(2000). The last review discusses antisense technology as it applies to HCV.

In the cell, they hybridize to that mRNA, forming a double stranded DNA:RNA or RNA:RNA molecule. The cell does not translate an mRNA in this double-stranded form. Therefore, antisense nucleic acids interfere with the expression of mRNA into protein. Oligomers of about fifteen nucleotides and molecules that hybridize to the AUG initiation codon will be particularly efficient, since they are easy to synthesize and are likely to pose fewer problems than larger molecules when introducing them into organ cells. Antisense methods have been used to inhibit the expression of many genes *in vitro.* Preferably synthetic antisense nucleotides contain phosphoester analogs, such as phosphorothiolates, or thioesters, rather than natural phophoester bonds. Such phosphoester bond analogs are more resistant to degradation, increasing the stability, and therefore the efficacy, of the antisense nucleic acids.

In the genetic antisense approach, expression of the wild-type allele is suppressed because of expression of antisense RNA. This technique has been used to inhibit TK synthesis in tissue culture and to produce phenotypes of the *Kruppel* mutation in *Drosophila,* and the *Shiverer* mutation in mice [Izant et al., Cell, 36:1007-1015 (1984); Green et al., Annu. Rev. Biochem., 55:569-597 (1986); Katsuki et al., Science, 241:593-595 (1988)]. An important advantage of this approach is that only a small portion of the gene need be expressed for effective inhibition of expression of the entire cognate mRNA. The antisense transgene will be placed under control of its own promoter or another promoter expressed in the correct cell type, and placed upstream of the SV40 polyA site.

Ribozymes are RNA molecules possessing the ability to specifically cleave other single stranded RNA molecules in a manner somewhat analogous to DNA restriction endonucleases. Ribozymes were discovered from the observation that certain mRNAs have the ability to excise their own introns. By modifying the nucleotide sequence of these RNAs, researchers have been able to engineer molecules that recognize specific nucleotide sequences in an RNA molecule and cleave it. Recent reviews include Shippy et al., Mol. Biotechnol. 12:117-129 (1999); Schmidt, Mol. Cells 9:459-463 (1999); Phylactou et al., Meth. Enzymol. 313:485-506 (2000); Oketani et al., J. Hepatol. 31:628-634 (1999); Macejak et al., Hepatology 31:769-776 (2000). The last two references disclose the use of ribozymes for inhibiting HCV. Because they are sequence-specific, only mRNAs with particular sequences are inactivated.

Investigators have identified two types ofribozymes, *Tetrahymena-type* and "hammerhead"-type. *Tetrahymena-type* ribozymes recognize four-base sequences, while "hammerhead"-type recognize eleven- to eighteen-base sequences. The longer the recognition sequence, the more likely it is to occur exclusively in the target mRNA species. Therefore, hammerhead-type ribozymes are preferable to *Tetrahymena-type* ribozymes for inactivating a specific mRNA species, and eighteen base recognition sequences are preferable to shorter recognition sequences.

*Screening compound libraries for anti-HCV activity.* Various natural product or synthetic libraries can be screened for anti-HCV activity in the *in vitro* or *in vivo* models comprising HCV variants as provided by the invention. One approach to preparation of a combinatorial library uses primarily chemical methods, of which the Geysen method [Geysen et al., Molecular Immunology 23:709-715 (1986); Geysen et al.J. Immunologic Method 102:259-274 (1987)] and the method of Fodor et al. [Science 251:767-773 (1991)] are examples. Furka et al. [14th International Congress of Biochemistry, Volume 5, Abstract FR:013 (1988); Furka, Int. J. Peptide Protein Res. 37:487-493 (1991)], Houghton [U.S. Patent No. 4,631,211, issued December 1986] and Rutter et al. [U.S. Patent No. 5,010,175, issued April 23, 1991] describe methods to produce a mixture of peptides that can be tested for anti-HCV activity.

In another aspect, synthetic libraries [Needels et al., Proc. Natl. Acad. Sci. USA 90:10700-4 (1993); Ohlmeyer et al., Proc. Natl. Acad. Sci. USA 90:10922-10926 (1993); Lam *et al.,* International Patent Publication No. WO 92/00252; *Kocis et al.,* International Patent Publication No. WO 9428028], and the like can be used to screen for anti-HCV compounds according to the present invention. The references describe adaption of the library screening techniques in biological assays.

*Defined*/*engineered HCV variant virus particles for neutralization assays.* The variants described herein can be used to produce defmed stocks of HCV particles for infectivity and neutralization assays. Homogeneous stocks can be produced in the chimpanzee model, in cell culture systems, or using various heterologous expression systems (*e.g*., baculovirus, yeast, mammalian cells; see *supra).* These stocks can be used in cell culture or *in vivo* assays to define molecules or gene therapy approaches capable of neutralizing HCV particle production or infectivity. Examples of such molecules include, but are not restricted to, polyclonal antibodies, monoclonal antibodies, artificial antibodies with engineered/optimized specificity, single-chain antibodies (see the section on antibodies, *infra),* nucleic acids or derivatized nucleic acids selected for specific binding and neutralization, small orally bioavailable compounds, etc. Such neutralizing agents, targeted to conserved viral or cellular targets, can be either genotype or isolate-specific or broadly cross-reactive. They could be used either prophylactically or for passive immunotherapy to reduce viral load and perhaps increase the chances of more effective treatment in combination with other antiviral agents (*e.g*., IFN-α, ribavirin, etc.). Directed manipulation of HCV infectious clones can also be used to produce HCV stocks with defined changes in the glycoprotein hypervariable regions or in other epitopes to study mechanisms of antibody neutralization, CTL recognition, immune escape and immune enhancement. These studies will lead to identification of other virus-specific functions for anti-viral therapy.

### Dissection of HCV Replication

*Other HCV replication assays.* This invention allows directed molecular genetic dissection of HCV replication. Such analyses are expected to (i) validate antiviral targets which are currently being pursued; and (ii) uncover unexpected new aspects of HCV replication amenable to therapeutic intervention. Targets for immediate validation through mutagenesis studies include the following: the 5' NTR, the HCV polyprotein and cleavage products, and the 3' NTR. As described above, analyses using the HCV variants and permissive cell cultures can be used to compare parental and mutant replication phenotypes after transfection of cell cultures with infectious RNA. Even though RT-PCR allows sensitive detection of viral RNA accumulation, mutations which decrease the efficiency of RNA replication may be difficult to analyze, unless conditional mutations are recovered. As a complement to first cycle analyses, trans-complementation assays can be used to facilitate analysis of HCV mutant phenotypes and inhibitor screening. Chimeric variants comprising portions of heterologous systems (vaccinia, Sindbis, or non-viral) can be used to drive expression of the HCV RNA replicase proteins and/or packaging machinery [see Lemm and Rice, J. Virol. 67: 1905-1915 (1993a); Lemm and Rice, J. Virol. 67: 1916-1926 (1993b); Lemm et al., EMBO J. 13: 2925-2934 (1994); Li et al., J. Virol. 65: 6714-6723 (1991)]. If these elements are capable of functioning in *trans,* then co-expression of RNAs with appropriate cis-elements should result in RNA replication/packaging. Such systems therefore mimic steps in authentic RNA replication and virion assembly, but uncouple production of viral components from HCV replication. If HCV replication is somehow self-limiting, heterologous systems may drive significantly higher levels of RNA replication or particle production, facilitating analysis of mutant phenotypes and antiviral screening. A third approach is to devise cell-free systems for HCV template-dependent RNA replication. A coupled translation/replication and assembly system has been described for poliovirus in HeLa cells [Barton and Flanegan, J. Virol. 67: 822-831 (1993); Molla et al., Science 254: 1647-1651 (1991)], and a template-dependent *in vitro* assay for initiation of negative-strand synthesis has been established for Sindbis virus. Similar *in vitro* systems using HCV variants are invaluable for studying many aspects of HCV replication as well as for inhibitor screening and evaluation. An example of each of these strategies follows.

*Trans-complementation of HCV RNA replication and*/*or packaging using viral or non-viral expression systems.* Heterologous systems can be used to drive HCV replication. For example, the vaccinia/T7 cytoplasmic expression system has been extremely useful for trans-complementation of RNA virus replicase and packaging functions [see Ball, (1992) *supra;* Lemm and Rice, (1993a) *supra;* Lemm and Rice, (1993b) *supra;* Lemm *et al.,* (1994) *supra; Pattnaik et al.,* (1992) *supra;* Pattnaik et al., Virology 206: 760-4 (1995); Porter et al., J. Virol. 69: 1548-1555 (1995)]. In brief, a vaccinia recombinant (vTF7-3) is used to express T7 RNA polymerase (T7RNApol) in the cell type of interest. Target cDNAs, positioned downstream from the T7 promoter, are delivered either as vaccinia recombinants or by plasmid transfection. This system leads to high level RNA and protein expression. A variation of this approach, which obviates the need for vaccinia (which could interfere with HCV RNA replication or virion formation), is the pT7T7 system where the T7 promoter drives expression of T7RNApol [Chen et al., Nucleic Acids Res. 22: 2114-2120. (1994)]. pT7T7 is mixed with T7RNApol (the protein) and co-transfected with the T7-driven target plasmid of interest. Added T7RNApol initiates transcription, leading to it own production and high level expression of the target gene. Using either approach, RNA transcripts of variants with precise 5' and 3' termini can be produced using the T7 transcription start site (5') and the cis-cleaving HCV ribozyme (Rz) (3`) [Ball, (1992) *supra;* Pattnaik *et al.,* (1992) *supra].*

These or similar expression systems can be used to establish assays for HCV RNA replication and particle formation using HCV variants, and for evaluation of compounds which might inhibit these processes. T7-driven protein expression constructs and full-length HCV variants incorporating the HCV ribozyme following the 3' NTR can also be used. A typical experimental plan to validate the assay as described for pT7T7, although essentially similar assays can be envisioned using vTF7-3 or cell lines expressing the T7 RNA polymerase. HCV-permissive cells are co-transfected with pT7T7+T7RNApol+p90/HCVFLlong pU Rz (or a negative control, such as ΔGDD). At different times post-transfection, accumulation of HCV proteins and RNAs, driven by the pT7T7 system, are followed by Western and Northern blotting, respectively. To assay for HCV-specific replicase function, actinomycin D is added to block DNA-dependent T7 transcription [Lemm and Rice, (1993a), *supra]* and actinomycin D-resistant RNA synthesis is monitored by metabolic labeling. Radioactivity will be incorporated into full-length HCV RNAs for p90/HCVFL long pU/Rz, but not for p90/HCVFLΔGDD/Rz. Using HCV variants of the invention, this assay system, or elaborated derivatives, can be used to screen for inhibitors and to study their effects on HCV RNA replication.

*Cell-free systems for assaying HCV replication and inhibitors thereof.* Cell-free assays for studying HCV RNA replication and inhibitor screening can also be established using the variants described in this invention. Either virion or transcribed RNAs are used as substrate RNA. For HCV, full-length HCV variant RNAs transcribed *in vitro* can be used to program such *in vitro* systems and replication assayed essentially as described for poliovirus [see Barton *et al.,* (1995) *supra].* In case hepatocyte-specific or other factors are required for HCV variant RNA replication, the system can be supplemented with hepatocyte or other cell extracts, or alternatively, a comparable system can be established using cell lines which have been shown to be permissive for replication of the HCV variants.

One concern about this approach is that proper cell-free synthesis and processing of the HCV polyprotein must occur. Sufficient quantities of properly processed replicase components may be difficult to produce. To circumvent this problem, the T7 expression system can be used to express high levels of HCV replicase components in appropriate cells [see Lemm *et al.,* (1997) *supra].* P15 membrane fractions from these cells (with added buffer, Mg²⁺, an ATP regenerating system, and NTPs) should be able to initiate and synthesize full-length negative-strand RNAs upon addition of HCV-specific template RNAs.

Establishment of either or both of the above assays allows rapid and precise analysis of the effects of HCV mutations, host factors, involved in replication and inhibitors of the various steps in HCV RNA replication. These systems will also establish the requirements for helper systems for preparing replication-deficient HCV vectors.

### Vaccination and Protective Immunity

There are still many unknown parameters that impact on development of effective HCV vaccines. It is clear in both man and the chimpanzee that some individuals can clear the infection. Also, 10-20% of those treated with IFN or about twice this percentage treated with IFN and ribavirin show a sustained response as evidenced by lack of circulating HCV RNA. Other studies have shown a lack of protective immunity, as evidenced by successful reinfection with both homologous virus as well as with more distantly related HCV types [Farci *et al.,* (1992) *supra;* Prince *et al.,* (1992) *supra].* Nonetheless, chimpanzees immunized with subunit vaccines consisting of E1E2 oligomers and vaccinia recombinants expressing these proteins are partially protected against low dose challenges [Choo et al., Proc. Natl. Acad. Sci. USA 91:1294 (1994)]. The HCV variant technology described in this invention has utility not only for basic studies aimed at understanding the nature of protective immune responses against HCV, but also for novel vaccine production methods.

Active immunity against HCV can be induced by immunization (vaccination) with an immunogenic amount of an attenuated or inactivated HCV variant virion, or HCV virus particle proteins, preferably with an immunologically effective adjuvant. An "immunologically effective adjuvant" is a material that enhances the immune response.

Selection of an adjuvant depends on the subject to be vaccinated. Preferably, a pharmaceutically acceptable adjuvant is used. For example, a vaccine for a human should avoid oil or hydrocarbon emulsion adjuvants, including complete and incomplete Freund's adjuvant. One example of an adjuvant suitable for use with humans is alum (alumina gel). A vaccine for an animal, however, may contain adjuvants not appropriate for use with humans.

An alternative to a traditional vaccine comprising an antigen and an adjuvant involves the direct *in vivo* introduction of DNA or RNA encoding the antigen into tissues of a subject for expression of the antigen by the cells of the subject's tissue. Such vaccines are termed herein genetic vaccines, DNA vaccines, genetic vaccination, or nucleic acid-based vaccines. Methods of transfection as described above, such as DNA vectors or vector transporters, can be used for DNA vaccines.

DNA vaccines are described, e.g., in International Patent Publication WO 95/20660 and International Patent Publication WO 93/19183. The ability of directly injected DNA that encodes a viral protein or genome to elicit a protective immune response has been demonstrated in numerous experimental systems [Conry et al., Cancer Res., 54:1164-1168 (1994); Cox et al., Virol, 67:5664-5667 (1993); Davis et al., Hum. Mole. Genet., 2:1847-1851 (1993); Sedegah et al., Proc. Natl. Acad. Sci., 91:9866-9870 (1994); Montgomery et al., DNA Cell Bio., 12:777-783 (1993); Ulmer et al., Science, 259:1745-1749 (1993); Wang et al., Proc. Natl. Acad Sci., 90:4156-4160 (1993); Xiang et al., Virology, 199:132-140 (1994)]. Studies to assess this strategy in neutralization of influenza virus have used both envelope and internal viral proteins to induce the production of antibodies, but in particular have focused on the viral hemagglutinin protein (HA) [Fynan et al., DNA Cell. Biol., 12:785-789 (1993A); Fynan et al., Proc. Natl. Acad. Sci., 90:11478-11482 (1993B); Robinson et al., Vaccine, 11:957, (1993); Webster et al., Vaccine, 12:1495-1498 (19,94)].

Vaccination through directly injecting DNA or RNA that encodes a protein to elicit a protective immune response produces both cell-mediated and humoral responses. This is analogous to results obtained with live viruses [Raz et al., Proc. Natl. Acad. Sci., 91:9519-9523 (1994); Ulmer, 1993, *supra;* Wang, 1993, *supra;* Xiang, 1994, *supra].* Studies with ferrets indicate that DNA vaccines against conserved internal viral proteins of influenza, together with surface glycoproteins, are more effective against antigenic variants of influenza virus than are either inactivated or subvirion vaccines [Donnelly et al., Nat.Medicine, 6:583-587 (1995)]. Indeed, reproducible immune responses to DNA encoding nucleoprotein have been reported in mice that last essentially for the lifetime of the animal [Yankauckas et al., DNA Cell Biol., 12: 771-776 (1993)].

A vaccine can be administered via any parenteral route, including but not limited to intramuscular, intraperitoneal, intravenous, intraarterial (*e.g*., Ripatic artery) and the like. Preferably, since the desired result of vaccination is to elucidate an immune response to HCV, administration directly, or by targeting or choice of a viral vector, indirectly, to lymphoid tissues, *e.g*., lymph nodes or spleen. Since immune cells are continually replicating, they are ideal target for retroviral vector-based nucleic acid vaccines, since retroviruses require replicating cells.

Passive immunity can be conferred to an animal subject suspected of suffering an infection with HCV by administering antiserum, neutralizing polyclonal antibodies, or a neutralizing monoclonal antibody against HCV to the patient. Although passive immunity does not confer long-term protection, it can be a valuable tool for the treatment of an acute infection of a subject who has not been vaccinated. Preferably, the antibodies administered for passive immune therapy are autologous antibodies. For example, if the subject is a human, preferably the antibodies are of human origin or have been "humanized," in order to minimize the possibility of an immune response against the antibodies. In addition, genes encoding neutralizing antibodies can be introduced in vectors for expression *in vivo, e.g.,* in hepatocytes.

*Antibodies for passive immune therapy.* Preferably, HCV variant virions or virus particle proteins prepared as described above are used as an immunogen to generate antibodies that recognize HCV. The variants utilized should have wild-type coat Such antibodies include but are not limited to polyclonal, monoclonal, chimeric, single chain, Fab fragments, and an Fab expression library. Various procedures known in the art may be used for the production of polyclonal antibodies to HCV. For the production of antibody, various host animals can be immunized by injection with the HCV virions or polypeptide, *e.g*., as describe infra, including but not limited to rabbits, mice, rats, sheep, goats, etc. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG *(bacille Calmette-Guerin)* and *Corynebacterium parvum.*

For preparation of monoclonal antibodies directed toward HCV as described above, any technique that provides for the production of antibody molecules by continuous cell lines in culture may be used. These include but are not limited to the hybridoma technique originally developed by Kohler and Milstein [Nature 256:495-497 (1975)], as well as the trioma technique, the human B-cell hybridoma technique [Kozbor et al., Immunology Today 4:72 1983); Cote et al,, Proc. Natl. Acad. Sci. U.S.A. 80:2026-2030 (1983)], and the EBV-hybridoma technique to produce human monoclonal antibodies [Cole et al., in Monoclon.al Antibodies and Cancer Therapy, Alan R. Liss, Inc., pp. 77-96 (1985)]. In an additional embodiment of the invention, monoclonal antibodies can be produced in germ-free animals [International Patent Publication No. WO 89/12690, published 28 December 1989]. In fact, according to the invention, techniques developed for the production of "chimeric antibodies" [Morrison et al., J. Bacteriol. 159:870 (1984); Neuberger et al., Nature 312:604-608 (1984); Takeda et al., Nature 314:452-454 (1985)] by splicing the genes from a mouse antibody molecule specific for HCV together with genes from a human antibody molecule of appropriate biological activity can be used; such antibodies are within the scope of this invention. Such human or humanized chimeric antibodies are preferred for use in therapy of human diseases or disorders (described *infra),* since the human or humanized antibodies are much less likely than xenogenic antibodies to induce an immune response, in particular an allergic response, themselves.

According to the invention, techniques described for the production of single chain antibodies [U.S. Patent Nos. 5,476,786 and 5,132,405 to Huston; U.S. Patent 4,946,778] can be adapted to produce HCV-specific single chain antibodies. An additional embodiment of the invention utilizes the techniques described for the construction of Fab expression libraries [Huse et al., Science 246:1275-1281 (1989)] to allow rapid and easy identification of monoclonal Fab fragments with the desired specificity.

Antibody fragments containing the idiotype of the antibody molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent.

*HCV particles for subunit vaccination.* The functional HCV variants of the present invention can be used to produce HCV-like particles for vaccination. Proper glycosylation, folding, and assembly of HCV particles may be important for producing appropriately antigenic and protective subunit vaccines. Several methods can be used for particle production. They include engineering of stable cell lines for inducible or constitutive expression of HCV-like particles (using bacterial, yeast or mammalian cells), or the use of higher level eukaryotic heterologous expression systems such as recombinant baculoviruses, vaccinia viruses [Moss, Proc. Natl. Acad. Sci. U.S.A. 93: 11341-11348 (1996)], or alphaviruses [Frolov *et al.,* (1996) *supra].* HCV particles for immunization may be purified from either the media or disrupted cells, depending upon their localization. Such purified HCV particles or mixtures of particles representing a spectrum of HCV genotypes, can be injected with our without various adjuvants to enhance immunogenicity.

*Infectious non-replicating HCV particles.* In another manifestation, particles of HCV variants capable of receptor binding, entry, and translation of genome RNA can be produced. Heterologous expression approaches for production of such particles include, but are not restricted to, *E. coli,* yeast, or mammalian cell lines, appropriate host cells infected or harboring recombinant baculoviruses, recombinant vaccinia viruses, recombinant alphaviruses or RNA replicons, or recombinant adenoviruses, engineered to express appropriate HCV RNAs and proteins. In one example, two recombinant baculoviruses are engineered. One baculovirus expresses the HCV structural proteins (*e.g*. C-E1-E2-p7) required for assembly of HCV particles. A second recombinant expresses the entire HCV genome RNA, with precise 5' and 3' ends, except that a deletion, such as ΔGDD or GDD→AAG (see example 1), is included to inactivate the HCV NS5B RDRP. Other mutations abolishing productive HCV replication could also be utilized instead or in combination. Cotransfection of appropriate host cells (Sf9, Sf21, etc.) with both recombinants will produce high levels of HCV structural proteins and genome RNA for packaging into HCV-like particles. Such particles can be produced at high levels, purified, and used for vaccination. Once introduced into the vaccinee, such particles will exhibit normal receptor binding and infection of HCV-susceptible cells. Entry will occur and the genome RNA will be translated to produce all of the normal HCV antigens, except that further replication of the genome will be completely blocked given the inactivated NS5B polymerase. Such particles are expected to elicit effective CTL responses against structural and nonstructural HCV protein antigens. This vaccination strategy alone or preferably in conjunction with the subunit strategy described above can be used to elicit high levels of both neutralizing antibodies and CTL responses to help clear the virus. A variety of different HCV genome RNA sequences can be utilized to ensure broadly cross-reactive and protective immune responses. In addition, modification of the HCV particles, either through genetic engineering, or by derivatization *in vitro,* could be used to target infection to cells most effective at eliciting protective and long lasting immune responses.

*Live-attenuated HCV derivatives. -* The ability to manipulate the HCV genome RNA sequence and thereby produce mutants with altered pathogenicity provides a means of constructing live-attenuated HCV variants appropriate for vaccination. Such vaccine candidates express protective antigens but would be impaired in their ability to cause disease, establish chronic infections, trigger autoimmune responses, and transform cells.

Additionally, viruses propagated in cell culture frequently acquire mutations in their RNA genomes that display attenuated phenotypes *in vivo,* while still retaining their immunogenicity. Attenuated virus strains would be impaired in their ability to cause disease and establish chronic infections. Production of HCV variants adapted for tissue culture may represent potential candidates for live-attenuated vaccines. An attractive possibility is the production of HCV derivatives containing the deletion in NS5A described in this application as clone I (see Example 1). Such a variant is less likely to revert to wild type in the host.

### HCV Variant-based Gene Expression Vectors

Some of the same properties of HCV leading to chronic liver infection of humans may also be of great utility for designing vectors for gene expression in cell culture systems, genetic vaccination, and gene therapy. The HCV variants described herein can be engineered to produce chimeric RNAs designed for the expression of heterologous gene products (RNAs and proteins). Strategies have been described above and elsewhere [Bredenbeek and Rice, (1992) *supra;* Frolov *et al.,* (1996) *supra]* and include, but are not limited to (i) in-frame fusion of the heterologous coding sequences with the HCV polyprotein; (ii) creation of additional cistrons in the HCV genome RNA; and (iii) inclusion of IRES elements to create multicistronic self-replicating HCV vector RNAs capable of expressing one or more heterologous genes (Figure 2). Functional HCV RNA backbones utilized for such vectors include, but are not limited to, (i) live-attenuated derivatives capable of replication and spread; (ii) RNA replication competent "dead end" derivatives lacking one or more viral components (*e.g*. the structural proteins) required for viral spread; (iii) mutant derivatives capable of high and low levels of HCV-specific RNA synthesis and accumulation; (iv) mutant derivatives adapted for replication in different human cell types; (v) engineered or selected mutant derivatives capable of prolonged noncytopathic replication in human cells. Vectors competent for RNA replication but not packaging or spread can be introduced either as naked RNA, DNA, or packaged into virus-like particles. Such virus-like particles can be produced as described above and composed of either unmodified or altered HCV virion components designed for targeted transfection of the hepatocytes or other human cell types. Alternatively, HCV RNA vectors can be encapsidated and delivered using heterologous viral packaging machineries or encapsulated into liposomes modified for efficient gene delivery. These packaging strategies, and modifications thereof, can be utilized to efficiently target HCV vector RNAs to specific cell types. Using methods detailed above, similar HCV-derived vector systems, competent for replication and expression in other species, can also be derived.

Various methods, *e.g*., as set forth *supra* in connection with transfection of cells and DNA vaccines, can be used to introduce an HCV vector of the invention. Of primary interest is direct injection of functional HCV RNA or virions, *e.g*., in the liver. Targeted gene delivery is described in International Patent Publication WO 95/28494, published October 1995. Alternatively, the vector can be introduced *in vivo* by lipofection. For the past decade, there has been increasing use of liposomes for encapsulation and transfection of nucleic acids *in vitro.* Synthetic cationic lipids designed to limit the difficulties and dangers encountered with liposome mediated transfection can be used to prepare liposomes for *in vivo* transfection of a gene encoding a marker [Felgner, et. al., Proc. Natl. Acad. Sci. U.S.A. 84:7413-7417 (1987); *see* Mackey, et al., Proc. Natl. Acad. Sci. U.S.A. 85:8027-8031 (1988); Ulmer et al., Science 259:1745-1748 (1993)]. The use of cationic lipids may promote encapsulation of negatively charged nucleic acids, and also promote fusion with negatively charged cell membranes [Felgner and Ringold, Science 337:387-388 (1989)]. The use of lipofection to introduce exogenous genes into the specific organs *in vivo* has certain practical advantages. Molecular targeting of liposomes to specific cells represents one area of benefit. It is clear that directing transfection to particular cell types would be particularly advantageous in a tissue with cellular heterogeneity, such as pancreas, liver, kidney, and the brain. Lipids may be chemically coupled to other molecules for the purpose of targeting [*see* Mackey, et. al., *supra].* Targeted peptides, *e.g*., hormones or neurotransmitters, and proteins such as antibodies, or non-peptide molecules could be coupled to liposomes chemically. Receptor-mediated DNA delivery approaches can also be used [Curiel et al., Hum. Gene Ther. 3:147-154 (1992); Wu and Wu, J. Biol. Chem. 262:4429-4432 (1987)].

Examples of applications for gene therapy include, but are not limited to, (i) expression of enzymes or other molecules to correct inherited or acquired metabolic defects; (ii) expression of molecules to promote wound healing; (iii) expression of immunomodulatory molecules to promote immune-mediated regression or elimination of human cancers; (iv) targeted expression of toxic molecules or enzymes capable of activating cytotoxic drugs in tumors; (v) targeted expression of anti-viral or anti-microbial agents in pathogen-infected cells. Various therapeutic heterologous genes can be inserted in a gene therapy vector of the invention, such as but not limited to adenosine deaminase (ADA) to treat severe combined immunodeficiency (SCID); marker genes or lymphokine genes into tumor infiltrating (TIL) T cells [Kasis et al., Proc. Natl. Acad. Sci. U.S.A. 87:473 (1990); Culver et al., ibid. 88:3155 (1991)]; genes for clotting factors such as Factor VIII and Factor IX for treating hemophilia [Dwarki et al.Proc. Natl. Acad Sci. USA, 92:1023-1027 (19950); Thompson, Thromb. and Haemostatis, 66:119-122 (1991)]; and various other well known therapeutic genes such as, but not limited to, -globin, dystrophin, insulin, erythropoietin, growth hormone, glucocerebrosidase, -glucuronidase, α-antitrypsin, phenylalanine hydroxylase, tyrosine hydroxylase, ornithine transcarbamylase, apolipoproteins, and the like. In general, see U.S. Patent No. 5,399,346 to Anderson et al.

Examples of applications for genetic vaccination (for protection from pathogens other than HCV) include, but are not limited to, expression of protective antigens from bacterial (*e.g*., uropathogenic *E. coli, Streptoccoci, Staphlococci, Nisseria),* parasitic *(e.g., Plasmodium, Leishmania, Toxoplama),* fungal *(e.g., Candida, Histoplasma) ,* and viral (*e*.*g*., HIV, HSV, CMV, influenza) human pathogens. Immunogenicity of protective antigens expressed using HCV-derived RNA expression vectors can be enhanced using adjuvants, including co-expression of immunomodulatory molecules, such as cytokines (*e.g*., IL-2, GM-CSF) to facilitate development of desired Th1 versus Th2 responses. Such adjuvants can be either incorporated and co-expressed by HCV vectors themselves or administered in combination with these vectors using other methods.

### Diagnostic Methods for Infectious HCV

*Diagnostic cell lines.* The invention described herein can also be used to derive cell lines for sensitive diagnosis of infectious HCV in patient samples. In concept, functional HCV components are used to test and create susceptible cell lines (as identified above) in which easily assayed reporter systems are selectively activated upon HCV infection. Examples include, but are not restricted to, (i) defective HCV RNAs lacking replicase components that are incorporated as transgenes and whose replication is upregulated or induced upon HCV infection; and (ii) sensitive heterologous amplifiable reporter systems activated by HCV infection. In the first manifestation, RNA signals required for HCV RNA amplification flank a convenient or a selectable marker (see above). Expression of such chimeric RNAs is driven by an appropriate nuclear promoter and elements required for proper nuclear processing and transport to the cytoplasm. Upon infection of the engineered cell line with HCV, cytoplasmic replication and amplification of the transgene is induced, triggering higher levels of reporter expression, as an indicator of productive HCV infection.

In the second example, cell lines are designed for more tightly regulated but highly inducible reporter gene amplification and expression upon HCV infection. Although this amplfied system is described in the context of specific components, other equivalent components can be used. In one such system, an engineered alphavirus replicon transgene is created which lacks the alphavirus nsP4 polymerase, an enzyme absolutely required for alphavirus RNA amplification and normally produced by cleavage from the nonstructural polyprotein. Additional features of this defective alphavirus replicon include a subgenomic RNA promoter, driving expression of a luciferase or GFP reporter gene. This promoter element is quiescent in the absence of productive cytoplasmic alphavirus replication. The cell line contains a second transgene for expression of gene fusion consisting of the HCV NS4A protein and the alphavirus nsP4 RDRP. This fused gene is expressed and targeted to the cytoplasmic membrane compartment, but this form of nsP4 would be inactive as a functional component of the alphavirus replication complex because a discrete nsP4 protein, with a precise N terminus is required for nsP4 activity [Lemm et al., EMBO J. 13:2925 (1994)]. An optional third transgene expresses a defective alphavirus RNA with *cis* signals for replication, transcription of subgenomic RNA encoding a ubiquitin-nsP4 fusion, and an alphavirus packaging signal. Upon infection of such a cell line by HCV, the HCV NS3 proteinase is produced, mediating *trans* cleavage of the NS4A-nsP4 fusion protein, activating the nsP4 polymerase. This active polymerase, which functions in *trans* and is effective in minute amounts, then forms a functional alphavirus replication complex leading to amplification of the defective alphavirus replicon as well as the defective alphavirus RNA encoding ubiquitin-nsP4. Ubiquitin-nsP4, expressed from its subgenomic RNA, is cleaved efficiently by cellular ubiquitin carboxyterminal hydrolase to product additional nsP4, in case this enzyme is limiting. Once activated, this system would produce extremely high levels of the reporter protein. The time scale of such an HCV infectivity assay is expected to be from hours (for sufficient reporter gene expression).

*Antibody diagnostic.* In addition to the cell lines described here, HCV variant virus particles (virions) or components thereof, produced by the transfected or infected cell lines, or isolated from an inflected animal, may be used as antigens to detect anti-HCV antibodies in patient blood or blood products. Because the HCV variant virus particles are derived from an authentic HCV genome, particular components such as the coat proteins are likely to have immunogenic properties that more closely resemble or are identical to natural HCV virus than if those components were produced outside of a replicating HCV. Examples of such immunogenic properties include the display of wild-type HCV immunogenic epitopes, and modulation of transcription of genes encoding cellular immune-modulating cytokines. These reagents can be used to establish that a patient is infected with HCV by detecting seroconversion, i.e., generation of a population of HCV-specific antibodies.

Alternatively, antibodies generated to the HCV variant products prepared as described herein can be used to detect the presence of HCV in biological samples from a subject.

Preferred embodiments of the invention are described in the following example. Other embodiments within the scope of the claims herein will be apparent to one skilled in the art from consideration of the specification or practice of the invention as disclosed herein. It is intended that the specification, together with the examples, be considered exemplary only, with the scope of the invention being indicated by the claims which follow the examples.

### Example 1

This example describes the production and evaluation of replicons comprising a *neo* selectable marker and a polyprotein coding region encoding subtype 1b nonstructural proteins.

### Materials and Methods

**Cell lines.** The Huh7 cell lines were generously provided by Robert Lanford (Southwest Foundation for Biomedical Research, San Antonio, U.S.A.) and Ralf Bartenschlager (Johannes Gutenberg University Mainz, Mainz, Germany) and maintained in Dulbecco's modified minimal essential media (DMEM; Gibco-BRL) supplemented with 10% fetal calf serum (FCS), and nonessential amino acids.

**Assembly of a selectable subtype 1b replicon.** An HCV subtype 1b replicon was constructed which is similar to the replicon described in Lohmann et al., Science 285:110-113 (1999). For that construction, a step-wise PCR-based assay utilizing KlenTaqLA DNA polymerase (Wayne Barnes, Washington University) was developed. cDNAs spanning 600-750 bases in length were assembled from 10-12 gel-purified oligonucleotides (60-80 nucleotides in length) with unique complementary overlaps of 16 nucleotides. Four or six oligonucleotides representing the 5' portion of the region to be assembled were annealed and extended in a standard PCR. The remaining six oligonucleotides for the synthesis of the 3' half of the intended cDNA were mixed in a parallel PCR reaction. After 12 cycles of PCR, the extended double-stranded DNA products were combined and subjected to an additional 12 cycles. The product of this reaction resolved as a smear on agarose gels which was excised and the DNA isolated from the agarose. One-fifth of the purified double-stranded DNA product was amplified by PCR using an outer primer pair containing unique restriction enzyme sites to facilitate directional cloning into the pGEM3Zf(+) plasmid vector (Promega). PCR products were purified, digested with appropriate restriction enzymes, and ligated into similarly cleaved pGEM3Zf(+). Multiple recombinant clones were sequenced and the correct clones identified. The overlapping cDNA fragments were assembled into the contiguous replicon sequence. In parallel, a replicon carrying the lethal mutation in the NS5B active site (Gly-Asp-Asp [GDD] to Ala-Ala-Gly [AGG]; pol-) was constructed.

**RNA transcription and transfection.** RNA transcripts were synthesized in a 100µl reaction mixture containing 40mM Tris-HCl (pH 7.9), 10mM NaCl, 12mM MgCl₂, 2mM spermidine, 3mM each ATP, CTP, GTP and UTP, 10mM dithiothreitol, 100 U RNasin (Promega) and 100 U T7 RNA polymerase (Epicentre), and 2g *Sca I*-linearized DNA. The DNA template was rigorously removed by serial digestions with 30 U DNase I (Boehringer). Ten µg of the DNase-digested RNA transcripts were electroporated into 6x 10⁶ Huh7 cells using a model T820 squareporator (BTX), and plated on 150mm dishes. For selection of replicon-containing cells, medium was changed to complete medium containing geneticin (G418; 1mg/ml; Gibco-BRL) at 24 hr post-transfection and thereafter the media was changed every 3-4 days.

**RNA analysis.** Approximately 5x 10⁵ cells were preincubated for 1 h in DMEM lacking phosphate supplemented with 5% dialyzed FCS, 1/20^{th} the normal concentration of phosphate and actinomycin D (4µg/ml; Sigma). [³²P]orthophosphate (200µCi/ml; ICN) was added and the incubation continued for an additional 12 h. Total cellular RNA was extracted with TRIZOL, precipitated, and resuspended in H₂0 (Gibco-BRL). Radiolabeled RNA was analyzed by denaturing agarose gel electrophoresis and visualized by autoradiography.

**Protein analysis.** For immunoprecipitation, cell monolayers were incubated for either 4, 8 or 12 h in methionine- and cysteine-deficient MEM containing 1/40^{th} the normal concentration of methionine, 5% dialyzed FCS and Express ³⁵S³⁵S protein labeling mix (100µCi/ml; NEN). Cells were lysed in 100mM NaPO₄ pH 7.0 containing 1% sodium dodecyl sulfate (SDS) and protease inhibitors, and cellular DNA sheared by repeated passage through a 27.5 gauge needle. Viral proteins were immunoprecipitated essentially as described **previously (Grakoui *etal,* 1993), using patient serum, JHF, recognizing NS3, NS4B and NS5A or rabbit anti-NS5B and Pansorbin cells (Calbiochem). Immunoprecipitates were separated on 10% SDS-PAGE and visualized by autoradiography.**

**Immunostaining.** Cells cultured in 8 well chamber slides (Falcon) were fixed in acetone for 10min at 4°C and allowed to air dry. Rehydrated monolayers were incubated at 37°C with an antibody directed against NS3, followed by incubation with a species-specific fluorescein-conjugated secondary antibody (Pierce), and mounted in 90% glycerol saline containing 50mM Tris-HCl (pH 8.8).

**Reverse transcription (RT)-PCR.** RNA was isolated from cells using TRIZOL (Gibco-BRL), precipitated and resuspended in H₂0. Levels of HCV RNA were quantitated using competitive RT-PCR assays designed to amplify the 5' and 3' NTR sequences of HCV (Kolykhalov *et al,* 1996). For RT-PCR designed to amplify long cDNA fragments, about 1000 molecules of HCV RNA was mixed with the HCV-specific primer, and the primer extended at 43.5°C for 1 h using Superscript II reverse transcriptase (Gibco-BRL). cDNAs were then amplified with KlenTaqLA DNA polymerase using 35 cycles of 95°C for 30 s, 55-60°C for 30 s, and 68°C for 4 min. PCR products were recovered from preparative low melting-point agarose electrophoresis by phenol extraction, and ~40ng of purified PCR product directly sequenced.

### Results

**Establishment of G418-resistant colonies.** Replicons similar to that described in Lohmann *et al, supra,* but derived from the H77 infectious clone, failed to confer resistance to G418 in five different hepatoma cell lines. Sequences of subtype 1b were also used to assemble the replicon I₃₇₇/NS3-3' (EMBL accession number AJ242652). Replicon RNAs were composed of the HCV internal ribosome entry site (IRES) driving neomycin phosphotransferase gene (Neo) expression and the IRES from encephalomyocarditis virus (EMCV), directing translation of HCV proteins NS3 to NS5B, followed by the 3'NTR ) (Figure 3). Two derivatives were constructed which either lacked 2 U nucleotides in the poly (U/UC) tract or carried an *Ava*II restriction enzyme site in the variable region of the 3' NTR, designated HCVrep1bBartMan/Δ2U's and HCVreplbBartMan/AvaII, respectively. Prior to transfection, translation and correct polyprotein processing was confirmed for each cDNA sequence using the vaccinia-T7 RNA polymerase expression system (data not shown).

DNase-treated replicon RNAs were electroporated into Huh7 cells and after 2-3 weeks in culture G418-resistant colonies were clearly visible. Both replicon derivatives were able to confer G418 resistance, and on average, only 1 in 10⁶ cells became G418 resistant. In contrast, colonies were never observed for Huh7 cells electroporated in parallel with the replicon RNAs containing an inactive NS5B polymerase.

**Verification of autonomous replication.** Twenty two independent colonies were isolated, 5 colonies corresponded to Huh7 cells transfected with RNA transcribed from HCVrep1bBartMan/Δ2U's and the remaining 17 colonies were derived from HCVrep1bBart/Man/AvaI RNA. A number of assays were performed to verify that G418 resistance was mediated by autonomously replicating HCV. Amplification of sequences within the 5' and 3' NTRs in a quantitative RT-PCR assay revealed copy numbers ranging from 50 to 5000 HCV RNA molecules per cell (Figure 4). ³²P-labeled, actinomycin D-resistant RNA of the expected size was observed in the four independent G418-resistant cell clones analyzed (Figure 5A). The HCV proteins, NS3, NS4B, NS5A and NS5B, were immunoprecipitated from radiolabeled cell lysates (Figure 5B). In addition, immunostaining of cell monolayers revealed a punctate staining pattern for NS3 within the cytoplasm (Figure 6), similar to HCV protein localization observed in liver sections from HCV-infected patients (Blight and Gowans, 1996). In G418-resistant cell clones the fluorescent signal tended to vary between cells, probably reflecting the different levels of replication per cell.

**Identification of mutations in HCV replicons.** The low frequency of G418-resistant colonies may be attributed to either a cell factor(s) requirement for replication or adaptive changes within the replicon sequence necessary for the establishment of HCV replication. To address the latter possibility, the entire replicon sequence was amplified from cDNA reverse transcribed from RNA isolated from five independent G418-resistant cell clones. Upon direct sequencing of the purified PCR population, multiple mutations were identified. The striking observation was that each cell clone carried a single nucleotide change within NS5A resulting in a coding change (Figure 7). In one instance, a deletion of 47 amino acids (I; Figure 7), encompassing the interferon sensitivity determining region (ISDR), was found. Sequence analysis of NS5A from another 8 G418-resistant cell clones revealed similar point mutations, although 2 clones, which have low levels of HCV replication and slow growth rates (e.g., clone E in Figure 4), were found to contain wild type NS5A. In addition to the identified NS5A mutations, nucleotide substitutions were also noted in NS3 and NS4B; Clone II (SEQ ID NO:9) contains substitutions at nt 3550 (NS3) and nt 4573 (NS4B) (Lys (584) to Glu, and Ser(925) to Gly of SEQ ID NO:3, embodied in SEQ ID NO:17), whereas nt 2060 (NS3) was mutated in Clone VI (Figure 7, corresponding to Gln (87) to Arg of SEQ ID NO:3, embodied in SEQ ID NO:15).

**Reconstruction of mutant replicons.** To determine if the nucleotide changes and the deletion identified in NS5A were adaptive, each mutation, except mutation II, was independently engineered back into the HCVrep1bBartMan/AvaII backbone. RNA transcribed from each reconstructed replicon was electroporated into naive Huh7 cells, and the number of G418-resistant colonies compared to that obtained for the HCVrep1bBartMan/AvaII replicon containing wild type NS5A. The 47 amino acid deletion, as well as the point mutations, were capable of increasing the frequency of G418-resistant colonies to at least 1% of the initial electroporated cell population (Figure 8), indicating these mutations targeting NS5A are adaptive allowing efficient HCV replication in Huh7 cells. In addition, G418-resistant colonies were observed after transfection of HeLa cells, a human epithelial cell line, with replicon RNA of clone I. Therefore, at least one of the mutations that was adaptive in Huh7 cells also allows the establishment of HCV replication in a non-hepatic cell line.

### Example 2

This example describes the production of cell lines permissive for HCV replication; a replicon comprising the NS2 coding region; and full-length HCV cDNA clones comprising the Ser to Ile substitution at position 1179 of SEQ ID NO: 3.

**Generation of cell lines.** As shown in the previous example, G418-resistant cell clones harboring persistently replicating HCV RNAs were isolated. Two of these G418-resistant cell clones were treated extensively with the antiviral, interferon-α, to obtain 2 cell lines void of HCV RNA. These are refered to as interferon-treated cell lines I and II.

HCVrep1bBartMan/AvaII, HCV adaptive replicon I or HCV adaptive replicon VII were transfected into the interferon-treated cell lines, I and II. This resulted in a greater G418 transduction efficiency than that observed for the parental Huh-7 cells (see Table 1). Early post-transfection HCV RNA amplification was greatest for the IFN-treated cell line. These results indicate that the cell lines, interferon-treated cell lines I and II, are more permissive for HCV replication than is the parental Huh-7 cell line.

**Table 1: Relative G418 transduction efficiencies of HCV replicons after transfection into interferon-treated cell clones**

| | Transfected replicon | | |
|---|---|---|---|
| Cell Line | Bartman | I | VII |
| Parental Huh-7 | 0.0005% | 0.15% | 9% |
| IFN-treated I | 0.005% | 5% | 30% |
| IFN-treated II | 0.001% | 1.3% | 11% |

Such cell lines are not only valuable for genetic study of HCV, but also for examining the cellular environments more permissive for HCV replication. For example, microarray technology will allow us to look globally at differences in gene expression profiles between the different cell lines.

**Construction of replicons.** A replicon was constructed wherein the 5'NTR of HCV was fused to the IRES of EMCV upstream of NS3, thus creating a replicon lacking the neomycin phosphotransferase gene. This replicon, 5'NTR-EMCV/HCVrepVII (SEQ ID NO:25), replicates to high levels in Huh7 cells, as shown in Figure 10. Another replicon, HCVrep/NS2-5B (SEQ ID NO:22) was made wherein the non-structural protein, NS2, is upstream of NS3. As shown in Figure 10, this replicon is also replication-competent in Huh7 cells. This latter replicon can be used advantageously, for example, in testing compounds for inhibiting HCV replication. The addition of the NS2 coding region provides an additional target for such antiviral compounds, as well as providing an additional protein for genetic study.

**Full-length HCV RNAs.** Two full-length HCV cDNA clones were assembled. The first, HCV FL (SEQ ID NO:24), contains the mutation that encodes a Ser to Ile substitution in NS5A, as shown at position 1179 of SEQ ID NO:3 (see Figure 9). The second, HCV FL-Neo (SEQ ID NO:23), also encodes the Ser to Ile mutation, and in addition, comprises the neomycin phosphotransferase gene immediately 3' of the 5' NTR and the EMCV IRES immediately 5' to the HCV open reading frame (see Figure 9). Both of these full-length clones replicate in the interferon-treated cell line I, as shown in Figure 10. This result indicates that HCV replication is not dependent on the EMCV IRES driving the non-structural proteins of HCV, because the non-structural proteins of the HCV FL clone are driven by the HCV IRES in the full-length clone HCV FL.

In addition, a G418 resistant cell line comprising the HCV FL-Neo clone has been generated from the interferon-treated cell line I described above. This cell line supports high levels of persistently replicating HCV FL-Neo RNA.

### Appendix

### SEQ QID NOs

SEQ ID NO:1: 5' portion of an HCV 5' NTR GGCGACACTC CACCATAGAT C

SEQ ID NO:2: 3' portion of a 3' NTR from a wild-type HCV subtype la

TGGTGGCTCCATCTTAGCCCTAGTCACGGCTAGCTGTGAAAGGTCCGTGAGCCGC
ATGACTGCAGAGAGTGCTGATACTGGCCTCTCTGCTGATCATGT

SEQ ID NO:3: Amino acid sequence of the polyprotein region of HCVrep1bBartMan

MAPITAYSQQTRGLLGCIITSLTGRDRNQVEGEVQVVSTATQSFLATCVNGVCWTVY
HGAGSKTLAGPKGPITQMYTNVDQDLVGWQAPPGARSLTPCTCGSSDLYLVTRHAD
VIPVRRRGDSRGSLLSPRPVSYLKGSSGGPLLCPSGHAVGIFRAAVCTRGVAKAVDFV
PVESMETTMRSPVFTDNSSPPAVPQTFQVAHLHAPTGSGKSTKVPAAYAAQGYKVL
VLNPSVAATLGFGAYMSKAHGIDPNIRTGVRTITTGAPITYSTYGKFLADGGCSGGAY
DIIICDECHSTDSTTILGIGTVLDQAETAGARLVVLATATPPGSVTVPHPNIEEVALSST
GEIPFYGKAIPIETIKGGRHLIFCHSKKKCDELAAKLSGLGLNAVAYYRGLDVSVIPTS
GDVIVVATDALMTGFTGDFDSVIDCNTCVTQTVDFSLDPTFTIETTTVPQDAVSRSQR
RGRTGRGRMGIYRFVTPGERPSGMFDSSVLCECYDAGCAWYELTPAETSVRLRAYL
NTPGLPVCQDHLEFWESVFTGLTHIDAHFLSQTKQAGDNFPYLVAYQATVCARAQA
PPPSWDQMWKCLIRLKPTLHGPTPLLYRLGAVQNEVTTTHPITKYIMACMSADLEVV
TSTWVLVGGVLAALAAYCLTTGSVVIVGRIILSGKPAIIPDREVLYREFDEMEECASH
LPYIEQGMQLAEQFKQKAIGLLQTATKQAEAAAPVVESKWRTLEAFWAKHMWNFIS
GIQYLAGLSTLPGNPAIASLMAFTASITSPLTTQHTLLFNILGGWVAAQLAPPSAASAF
VGAGIAGAAVGSIGLGKVLVDILAGYGAGVAGALVAFKVMSGEMPSTEDLVNLLPA
ILSPGALVVGVVCAAILRRHVGPGEGAVQWMNRLIAFASRGNHVSPTHYVPESDAA
ARVTQILSSLTITQLLKRLHQWINEDCSTPCSGSWLRDVWDWICTVLTDFKTWLQSK
LLPRLPGVPFFSCQRGYKGVWRGDGIMQTTCPCGAQITGHVKNGSMRIVGPRTCSNT
WHGTFPINAYTTGPCTPSPAPNYSRALWRVAAEEYVEVTRVGDFHYVTGMTTDNVK
CPCQVPAPEFFTEVDGVRLHRYAPACKPLLREEVTFLVGLNQYLVGSQLPCEPEPDV
AVLTSMLTDPSHITAETAKRRLARGSPPSLASSSASQLSAPSLKATCTTRHDSPDADLI
EANLLWRQEMGGNITRVESENKVVILDSFEPLQAEEDEREVSVPAEILRRSRKFPRAM
PIWARPDYNPPLLESWKDPDYVPPVVHGCPLPPAKAPPIPPPRRKRTVVLSESTVSSAL
AELATKTFGSSESSAVDSGTATASPDQPSDDGDAGSDVESYSSMPPLEGEPGDPDLSD
GSWSTVSEEASEDVVCCSMSYTWTGALITPCAAEETKLPINALSNSLLRHHNLVYAT
TSRSASLRQKKVTFDRLQVLDDHYRDVLKEMKAKASTVKAKLLSVEEACKLTPPHS
ARSKFGYGAKDVRNLSSKAVNHIRSVWKDLLEDTETPIDTTIMAKNEVFCVQPEKGG
RKPARLIVFPDLGVRVCEKMALYDVVSTLPQAVMGSSYGFQYSPGQRVEFLVNAWK
AKKCPMGFAYDTRCFDSTVTENDIRVEESIYQCCDLAPEARQAIRSLTERLYIGGPLT
NSKGQNCGYRRCRASGVLTTSCGNTLTCYLKAAAACRAAKLQDCTMLVCGDDLVV
ICESAGTQEDEASLRAFTEAMTRYSAPPGDPPKPEYDLELITSCSSNVSVAHDASGKR
VYYLTRDPTTPLARAAWETARHTPVNSWLGNIIMYAPTLWARMILMTHFFSILLAQE
QLEKALDCQIYGACYSIEPLDLPQIIQRLHGLSAFSLHSYSPGEINRVASCLRKLGVPPL
RVWRHRARSVRARLLSQGGRAATCGKYLFNWAVRTKLKLTPIPAASQLDLSSWFVA
GYSGGDIYHSLSRARPRWFMWCLLLLSVGVGIYLLPNR

SEQ ID NO:4: Amino acid sequence of the NS5A protein of HCVreplbBartMan

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRRLARGSPPSLA
SSSASQLSAPSLKATCTTRHDSPDADLIEANLLWRQEMGGNITRVESENKVVILDSFE
PLQAEEDEREVSVPAEILRRSRKFPRAMPIWARPDYNPPLLESWKDPDYVPPVVHGCP
LPPAKAPPIPPPRRKRTVVLSESTVSSALAELATKTFGSSESSAVDSGTATASPDQPSD
DGDAGSDVESYSSMPPLEGEPGDPDLSDGSWSTVSEEASEDVVCC

SEQ ID NO:5: Nucleotide sequence of DNA clone ofHCVrep1bBartMan/Δ2U's

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA

CCAATGTGGACCAGGACCTCGTCGGCTGGCAAGCGCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG
GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGGAGGCACCTCATTTTCT
GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG
CCAGGGCTCAGGCTCCACCTCCATCGTGGGACCAAATGTGGAAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA
AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC
TCCCCTGGCGCCCTAGTCGTCGGGGTCGTGTGCGCAGCGATACTGCGTCGGCACG
TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTAGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT

GCACGCCCTCCCCGGCGCCAAATTATTCTAGGGCGCTGTGGCGGGTGGCTGCTGA
GGAGTACGTGGAGGTTACGCGGGTGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTAGGCTGGCCAGGGGATCTCCCCCCTCCT
TGGCCAGCTCATCAGCTAGCCAGCTGTCTGCGCCTTCCTTGAAGGCAACATGCAC
TACCCGTCATGACTCCCCGGACGCTGACCTCATCGAGGCCAACCTCCTGTGGCGG
CAGGAGATGGGCGGAACATCACCCGCGTGGAGTCAGAAAATAAGGTAGTAATT
TTGGACTCTTTCGAGCCGCTCCAAGCGGAGGAGGATGAGAGGGAAGTATCCGTTC
CGGCGGAGATCCTGCGGAGGTCCAGGAAATTCCCTCGAGCGATGCCCATATGGG
CACGCCCGGATTACAACCCTCCACTGTTAGAGTCCTGGAAGGACCCGGACTACGT
CCCTCCAGTGGTACACGGGTGTCCATTGCCGCCTGCCAAGGCCCCTCCGATACCA
CCTCCACGGAGGAAGAGGACGGTTGTCCTGTCAGAATCTACCGTGTCTTCTGCCT
TGGCGGAGCTCGCCACAAAGACCTTCGGCAGCTCCGAATCGTCGGCCGTCGACA
GCGGCACGGCAACGGCCTCTCCTGACCAGCCCTCCGACGACGGCGACGCGGGAT
CCGACGTTGAGTCGTACTCCTCCATGCCCCCCCTTGAGGGGGAGCCGGGGGATCC
CGATCTCAGCGACGGGTCTTGGTCTACCGTAAGCGAGGAGGCTAGTGAGGACGT
CGTCTGCTGCTCGATGTCCTACACATGGACAGGCGCCCTGATCACGCCATGCGCT
GCGGAGGAAACCAAGCTGCCCATCAATGCACTGAGCAACTCTTTGCTCCGTCACC
ACAACTTGGTCTATGCTACAACATCTCGCAGCGCAAGCCTGCGGCAGAAGAAGG
TCACCTTTGACAGACTGCAGGTCCTGGACGACCACTACCGGGACGTGCTCAAGGA
GATGAAGGCGAAGGCGTCCACAGTTAAGGCTAAACTTCTATCCGTGGAGGAAGC
CTGTAAGCTGACGCCCCCACATTCGGCCAGATCTAAATTTGGCTATGGGGCAAAG
GACGTCCGGAACCTATCCAGCAAGGCCGTTAACCACATCCGCTCCGTGTGGAAGG
ACTTGCTGGAAGACACTGAGACACCAATTGACACCACCATCATGGCAAAAAATG
AGGTTTTCTGCGTCCAACCAGAGAAGGGGGGCCGCAAGCCAGCTCGCCTTATCGT
ATTCCCAGATTTGGGGGTTCGTGTGTGCGAGAAAATGGCCCTTTACGATGTGGTC
TCCACCCTCCCTCAGGCCGTGATGGGCTCTTCATACGGATTCCAATACTCTCCTGG
ACAGCGGGTCGAGTTCCTGGTGAATGCCTGGAAAGCGAAGAAATGCCCTATGGG
CTTCGCATATGACACCCGCTGTTTTGACTCAACGGTCACTGAGAATGACATCCGT
GTTGAGGAGTCAATCTACCAATGTTGTGACTTGGCCCCCGAAGCCAGACAGGCCA
TAAGGTCGCTCACAGAGCGGCTTTACATCGGGGGCCCCCTGACTAATTCTAAAGG
GCAGAACTGCGGCTATCGCCGGTGCCGCGCGAGCGGTGTACTGACGACCAGCTG
CGGTAATACCCTCACATGTTACTTGAAGGCCGCTGCGGCCTGTCGAGCTGCGAAG
CTCCAGGACTGCACGATGCTCGTATGCGGAGACGACCTTGTCGTTATCTGTGAAA
GCGCGGGGACCCAAGAGGACGAGGCGAGCCTACGGGCCTTCACGGAGGCTATGA
CTAGATACTCTGCCCCCCCTGGGGACCCGCCCAAACCAGAATACGACTTGGAGTT
GATAACATCATGCTCCTCCAATGTGTCAGTCGCGCACGATGCATCTGGCAAAAGG
GTGTACTATCTCACCCGTGACCCCACCACCCCCCTTGCGCGGGCTGCGTGGGAGA
CAGCTAGACACACTCCAGTCAATTCCTGGCTAGGCAACATCATCATGTATGCGCC
CACCTTGTGGGCAAGGATGATCCTGATGACTCATTTCTTCTCCATCCTTCTAGCTC
AGGAACAACTTGAAAAAGCCCTAGATTGTCAGATCTACGGGGCCTGTTACTCCAT
TGAGCCACTTGACCTACCTCAGATCATTCAACGACTCCATGGCCTTAGCGCATTTT
CACTCCATAGTTACTCTCCAGGTGAGATCAATAGGGTGGCTTCATGCCTCAGGAA
ACTTGGGGTACCGCCCTTGCGAGTCTGGAGACATCGGGCCAGAAGTGTCCGCGCT
AGGCTACTGTCCCAGGGGGGGAGGGCTGCCACTTGTGGCAAGTACCTCTTCAACT
GGGCAGTAAGGACCAAGCTCAAACTCACTCCAATCCCGGCTGCGTCCCAGTTGGA
TTTATCCAGCTGGTTCGTTGCTGGTTACAGCGGGGGAGACATATATCACAGCCTG
TCTCGTGCCCGACCCCGCTGGTTCATGTGGTGCCTACTCCTACTTTCTGTAGGGGT
AGGCATCTATCTACTCCCCAACCGATGAACGGGGAGCTAAACACTCCAGGCCAAT
AGGCCATCCTGTTTTTTTCCCTTTTTTTTTTTCTTTTTTTTTTTTTTTTTTTTTTTT

TTTTTCTCCTTTTTTTTTCCTCTTTTTTTCCTTTCTTTCCTTTGGTGGCTCCATCTTA
GCCCTAGTCACGGCTAGCTGTGAAAGGTCCGTGAGCCGCTTGACTGCAGAGAGTG
CTGATACTGGCCTCTCTGCAGATCAAGT

SEQ ID NO:6: Nucleotide sequence of DNA clone of HCVreplbBartMan/AvaII, where the nucleotide change creating the AvaII site is in lower case and highlighted in bold

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA
CCAATGTGGACCAGGACCTCGTCGGCTGGCAAGCGCCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG

GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGAGGCACCTCATTTTCT
GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG
CCAGGGCTCAGGCTCCACCTCCATCGTGGGACCAAATGTGGAAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA
AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC
TCCCCTGGCGCCCTAGTCGTCGGGGTCGTGTGCGCAGCGATACTGCGTCGGCACG
TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTAGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT
GCACGCCCTCCCCGGCGCCAAATTATTCTAGGGCGCTGTGGCGGGTGGCTGCTGA
GGAGTACGTGGAGGTTACGCGGGTGGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTAGGCTGGCCAGGGGATCTCCCCCCTCCT
TGGCCAGCTCATCAGCTAGCCAGCTGTCTGCGCCTTCCTTGAAGGCAACATGCAC

TACCCGTCATGACTCCCCGGACGCTGACCTCATCGAGGCCAACCTCCTGTGGCGG
CAGGAGATGGCGGGAACATCACCCGCGTGGAGTCAGAAAATAAGGTAGTAATT
TTGGACTCTTTCGAGCCGCTCCAAGCGGAGGAGGATGAGAGGGAAGTATCCGTTC
CGGCGGAGATCCTGCGGAGGTCCAGGAAATTCCCTCGAGCGATGCCCATATGGG
CACGCCCGGATTACAACCCTCCACTGTTAGAGTCCTGGAAGGACCCGGACTACGT
CCCTCCAGTGGTACACGGGTGTCCATTGCCGCCTGCCAAGGCCCCTCCGATACCA
CCTCCACGGAGGAAGAGGACGGTTGTCCTGTCAGAATCTACCGTGTCTTCTGCCT
TGGCGGAGCTCGCCACAAAGACCTTCGGCAGCTCCGAATCGTCGGCCGTCGACA
GCGGCACGGCAACGGCCTCTCCTGACCAGCCCTCCGACGACGGCGACGCGGGAT
CCGACGTTGAGTCGTACTCCTCCATGCCCCCCCTTGAGGGGGAGCCGGGGGATCC
CGATCTCAGCGACGGGTCTTGGTCTACCGTAAGCGAGGAGGCTAGTGAGGACGT
CGTCTGCTGCTCGATGTCCTACACATGGACAGGCGCCCTGATCACGCCATGCGCT
GCGGAGGAAACCAAGCTGCCCATCAATGCACTGAGCAACTCTTTGCTCCGTCACC
ACAACTTGGTCTATGCTACAACATCTCGCAGCGCAAGCCTGCGGCAGAAGAAGG
TCACCTTTGACAGACTGCAGGTCCTGGACGACCACTACCGGGACGTGCTCAAGGA
GATGAAGGCGAAGGCGTCCACAGTTAAGGCTAAACTTCTATCCGTGGAGGAAGC
CTGTAAGCTGACGCCCCCACATTCGGCCAGATCTAAATTTGGCTATGGGGCAAAG
GACGTCCGGAACCTATCCAGCAAGGCCGTTAACCACATCCGCTCCGTGTGGAAGG
ACTTGCTGGAAGACACTGAGACACCAATTGACACCACCATCATGGCAAAAAATG
AGGTTTTCTGCGTCCAACCAGAGAAGGGGGGCCGCAAGCCAGCTCGCCTTATCGT
ATTCCCAGATTTGGGGGTTCGTGTGTGCGAGAAAATGGCCCTTTACGATGTGGTC
TCCACCCTCCCTCAGGCCGTGATGGGCTCTTCATACGGATTCCAATACTCTCCTGG
ACAGCGGGTCGAGTTCCTGGTGAATGCCTGGAAAGCGAAGAAATGCCCTATGGG
CTTCGCATATGACACCCGCTGTTTTGACTCAACGGTCACTGAGAATGACATCCGT
GTTGAGGAGTCAATCTACCAATGTTGTGACTTGGCCCCCGAAGCCAGACAGGCCA
TAAGGTCGCTCACAGAGCGGCTTTACATCGGGGGCCCCCTGACTAATTCTAAAGG
GCAGAACTGCGGCTATCGCCGGTGCCGCGCGAGCGGTGTACTGACGACCAGCTG
CGGTAATACCCTCACATGTTACTTGAAGGCCGCTGCGGCCTGTCGAGCTGCGAAG
CTCCAGGACTGCACGATGCTCGTATGCGGAGACGACCTTGTCGTTATCTGTGAAA
GCGCGGGGACCCAAGAGGACGAGGCGAGCCTACGGGCCTTCACGGAGGCTATGA
CTAGATACTCTGCCCCCCCTGGGGACCCGCCCAAACCAGAATACGACTTGGAGTT
GATAACATCATGCTCCTCCAATGTGTCAGTCGCGCACGATGCATCTGGCAAAAGG
GTGTACTATCTCACCCGTGACCCCACCACCCCCCTTGCGCGGGCTGCGTGGGAGA
CAGCTAGACACACTCCAGTCAATTCCTGGCTAGGCAACATCATCATGTATGCGCC
CACCTTGTGGGCAAGGATGATCCTGATGACTCATTTCTTCTCCATCCTTCTAGCTC
AGGAACAACTTGAAAAAGCCCTAGATTGTCAGATCTACGGGGCCTGTTACTCCAT
TGAGCCACTTGACCTACCTCAGATCATTCAACGACTCCATGGCCTTAGCGCATTTT
CACTCCATAGTTACTCTCCAGGTGAGATCAATAGGGTGGCTTCATGCCTCAGGAA
ACTTGGGGTACCGCCCTTGCGAGTCTGGAGACATCGGGCCAGAAGTGTCCGCGCT
AGGCTACTGTCCCAGGGGGGGAGGGCTGCCACTTGTGGCAAGTACCTCTTCAACT
GGGCAGTAAGGACCAAGCTCAAACTCACTCCAATCCCGGCTGCGTCCCAGTTGGA
TTTATCCAGCTGGTTCGTTGCTGGTTACAGCGGGGGAGACATATATCACAGCCTG
TCTCGTGCCCGACCCCGCTGGTTCATGTGGTGCCTACTCCTACTTTCTGTAGGGGT
AGGCATCTATCTACTCCCCAACCGATGAACGGGGAcCTAAACACTCCAGGCCAAT
AGGCCATCCTGTTTTTTTCCCTTTTTTTTTTTCTTTTTTTTTTTTTTTTTTTTTTTTT
TTTTTTTCTCCTTTTTTTTTCCTCTTTTTTTCCTTTTCTTTCCTTTGGTGGCTCCATCT
TAGCCCTAGTCACGGCTAGCTGTGAAAGGTCCGTGAGCCGCTTGACTGCAGAGAG
TGCTGATACTGGCCTCTCTGCAGATCAAGT

SEQ ID NO:7: Nucleotide sequence of DNA clone of HCV adaptive replicon I, where the amino acid generated by the deletion is identified in lower case and highlighted in bold

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA
CCAATGTGGACCAGGACCTCGTCGGCTGGCAAGCGCCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG
GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGGAGGCACCTCATTTTCT

GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG
CCAGGGCTCAGGCTCCACCTCCATCGTGGGACCAAATGTGGAAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA
AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC
TCCCCTGGCGCCCTAGTCGTCGGGGTCGTGTGCGCAGCGATACTGCGTCGGCACG
TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTAGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT
GCACGCCCTCCCCGGCGCCAAATTATTCTAGGGCGCTGTGGCGGGTGGCTGCTGA
GGAGTACGTGGAGGTTACGCGGGTGGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTAGGCTGGCCAGGGGATCTCCCCCCTCCT
TGGCCAGCTCATCAGCTAGCCAGCTGtacTCTTTCGAGCCGCTCCAAGCGGAGGAG
GATGAGAGGGAAGTATCCGTTCCGGCGGAGATCCTGCGGAGGTCCAGGAAATTC
CCTCGAGCGATGCCCATATGGGCACGCCCGGATTACAACCCTCCACTGTTAGAGT
CCTGGAAGGACCCGGACTACGTCCCTCCAGTGGTACACGGGTGTCCATTGCCGCC
TGCCAAGGCCCCTCCGATACCACCTCCACGGAGGAAGAGGACGGTTGTCCTGTCA
GAATCTACCGTGTCTTCTGCCTTGGCGGAGCTCGCCACAAAGACCTTCGGCAGCT
CCGAATCGTCGGCCGTCGACAGCGGCACGGCAACGGCCTCTCCTGACCAGCCCTC
CGACGACGGCGACGCGGGATCCGACGTTGAGTCGTACTCCTCCATGCCCCCCCTT
GAGGGGGAGCCGGGGGATCCCGATCTCAGCGACGGGTCTTGGTCTACCGTAAGC

GAGGAGGCTAGTGAGGACGTCGTCTGCTGCTCGATGTCCTACACATGGACAGGC
GCCCTGATCACGCCATGCGCTGCGGAGGAAACCAAGCTGCCCATCAATGCACTG
AGCAACTCTTTGCTCCGTCACCACAACTTGGTCTATGCTACAACATCTCGCAGCG
CAAGCCTGCGGCAGAAGAAGGTCACCTTTGACAGACTGCAGGTCCTGGACGACC
ACTACCGGGACGTGCTCAAGGAGATGAAGGCGAAGGCGTCCACAGTTAAGGCTA
AACTTCTATCCGTGGAGGAAGCCTGTAAGCTGACGCCCCCACATTCGGCCAGATC
TAAATTTGGCTATGGGGCAAAGGACGTCCGGAACCTATCCAGCAAGGCCGTTAA
CCACATCCGCTCCGTGTGGAAGGACTTGCTGGAAGACACTGAGACACCAATTGAC
ACCACCATCATGGCAAAAAATGAGGTTTTCTGCGTCCAACCAGAGAAGGGGGGC
CGCAAGCCAGCTCGCCTTATCGTATTCCCAGATTTGGGGGTTCGTGTGTGCGAGA
AAATGGCCCTTTACGATGTGGTCTCCACCCTCCCTCAGGCCGTGATGGGCTCTTCA
TACGGATTCCAATACTCTCCTGGACAGCGGGTCGAGTTCCTGGTGAATGCCTGGA
AAGCGAAGAAATGCCCTATGGGCTTCGCATATGACACCCGCTGTTTTGACTCAAC
GGTCACTGAGAATGACATCCGTGTTGAGGAGTCAATCTACCAATGTTGTGACTTG
GCCCCCGAAGCCAGACAGGCCATAAGGTCGCTCACAGAGCGGCTTTACATCGGG
GGCCCCCTGACTAATTCTAAAGGGCAGAACTGCGGCTATCGCCGGTGCCGCGCGA
GCGGTGTACTGACGACCAGCTGCGGTAATACCCTCACATGTTACTTGAAGGCCGC
TGCGGCCTGTCGAGCTGCGAAGCTCCAGGACTGCACGATGCTCGTATGCGGAGAC
GACCTTGTCGTTATCTGTGAAAGCGCGGGACCCAAGAGGACGAGGCGAGCCTA
CGGGCCTTCACGGAGGCTATGACTAGATACTCTGCCCCCCCTGGGGACCCGCCCA
AACCAGAATACGACTTGGAGTTGATAACATCATGCTCCTCCAATGTGTCAGTCGC
GCACGATGCATCTGGCAAAAGGGTGTACTATCTCACCCGTGACCCCACCACCCCC
CTTGCGCGGGCTGCGTGGGAGACAGCTAGACACACTCCAGTCAATTCCTGGCTAG
GCAACATCATCATGTATGCGCCCACCTTGTGGGCAAGGATGATCCTGATGACTCA
TTTCTTCTCCATCCTTCTAGCTCAGGAACAACTTGAAAAAGCCCTAGATTGTCAGA
TCTACGGGGCCTGTTACTCCATTGAGCCACTTGACCTACCTCAGATCATTCAACG
ACTCCATGGCCTTAGCGCATTTTCACTCCATAGTTACTCTCCAGGTGAGATCAATA
GGGTGGCTTCATGCCTCAGGAAACTTGGGGTACCGCCCTTGCGAGTCTGGAGACA
TCGGGCCAGAAGTGTCCGCGCTAGGCTACTGTCCCAGGGGGGGAGGGCTGCCAC
TTGTGGCAAGTACCTCTTCAACTGGGCAGTAAGGACCAAGCTCAAACTCACTCCA
ATCCCGGCTGCGTCCCAGTTGGATTTATCCAGCTGGTTCGTTGCTGGTTACAGCGG
GGGAGACATATATCACAGCCTGTCTCGTGCCCGACCCCGCTGGTTCATGTGGTGC
CTACTCCTACTTTCTGTAGGGGTAGGCATCTATCTACTCCCCAACCGATGAACGG
GGACCTAAACACTCCAGGCCAATAGGCCATCCTGTTTTTTTCCCTTTTTTTTTTTCT
TTTTTTTTTTTTTTTTTTTTTTTTTTTTTTTCTCCTTTTTTTTTCCTCTTTTTTTCCTT
TTCTTTCCTTTGGTGGCTCCATCTTAGCCCTAGTCACGGCTAGCTGTGAAAGGTCC
GTGAGCCGCTTGACTGCAGAGAGTGCTGATACTGGCCTCTCTGCAGATCAAGT

SEQ ID NO:8: Nucleotide sequence of DNA clone of HCV adaptive replicon VI, where nucleotide changes are in lower case and highlighted in bold

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG

ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA
CCAATGTGGACCAGGACCTCGTCGGCTGGCgAGCGCCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG
GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGGAGGCACCTCATTTTCT
GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG

CCAGGGCTCAGGCTCCACCTCCATCGTGGACCAAATGTGGAAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA
AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC
TCCCCTGGCGCCCTAGTCGTCGGGGTCGTGTGCGCAGCGATACTGCGTCGGCACG
TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTAGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT
GCACGCCCTCCCCGGCGCCAAATTATTCTAGGGCGCTGTGGCGGGTGGCTGCTGA
GGAGTACGTGGAGGTTACGCGGGTGGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTAGGCTGGCCAGGGGATCTCCCCCCTCCT
TGGCCAGCTCATCAGCTAtCCAGCTGTCTGCGCCTTCCTTGAAGGCAACATGCACT
ACCCGTCATGACTCCCCGGACGCTGACCTCATCGAGGCCAACCTCCTGTGGCGGC
AGGAGATGGGCGGGAACATCACCCGCGTGGAGTCAGAAAATAAGGTAGTAATTT
TGGACTCTTTCGAGCCGCTCCAAGCGGAGGAGGATGAGAGGGAAGTATCCGTTC
CGGCGGAGATCCTGCGGAGGTCCAGGAAATTCCCTCGAGCGATGCCCATATGGG
CACGCCCGGATTACAACCCTCCACTGTTAGAGTCCTGGAAGGACCCGGACTACGT
CCCTCCAGTGGTACACGGGTGTCCATTGCCGCCTGCCAAGGCCCCTCCGATACCA
CCTCCACGGAGGAAGAGGACGGTTGTCCTGTCAGAATCTACCGTGTCTTCTGCCT
TGGCGGAGCTCGCCACAAAGACCTTCGGCAGCTCCGAATCGTCGGCCGTCGACA
GCGGCACGGCAACGGCCTCTCCTGACCAGCCCTCCGACGACGGCGACGCGGGAT
CCGACGTTGAGTCGTACTCCTCCATGCCCCCCCTTGAGGGGGAGCCGGGGGATCC
CGATCTCAGCGACGGGTCTTGGTCTACCGTAAGCGAGGAGGCTAGTGAGGACGT
CGTCTGCTGCTCGATGTCCTACACATGGACAGGCGCCCTGATCACGCCATGCGCT
GCGGAGGAAACCAAGCTGCCCATCAATGCACTGAGCAACTCTTTGCTCCGTCACC
ACAACTTGGTCTATGCTACAACATCTCGCAGCGCAAGCCTGCGGCAGAAGAAGG
TCACCTTTGACAGACTGCAGGTCCTGGACGACCACTACCGGGACGTGCTCAAGGA
GATGAAGGCGAAGGCGTCCACAGTTAAGGCTAAACTTCTATCCGTGGAGGAAGC
CTGTAAGCTGACGCCCCCACATTCGGCCAGATCTAAATTTGGCTATGGGGCAAAG
GACGTCCGGAACCTATCCAGCAAGGCCGTTAACCACATCCGCTCCGTGTGGAAGG
ACTTGCTGGAAGACACTGAGACACCAATTGACACCACCATCATGGCAAAAAATG

AGGTTTTCTGCGTCCAACCAGAGAAGGGGGGCCGCAAGCCAGCTCGCCTTATCGT
ATTCCCAGATTTGGGGGTTCGTGTGTGCGAGAAAATGGCCCTTTACGATGTGGTC
TCCACCCTCCCTCAGGCCGTGATGGGCTCTTCATACGGATTCCAATACTCTCCTGG
ACAGCGGGTCGAGTTCCTGGTGAATGCCTGGAAAGCGAAGAAATGCCCTATGGG
CTTCGCATATGACACCCGCTGTTTTGACTCAACGGTCACTGAGAATGACATCCGT
GTTGAGGAGTCAATCTACCAATGTTGTGACTTGGCCCCCGAAGCCAGACAGGCCA
TAAGGTCGCTCACAGAGCGGCTTTACATCGGGGGCCCCCTGACTAATTCTAAAGG
GCAGAACTGCGGCTATCGCCGGTGCCGCGCGAGCGGTGTACTGACGACCAGCTG
CGGTAATACCCTCACATGTTACTTGAAGGCCGCTGCGGCCTGTCGAGCTGCGAAG
CTCCAGGACTGCACGATGCTCGTATGCGGAGACGACCTTGTCGTTATCTGTGAAA
GCGCGGGGACCCAAGAGGACGAGGCGAGCCTACGGGCCTTCACGGAGGCTATGA
CTAGATACTCTGCCCCCCCTGGGGACCCGCCCAAACCAGAATACGACTTGGAGTT
GATAACATCATGCTCCTCCAATGTGTCAGTCGCGCACGATGCATCTGGCAAAAGG
GTGTACTATCTCACCCGTGACCCCACCACCCCCCTTGCGCGGGCTGCGTGGGAGA
CAGCTAGACACACTCCAGTCAATTCCTGGCTAGGCAACATCATCATGTATGCGCC
CACCTTGTGGGCAAGGATGATCCTGATGACTCATTTCTTCTCCATCCTTCTAGCTC
AGGAACAACTTGAAAAAGCCCTAGATTGTCAGATCTACGGGGCCTGTTACTCCAT
TGAGCCACTTGACCTACCTCAGATCATTCAACGACTCCATGGCCTTAGCGCATTTT
CACTCCATAGTTACTCTCCAGGTGAGATCAATAGGGTGGCTTCATGCCTCAGGAA
ACTTGGGGTACCGCCCTTGCGAGTCTGGAGACATCGGGCCAGAAGTGTCCGCGCT
AGGCTACTGTCCCAGGGGGGGAGGGCTGCCACTTGTGGCAAGTACCTCTTCAACT
GGGCAGTAAGGACCAAGCTCAAACTCACTCCAATCCCGGCTGCGTCCCAGTTGGA
TTTATCCAGCTGGTTCGTTGCTGGTTACAGCGGGGGAGACATATATCACAGCCTG
TCTCGTGCCCGACCCCGCTGGTTCATGTGGTGCCTACTCCTACTTTCTGTAGGGGT
AGGCATCTATCTACTCCCCAACCGATGAACGGGGAGCTAAACACTCCAGGCCAAT
AGGCCATCCTGTTTTTTTCCCTTTTTTTTTTTCTTTTTTTTTTTTTTTTTTTTTTTTT
TTTTTCTCCTTTTTTTTTCCTCTTTTTTTCCTTTTCTTTCCTTTGGTGGCTCCATCTTA
GCCCTAGTCACGGCTAGCTGTGAAAGGTCCGTGAGCCGCTTGACTGCAGAGAGTG
CTGATACTGGCCTCTCTGCAGATCAAGT

SEQ ID NO:9: Nucleotide sequence of DNA clone of HCV adaptive replicon II, where nucleotide changes are in lower case and highlighted in bold

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA

TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA
CCAATGTGGACCAGGACCTCGTCGGCTGGCAAGCGCCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG
GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGGAGGCACCTCATTTTCT
GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG
CCAGGGCTCAGGCTCCACCTCCATCGTGGGACCAAATGTGGgAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA

AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC
TCCCCTGGCGCCCTAGTCGTCGGGGTCGTGTGCGCAGCGATACTGCGTCGGCACG
TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTgGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT
GCACGCCCTCCCCGGCGCCAAATTATTCTAGGGCGCTGTGGCGGGTGGCTGCTGA
GGAGTACGTGGAGGTTACGCGGGTGGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTgGGCTGGCCAGGGGATCTCCCCCCTCCT
TGGCCAGCTCATCAGCTAGCCAGCTGTCTGCGCCTTCCTTGAAGGCAACATGCAC
TACCCGTCATGACTCCCCGGACGCTGACCTCATCGAGGCCAACCTCCTGTGGCGG
CAGGAGATGGGCGGGAACATCACCCGCGTGGAGTCAGAAAATAAGGTAGTAATT
TTGGACTCTTTCGAGCCGCTCCAAGCGGAGGAGGATGAGAGGGAAGTATCCGTTC
CGGCGGAGATCCTGCGGAGGTCCAGGAAATTCCCTCGAGCGATGCCCATATGGG
CACGCCCGGATTACAACCCTCCACTGTTAGAGTCCTGGAAGGACCCGGACTACGT
CCCTCCAGTGGTACACGGGTGTCCATTGCCGCCTGCCAAGGCCCCTCCGATACCA
CCTCCACGGAGGAAGAGGACGGTTGTCCTGTCAGAATCTACCGTGTCTTCTGCCT
TGGCGGAGCTCGCCACAAAGACCTTCGGCAGCTCCGAATCGTCGGCCGTCGACA
GCGGCACGGCAACGGCCTCTCCTGACCAGCCCTCCGACGACGGCGACGCGGGAT
CCGACGTTGAGTCGTACTCCTCCATGCCCCCCCTTGAGGGGGAGCCGGGGGATCC
CGATCTCAGCGACGGGTCTTGGTCTACCGTAAGCGAGGAGGCTAGTGAGGACGT
CGTCTGCTGCTCGATGTCCTACACATGGACAGGCGCCCTGATCACGCCATGCGCT
GCGGAGGAAACCAAGCTGCCCATCAATGCACTGAGCAACTCTTTGCTCCGTCACC
ACAACTTGGTCTATGCTACAACATCTCGCAGCGCAAGCCTGCGGCAGAAGAAGG
TCACCTTTGACAGACTGCAGGTCCTGGACGACCACTACCGGGACGTGCTCAAGGA
GATGAAGGCGAAGGCGTCCACAGTTAAGGCTAAACTTCTATCCGTGGAGGAAGC
CTGTAAGCTGACGCCCCCACATTCGGCCAGATCTAAATTTGGCTATGGGGCAAAG
GACGTCCGGAACCTATCCAGCAAGGCCGTTAACCACATCCGCTCCGTGTGGAAGG
ACTTGCTGGAAGACACTGAGACACCAATTGACACCACCATCATGGCAAAAAATG
AGGTTTTCTGCGTCCAACCAGAGAAGGGGGGCCGCAAGCCAGCTCGCCTTATCGT
ATTCCCAGATTTGGGGGTTCGTGTGTGCGAGAAAATGGCCCTTTACGATGTGGTC
TCCACCCTCCCTCAGGCCGTGATGGGCTCTTCATACGGATTCCAATACTCTCCTGG
ACAGCGGGTCGAGTTCCTGGTGAATGCCTGGAAAGCGAAGAAATGCCCTATGGG
CTTCGCATATGACACCCGCTGTTTTGACTCAACGGTCACTGAGAATGACATCCGT
GTTGAGGAGTCAATCTACCAATGTTGTGACTTGGCCCCCGAAGCCAGACAGGCCA
TAAGGTCGCTCACAGAGCGGCTTTACATCGGGGGCCCCCTGACTAATTCTAAAGG
GCAGAACTGCGGCTATCGCCGGTGCCGCGCGAGCGGTGTACTGACGACCAGCTG

CGGTAATACCCTCACATGTTACTTGAAGGCCGCTGCGGCCTGTCGAGCTGCGAAG
CTCCAGGACTGCACGATGCTCGTATGCGGAGACGACCTTGTCGTTATCTGTGAAA
GCGCGGGGACCCAAGAGGACGAGGCGAGCCTACGGGCCTTCACGGAGGCTATGA
CTAGATACTCTGCCCCCCCTGGGGACCCGCCCAAACCAGAATACGACTTGGAGTT
GATAACATCATGCTCCTCCAATGTGTCAGTCGCGCACGATGCATCTGGCAAAAGG
GTGTACTATCTCACCCGTGACCCCACCACCCCCCTTGCGCGGGCTGCGTGGGAGA
CAGCTAGACACACTCCAGTCAATTCCTGGCTAGGCAACATCATCATGTATGCGCC
CACCTTGTGGGCAAGGATGATCCTGATGACTCATTTCTTCTCCATCCTTCTAGCTC
AGGAACAACTTGAAAAAGCCCTAGATTGTCAGATCTACGGGGCCTGTTACTCCAT
TGAGCCACTTGACCTACCTCAGATCATTCAACGACTCCATGGCCTTAGCGCATTTT
CACTCCATAGTTACTCTCCAGGTGAGATCAATAGGGTGGCTTCATGCCTCAGGAA
ACTTGGGGTACCGCCCTTGCGAGTCTGGAGACATCGGGCCAGAAGTGTCCGCGCT
AGGCTACTGTCCCAGGGGGGGAGGGCTGCCACTTGTGGCAAGTACCTCTTCAACT
GGGCAGTAAGGACCAAGCTCAAACTCACTCCAATCCCGGCTGCGTCCCAGTTGGA
TTTATCCAGCTGGTTCGTTGCTGGTTACAGCGGGGGAGACATATATCACAGCCTG
TCTCGTGCCCGACCCCGCTGGTTCATGTGGTGCCTACTCCTACTTTCTGTAGGGGT
AGGCATCTATCTACTCCCCAACCGATGAACGGGGACCTAAACACTCCAGGCCAAT
AGGCCATCCTGTTTTTTTCCCTTTTTTTTTTTCTTTTTTTTTTTTTTTTTTTTTTTTT
TTTTTTTCTCCTTTTTTTTTCCTCTTTTTTTCCTTTTCTTTCCTTTGGTGGCTCCATCT
TAGCCCTAGTCACGGCTAGCTGTGAAAGGTCCGTGAGCCGCTTGACTGCAGAGAG
TGCTGATACTGGCCTCTCTGCAGATCAAGT

SEQ ID NO:10: Nucleotide sequence of DNA clone of HCV adaptive replicon V, where nucleotide change is in lower case and highlighted in bold

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG

ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA
CCAATGTGGACCAGGACCTCGTCGGCTGGCAAGCGCCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG
GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGGAGGCACCTCATTTTCT
GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG
CCAGGGCTCAGGCTCCACCTCCATCGTGGGACCAAATGTGGAAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA
AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC

TCCCCTGGCGCCCTAGTCGTCGGGGTCGTGTGCGCAGCGATACTGCGTCGGCACG
TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTAGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT
GCACGCCCTCCCCGGCGCCAAATTATTCTAGGGCGCTGTGGCGGGTGGCTGCTGA
GGAGTACGTGGAGGTTACGCGGGTGGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTAGGCTGGCCAGGGGATCTCCCCCCTCCT
TGtCCAGCTCATCAGCTAGCCAGCTGTCTGCGCCTTCCTTGAAGGCAACATGCACT
ACCCGTCATGACTCCCCGGACGCTGACCTCATCGAGGCCAACCTCCTGTGGCGGC
AGGAGATGGGCGGGAACATCACCCGCGTGGAGTCAGAAAATAAGGTAGTAATTT
TGGACTCTTTCGAGCCGCTCCAAGCGGAGGAGGATGAGAGGGAAGTATCCGTTC
CGGCGGAGATCCTGCGGAGGTCCAGGAAATTCCCTCGAGCGATGCCCATATGGG
CACGCCCGGATTACAACCCTCCACTGTTAGAGTCCTGGAAGGACCCGGACTACGT
CCCTCCAGTGGTACACGGGTGTCCATTGCCGCCTGCCAAGGCCCCTCCGATACCA
CCTCCACGGAGGAAGAGGACGGTTGTCCTGTCAGAATCTACCGTGTCTTCTGCCT
TGGCGGAGCTCGCCACAAAGACCTTCGGCAGCTCCGAATCGTCGGCCGTCGACA
GCGGCACGGCAACGGCCTCTCCTGACCAGCCCTCCGACGACGGCGACGCGGGAT
CCGACGTTGAGTCGTACTCCTCCATGCCCCCCCTTGAGGGGGAGCCGGGGGATCC
CGATCTCAGCGACGGGTCTTGGTCTACCGTAAGCGAGGAGGCTAGTGAGGACGT
CGTCTGCTGCTCGATGTCCTACACATGGACAGGCGCCCTGATCACGCCATGCGCT
GCGGAGGAAACCAAGCTGCCCATCAATGCACTGAGCAACTCTTTGCTCCGTCACC
ACAACTTGGTCTATGCTACAACATCTCGCAGCGCAAGCCTGCGGCAGAAGAAGG
TCACCTTTGACAGACTGCAGGTCCTGGACGACCACTACCGGGACGTGCTCAAGGA
GATGAAGGCGAAGGCGTCCACAGTTAAGGCTAAACTTCTATCCGTGGAGGAAGC
CTGTAAGCTGACGCCCCCACATTCGGCCAGATCTAAATTTGGCTATGGGGCAAAG
GACGTCCGGAACCTATCCAGCAAGGCCGTTAACCACATCCGCTCCGTGTGGAAGG
ACTTGCTGGAAGACACTGAGACACCAATTGACACCACCATCATGGCAAAAAATG
AGGTTTTCTGCGTCCAACCAGAGAAGGGGGGCCGCAAGCCAGCTCGCCTTATCGT
ATTCCCAGATTTGGGGGTTCGTGTGTGCGAGAAAATGGCCCTTTACGATGTGGTC
TCCACCCTCCCTCAGGCCGTGATGGGCTCTTCATACGGATTCCAATACTCTCCTGG
ACAGCGGGTCGAGTTCCTGGTGAATGCCTGGAAAGCGAAGAAATGCCCTATGGG
CTTCGCATATGACACCCGCTGTTTTGACTCAACGGTCACTGAGAATGACATCCGT
GTTGAGGAGTCAATCTACCAATGTTGTGACTTGGCCCCCGAAGCCAGACAGGCCA
TAAGGTCGCTCACAGAGCGGCTTTACATCGGGGGCCCCCTGACTAATTCTAAAGG
GCAGAACTGCGGCTATCGCCGGTGCCGCGCGAGCGGTGTACTGACGACCAGCTG
CGGTAATACCCTCACATGTTACTTGAAGGCCGCTGCGGCCTGTCGAGCTGCGAAG
CTCCAGGACTGCACGATGCTCGTATGCGGAGACGACCTTGTCGTTATCTGTGAAA
GCGCGGGGACCCAAGAGGACGAGGCGAGCCTACGGGCCTTCACGGAGGCTATGA
CTAGATACTCTGCCCCCCCTGGGGACCCGCCCAAACCAGAATACGACTTGGAGTT
GATAACATCATGCTCCTCCAATGTGTCAGTCGCGCACGATGCATCTGGCAAAAGG
GTGTACTATCTCACCCGTGACCCCACCACCCCCCTTGCGCGGGCTGCGTGGGAGA
CAGCTAGACACACTCCAGTCAATTCCTGGCTAGGCAACATCATCATGTATGCGCC
CACCTTGTGGGCAAGGATGATCCTGATGACTCATTTCTTCTCCATCCTTCTAGCTC

AGGAACAACTTGAAAAAGCCCTAGATTGTCAGATCTACGGGGCCTGTTACTCCAT
TGAGCCACTTGACCTACCTCAGATCATTCAACGACTCCATGGCCTTAGCGCATTTT
CACTCCATAGTTACTCTCCAGGTGAGATCAATAGGGTGGCTTCATGCCTCAGGAA
ACTTGGGGTACCGCCCTTGCGAGTCTGGAGACATCGGGCCAGAAGTGTCCGCGCT
AGGCTACTGTCCCAGGGGGGGAGGGCTGCCACTTGTGGCAAGTACCTCTTCAACT
GGGCAGTAAGGACCAAGCTCAAACTCACTCCAATCCCGGCTGCGTCCCAGTTGGA
TTTATCCAGCTGGTTCGTTGCTGGTTACAGCGGGGGAGACATATATCACAGCCTG
TCTCGTGCCCGACCCCGCTGGTTCATGTGGTGCCTACTCCTACTTTCTGTAGGGGT
AGGCATCTATCTACTCCCCAACCGATGAACGGGGACCTAAACACTCCAGGCCAAT
AGGCCATCCTGTTTTTTTCCCTTTTTTTTTTTCTTTTTTTTTTTTTTTTTTTTTTTTT
TTTTTTCTCCTTTTTTTTTCCTCTTTTTTTCCTTTTCTTTCCTTTGGTGGCTCCATCT
TAGCCCTAGTCACGGCTAGCTGTGAAAGGTCCGTGAGCCGCTTGACTGCAGAGAG
TGCTGATACTGGCCTCTCTGCAGATCAAGT

SEQ ID NO:11: NS5A gene of DNA clone of HCV adaptive replicon IV, where nucleotide change is in lower case and highlighted in bold

TCCGGCTCGTGGCTAAGAGATGTTTGGGATTGGATATGCACGGTGTTGACTGATT
TCAAGACCTGGCTCCAGTCCAAGCTCCTGCCGCGATTGCCGGGAGTCCCCTTCTT
CTCATGTCAACGTGGGTACAAGGGAGTCTGGCGGGCGACGGCATCATGCAAAC
CACCTGCCCATGTGGAGCACAGATCACCGGACATGTGAAAAACGGTTCCATGAG
GATCGTGGGGCCTAGGACCTGTAGTAACACGTGGCATGGAACATTCCCCATTAAC
GCGTACACCACGGGCCCCTGCACGCCCTCCCCGGCGCCAAATTATTCTAGGGCGC
TGTGGCGGGTGGCTGCTGAGGAGTACGTGGAGGTTACGCGGGTGGGGGATTTCC
ACTACGTGACGGGCATGACCACTGACAACGTAAAGTGCCCGTGTCAGGTTCCGGC
CCCCGAATTCTTCACAGAAGTGGATGGGGTGCGGTTGCACAGGTACGCTCCAGCG
TGCAAACCCCTCCTACGGGAGGAGGTCACATTCCTGGTCGGGCTCAATCAATACC
TGGTTGGGTCACAGCTCCCATGCGAGCCCGAACCGGACGTAGCAGTGCTCACTTC
CATGCTCACCGACCCCTCCCACATTACGGCGGAGACGGCTAAGCGTAGGCTGGCC
AGGGGATCTCCCCCCTgCTTGGCCAGCTCATCAGCTAGCCAGCTGTCTGCGCCTTC
CTTGAAGGCAACATGCACTACCCGTCATGACTCCCCGGACGCTGACCTCATCGAG
GCCAACCTCCTGTGGCGGCAGGAGATGGGCGGGAACATCACCCGCGTGGAGTCA
GAAAATAAGGTAGTAATTTTGGACTCTTTCGAGCCGCTCCAAGCGGAGGAGGAT
GAGAGGGAAGTATCCGTTCCGGCGGAGATCCTGCGGAGGTCCAGGAAATTCCCT
CGAGCGATGCCCATATGGGCACGCCCGGATTACAACCCTCCACTGTTAGAGTCCT
GGAAGGACCCGGACTACGTCCCTCCAGTGGTACACGGGTGTCCATTGCCGCCTGC
CAAGGCCCCTCCGATACCACCTCCACGGAGGAAGAGGACGGTTGTCCTGTCAGA
ATCTACCGTGTCTTCTGCCTTGGCGGAGCTCGCCACAAAGACCTTCGGCAGCTCC
GAATCGTCGGCCGTCGACAGCGGCACGGCAACGGCCTCTCCTGACCAGCCCTCCG
ACGACGGCGACGCGGGATCCGACGTTGAGTCGTACTCCTCCATGCCCCCCCTTGA
GGGGGAGCCGGGGGATCCCGATCTCAGCGACGGGTCTTGGTCTACCGTAAGCGA
GGAGGCTAGTGAGGACGTCGTCTGCTGC

SEQ ID NO: 12: NS5A gene of HCV adaptive replicon III, where nucleotide change is in lower case and highlighted in bold

TCCGGCTCGTGGCTAAGAGATGTTTGGGATTGGATATGCACGGTGTTGACTGATT
TCAAGACCTGGCTCCAGTCCAAGCTCCTGCCGCGATTGCCGGGAGTCCCCTTCTT
CTCATGTCAACGTGGGTACAAGGGAGTCTGGCGGGCGACGGCATCATGCAAAC
CACCTGCCCATGTGGAGCACAGATCACCGGACATGTGAAAAACGGTTCCATGAG
GATCGTGGGGCCTAGGACCTGTAGTAACACGTGGCATGGAACATTCCCCATTAAC

GCGTACACCACGGGCCCCTGCACGCCCTCCCCGGCGCCAAATTATTCTAGGGCGC
TGTGGCGGGTGGCTGCTGAGGAGTACGTGGAGGTTACGCGGTGGGGGATTTCC
ACTACGTGACGGGCATGACCACTGACAACGTAAAGTGCCCGTGTCAGGTTCCGGC
CCCCGAATTCTTCACAGAAGTGGATGGGGTGCGGTTGCACAGGTACGCTCCAGCG
TGCAAACCCCTCCTACGGGAGGAGGTCACATTCCTGGTCGGGCTCAATCAATACC
TGGTTGGGTCACAGCTCCCATGCGAGCCCGAACCGGACGTAGCAGTGCTCACTTC
CATGCTCACCGACCCCTCCCACATTACGGCGGAGACGGCTAAGCGTAGGCTGGCC
AGGGGATCTCCCCCcCCTTGGCCAGCTCATCAGCTAGCCAGCTGTCTGCGCCTTC
CTTGAAGGCAACATGCACTACCCGTCATGACTCCCCGGACGCTGACCTCATCGAG
GCCAACCTCCTGTGGCGGCAGGAGATGGGCGGGAACATCACCCGCGTGGAGTCA
GAAAATAAGGTAGTAATTTTGGACTCTTTCGAGCCGCTCCAAGCGGAGGAGGAT
GAGAGGGAAGTATCCGTTCCGGCGGAGATCCTGCGGAGGTCCAGGAAATTCCCT
CGAGCGATGCCCATATGGGCACGCCCGGATTACAACCCTCCACTGTTAGAGTCCT
GGAAGGACCCGGACTACGTCCCTCCAGTGGTACACGGGTGTCCATTGCCGCCTGC
CAAGGCCCCTCCGATACCACCTCCACGGAGGAAGAGGACGGTTGTCCTGTCAGA
ATCTACCGTGTCTTCTGCCTTGGCGGAGCTCGCCACAAAGACCTTCGGCAGCTCC
GAATCGTCGGCCGTCGACAGCGGCACGGCAACGGCCTCTCCTGACCAGCCCTCCG
ACGACGGCGACGCGGGATCCGACGTTGAGTCGTACTCCTCCATGCCCCCCCTTGA
GGGGGAGCCGGGGGATCCCGATCTCAGCGACGGGTCTTGGTCTACCGTAAGCGA
GGAGGCTAGTGAGGACGTCGTCTGCTGC

SEQ ID NO:13: Nucleotide sequence of DNA clone of HCV adaptive replicon VII, where nucleotide change is in lower case and highlighted in bold

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA

AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATACCATGGCGCCTAT
TACGGCCTACTCCCAACAGACGCGAGGCCTACTTGGCTGCATCATCACTAGCCTC
ACAGGCCGGGACAGGAACCAGGTCGAGGGGGAGGTCCAAGTGGTCTCCACCGCA
ACACAATCTTTCCTGGCGACCTGCGTCAATGGCGTGTGTTGGACTGTCTATCATG
GTGCCGGCTCAAAGACCCTTGCCGGCCCAAAGGGCCCAATCACCCAAATGTACA
CCAATGTGGACCAGGACCTCGTCGGCTGGCAAGCGCCCCCCGGGGCGCGTTCCTT
GACACCATGCACCTGCGGCAGCTCGGACCTTTACTTGGTCACGAGGCATGCCGAT
GTCATTCCGGTGCGCCGGCGGGGCGACAGCAGGGGGAGCCTACTCTCCCCCAGG
CCCGTCTCCTACTTGAAGGGCTCTTCGGGCGGTCCACTGCTCTGCCCCTCGGGGC
ACGCTGTGGGCATCTTTCGGGCTGCCGTGTGCACCCGAGGGGTTGCGAAGGCGGT
GGACTTTGTACCCGTCGAGTCTATGGAAACCACTATGCGGTCCCCGGTCTTCACG
GACAACTCGTCCCCTCCGGCCGTACCGCAGACATTCCAGGTGGCCCATCTACACG
CCCCTACTGGTAGCGGCAAGAGCACTAAGGTGCCGGCTGCGTATGCAGCCCAAG
GGTATAAGGTGCTTGTCCTGAACCCGTCCGTCGCCGCCACCCTAGGTTTCGGGGC
GTATATGTCTAAGGCACATGGTATCGACCCTAACATCAGAACCGGGGTAAGGAC
CATCACCACGGGTGCCCCCATCACGTACTCCACCTATGGCAAGTTTCTTGCCGAC
GGTGGTTGCTCTGGGGGCGCCTATGACATCATAATATGTGATGAGTGCCACTCAA
CTGACTCGACCACTATCCTGGGCATCGGCACAGTCCTGGACCAAGCGGAGACGG
CTGGAGCGCGACTCGTCGTGCTCGCCACCGCTACGCCTCCGGGATCGGTCACCGT
GCCACATCCAAACATCGAGGAGGTGGCTCTGTCCAGCACTGGAGAAATCCCCTTT
TATGGCAAAGCCATCCCCATCGAGACCATCAAGGGGGGGAGGCACCTCATTTTCT
GCCATTCCAAGAAGAAATGTGATGAGCTCGCCGCGAAGCTGTCCGGCCTCGGACT
CAATGCTGTAGCATATTACCGGGGCCTTGATGTATCCGTCATACCAACTAGCGGA
GACGTCATTGTCGTAGCAACGGACGCTCTAATGACGGGCTTTACCGGCGATTTCG
ACTCAGTGATCGACTGCAATACATGTGTCACCCAGACAGTCGACTTCAGCCTGGA
CCCGACCTTCACCATTGAGACGACGACCGTGCCACAAGACGCGGTGTCACGCTCG
CAGCGGCGAGGCAGGACTGGTAGGGGCAGGATGGGCATTTACAGGTTTGTGACT
CCAGGAGAACGGCCCTCGGGCATGTTCGATTCCTCGGTTCTGTGCGAGTGCTATG
ACGCGGGCTGTGCTTGGTACGAGCTCACGCCCGCCGAGACCTCAGTTAGGTTGCG
GGCTTACCTAAACACACCAGGGTTGCCCGTCTGCCAGGACCATCTGGAGTTCTGG
GAGAGCGTCTTTACAGGCCTCACCCACATAGACGCCCATTTCTTGTCCCAGACTA
AGCAGGCAGGAGACAACTTCCCCTACCTGGTAGCATACCAGGCTACGGTGTGCG
CCAGGGCTCAGGCTCCACCTCCATCGTGGGACCAAATGTGGAAGTGTCTCATACG
GCTAAAGCCTACGCTGCACGGGCCAACGCCCCTGCTGTATAGGCTGGGAGCCGTT
CAAAACGAGGTTACTACCACACACCCCATAACCAAATACATCATGGCATGCATGT
CGGCTGACCTGGAGGTCGTCACGAGCACCTGGGTGCTGGTAGGCGGAGTCCTAG
CAGCTCTGGCCGCGTATTGCCTGACAACAGGCAGCGTGGTCATTGTGGGCAGGAT
CATCTTGTCCGGAAAGCCGGCCATCATTCCCGACAGGGAAGTCCTTTACCGGGAG
TTCGATGAGATGGAAGAGTGCGCCTCACACCTCCCTTACATCGAACAGGGAATGC
AGCTCGCCGAACAATTCAAACAGAAGGCAATCGGGTTGCTGCAAACAGCCACCA
AGCAAGCGGAGGCTGCTGCTCCCGTGGTGGAATCCAAGTGGCGGACCCTCGAAG
CCTTCTGGGCGAAGCATATGTGGAATTTCATCAGCGGGATACAATATTTAGCAGG
CTTGTCCACTCTGCCTGGCAACCCCGCGATAGCATCACTGATGGCATTCACAGCC
TCTATCACCAGCCCGCTCACCACCCAACATACCCTCCTGTTTAACATCCTGGGGG
GATGGGTGGCCGCCCAACTTGCTCCTCCCAGCGCTGCTTCTGCTTTCGTAGGCGCC
GGCATCGCTGGAGCGGCTGTTGGCAGCATAGGCCTTGGGAAGGTGCTTGTGGATA
TTTTGGCAGGTTATGGAGCAGGGGTGGCAGGCGCGCTCGTGGCCTTTAAGGTCAT
GAGCGGCGAGATGCCCTCCACCGAGGACCTGGTTAACCTACTCCCTGCTATCCTC
TCCCCTGGCGCCCTAGTCGTCGGGGTCGTGTGCGCAGCGATACTGCGTCGGCACG

TGGGCCCAGGGGAGGGGGCTGTGCAGTGGATGAACCGGCTGATAGCGTTCGCTT
CGCGGGGTAACCACGTCTCCCCCACGCACTATGTGCCTGAGAGCGACGCTGCAGC
ACGTGTCACTCAGATCCTCTCTAGTCTTACCATCACTCAGCTGCTGAAGAGGCTTC
ACCAGTGGATCAACGAGGACTGCTCCACGCCATGCTCCGGCTCGTGGCTAAGAG
ATGTTTGGGATTGGATATGCACGGTGTTGACTGATTTCAAGACCTGGCTCCAGTC
CAAGCTCCTGCCGCGATTGCCGGGAGTCCCCTTCTTCTCATGTCAACGTGGGTAC
AAGGGAGTCTGGCGGGGCGACGGCATCATGCAAACCACCTGCCCATGTGGAGCA
CAGATCACCGGACATGTGAAAAACGGTTCCATGAGGATCGTGGGGCCTAGGACC
TGTAGTAACACGTGGCATGGAACATTCCCCATTAACGCGTACACCACGGGCCCCT
GCACGCCCTCCCCGGCGCCAAATTATTCTAGGGCGCTGTGGCGGGTGGCTGCTGA
GGAGTACGTGGAGGTTACGCGGGTGGGGGATTTCCACTACGTGACGGGCATGAC
CACTGACAACGTAAAGTGCCCGTGTCAGGTTCCGGCCCCCGAATTCTTCACAGAA
GTGGATGGGGTGCGGTTGCACAGGTACGCTCCAGCGTGCAAACCCCTCCTACGGG
AGGAGGTCACATTCCTGGTCGGGCTCAATCAATACCTGGTTGGGTCACAGCTCCC
ATGCGAGCCCGAACCGGACGTAGCAGTGCTCACTTCCATGCTCACCGACCCCTCC
CACATTACGGCGGAGACGGCTAAGCGTAGGCTGGCCAGGGGATCTCCCCCCTCCT
TGGCCAGCTCATCAGCTAtCCAGCTGTCTGCGCCTTCCTTGAAGGCAACATGCACT
ACCCGTCATGACTCCCCGGACGCTGACCTCATCGAGGCCAACCTCCTGTGGCGGC
AGGAGATGGGCGGGAACATCACCCGCGTGGAGTCAGAAAATAAGGTAGTAATTT
TGGACTCTTTCGAGCCGCTCCAAGCGGAGGAGGATGAGAGGGAAGTATCCGTTC
CGGCGGAGATCCTGCGGAGGTCCAGGAAATTCCCTCGAGCGATGCCCATATGGG
CACGCCCGGATTACAACCCTCCACTGTTAGAGTCCTGGAAGGACCCGGACTACGT
CCCTCCAGTGGTACACGGGTGTCCATTGCCGCCTGCCAAGGCCCCTCCGATACCA
CCTCCACGGAGGAAGAGGACGGTTGTCCTGTCAGAATCTACCGTGTCTTCTGCCT
TGGCGGAGCTCGCCACAAAGACCTTCGGCAGCTCCGAATCGTCGGCCGTCGACA
GCGGCACGGCAACGGCCTCTCCTGACCAGCCCTCCGACGACGGCGACGCGGGAT
CCGACGTTGAGTCGTACTCCTCCATGCCCCCCCTTGAGGGGGAGCCGGGGGATCC
CGATCTCAGCGACGGGTCTTGGTCTACCGTAAGCGAGGAGGCTAGTGAGGACGT
CGTCTGCTGCTCGATGTCCTACACATGGACAGGCGCCCTGATCACGCCATGCGCT
GCGGAGGAAACCAAGCTGCCCATCAATGCACTGAGCAACTCTTTGCTCCGTCACC
ACAACTTGGTCTATGCTACAACATCTCGCAGCGCAAGCCTGCGGCAGAAGAAGG
TCACCTTTGACAGACTGCAGGTCCTGGACGACCACTACCGGGACGTGCTCAAGGA
GATGAAGGCGAAGGCGTCCACAGTTAAGGCTAAACTTCTATCCGTGGAGGAAGC
CTGTAAGCTGACGCCCCCACATTCGGCCAGATCTAAATTTGGCTATGGGGCAAAG
GACGTCCGGAACCTATCCAGCAAGGCCGTTAACCACATCCGCTCCGTGTGGAAGG
ACTTGCTGGAAGACACTGAGACACCAATTGACACCACCATCATGGCAAAAAATG
AGGTTTTCTGCGTCCAACCAGAGAAGGGGGGCCGCAAGCCAGCTCGCCTTATCGT
ATTCCCAGATTTGGGGGTTCGTGTGTGCGAGAAAATGGCCCTTTACGATGTGGTC
TCCACCCTCCCTCAGGCCGTGATGGGCTCTTCATACGGATTCCAATACTCTCCTGG
ACAGCGGGTCGAGTTCCTGGTGAATGCCTGGAAAGCGAAGAAATGCCCTATGGG
CTTCGCATATGACACCCGCTGTTTTGACTCAACGGTCACTGAGAATGACATCCGT
GTTGAGGAGTCAATCTACCAATGTTGTGACTTGGCCCCCGAAGCCAGACAGGCCA
TAAGGTCGCTCACAGAGCGGCTTTACATCGGGGGCCCCCTGACTAATTCTAAAGG
GCAGAACTGCGGCTATCGCCGGTGCCGCGCGAGCGGTGTACTGACGACCAGCTG
CGGTAATACCCTCACATGTTACTTGAAGGCCGCTGCGGCCTGTCGAGCTGCGAAG
CTCCAGGACTGCACGATGCTCGTATGCGGAGACGACCTTGTCGTTATCTGTGAAA
GCGCGGGGACCCAAGAGGACGAGGCGAGCCTACGGGCCTTCACGGAGGCTATGA
CTAGATACTCTGCCCCCCCTGGGGACCCGCCCAAACCAGAATACGACTTGGAGTT
GATAACATCATGCTCCTCCAATGTGTCAGTCGCGCACGATGCATCTGGCAAAAGG
GTGTACTATCTCACCCGTGACCCCACCACCCCCCTTGCGCGGGCTGCGTGGGAGA
CAGCTAGACACACTCCAGTCAATTCCTGGCTAGGCAACATCATCATGTATGCGCC
CACCTTGTGGGCAAGGATGATCCTGATGACTCATTTCTTCTCCATCCTTCTAGCTC
AGGAACAACTTGAAAAAGCCCTAGATTGTCAGATCTACGGGGCCTGTTACTCCAT

TGAGCCACTTGACCTACCTCAGATCATTCAACGACTCCATGGCCTTAGCGCATTTT
CACTCCATAGTTACTCTCCAGGTGAGATCAATAGGGTGGCTTCATGCCTCAGGAA
ACTTGGGGTACCGCCCTTGCGAGTCTGGAGACATCGGGCCAGAAGTGTCCGCGCT
AGGCTACTGTCCCAGGGGGGGAGGGCTGCCACTTGTGGCAAGTACCTCTTCAACT
GGGCAGTAAGGACCAAGCTCAAACTCACTCCAATCCCGGCTGCGTCCCAGTTGGA
TTTATCCAGCTGGTTCGTTGCTGGTTACAGCGGGGGAGACATATATCACAGCCTG
TCTCGTGCCCGACCCCGCTGGTTCATGTGGTGCCTACTCCTACTTTCTGTAGGGGT
AGGCATCTATCTACTCCCCAACCGATGAACGGGGAGCTAAACACTCCAGGCCAAT
AGGCCATCCTGTTTTTTTCCCTTTTTTTTTTTTCTTTTTTTTTTTTTTTTTTTTTTTT
TTTTTCTCCTTTTTTTTTCCTCTTTTTTTCCTTTTCTTTCCTTTGGTGGCTCCATCTTA
GCCCTAGTCACGGCTAGCTGTGAAAGGTCCGTGAGCCGCTTGACTGCAGAGAGTG
CTGATACTGGCCTCTCTGCAGATCAAGT

SEQ ID NO:14: Amino acid sequence of the NS5A protein of HCV adaptive replicon I, where amino acid generated is highlighted in bold

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRRLARGSPPSLA
SSSASQLYSFEPLQAEEDEREVSVPAEILRRSRKFPRAMPIWARPDYNPPLLESWKDP
DYVPPVVHGCPLPPAKAPPIPPPRRKRTVVLSESTVSSALAELATKTFGSSESSAVDSG
TATASPDQPSDDGDAGSDVESYSSMPPLEGEPGDPDLSDGSWSTVSEEASEDVVCC

SEQ ID NO:15: Amino acid sequence of the polyprotein coding region of HCV adaptive replicon VI, where amino acid changes are highlighted in bold

MAPITAYSQQTRGLLGCIITSLTGRDRNQVEGEVQVVSTATQSFLATCVNGVCWTVY
HGAGSKTLAGPKGPITQMYTNVDQDLVGWRAPPGARSLTPCTCGSSDLYLVTRHAD
VIPVRRRGDSRGSLLSPRPVSYLKGSSGGPLLCPSGHAVGIFRAAVCTRGVAKAVDFV
PVESMETTMRSPVFTDNSSPPAVPQTFQVAHLHAPTGSGKSTKVPAAYAAQGYKVL
VLNPSVAATLGFGAYMSKAHGIDPNIRTGVRTITTGAPITYSTYGKFLADGGCSGGAY
DIIICDECHSTDSTTILGIGTVLDQAETAGARLVVLATATPPGSVTVPHPNIEEVALSST
GEIPFYGKAIPIETIKGGRHLIFCHSKKKCDELAAKLSGLGLNAVAYYRGLDVSVIPTS
GDVIVVATDALMTGFTGDFDSVIDCNTCVTQTVDFSLDPTFTIETTTVPQDAVSRSQR
RGRTGRGRMGIYRFVTPGERPSGMFDSSVLCECYDAGCAWYELTPAETSVRLRAYL
NTPGLPVCQDHLEFWESVFTGLTHIDAHFLSQTKQAGDNFPYLVAYQATVCARAQA
PPPSWDQMWKCLIRLKPTLHGPTPLLYRLGAVQNEVTTTHPITKYIMACMSADLEVV
TSTWVLVGGVLAALAAYCLTTGSVVIVGRIILSGKPAIIPDREVLYREFDEMEECASH
LPYIEQGMQLAEQFKQKAIGLLQTATKQAEAAAPVVESKWRTLEAFWAKHMWNFIS
GIQYLAGLSTLPGNPAIASLMAFTASITSPLTTQHTLLFNILGGWVAAQLAPPSAASAF
VGAGIAGAAVGSIGLGKVLVDILAGYGAGVAGALVAFKVMSGEMPSTEDLVNLLPA
ILSPGALVVGVVCAAILRRHVGPGEGAVQWMNRLIAFASRGNHVSPTHYVPESDAA
ARVTQILSSLTITQLLKRLHQWINEDCSTPCSGSWLRDVWDWICTVLTDFKTWLQSK
LLPRLPGVPFFSCQRGYKGVWRGDGIMQTTCPCGAQITGHVKNGSMRIVGPRTCSNT
WHGTFPINAYTTGPCTPSPAPNYSRALWRVAAEEYVEVTRVGDFHYVTGMTTDNVK
CPCQVPAPEFFTEVDGVRLHRYAPACKPLLREEVTFLVGLNQYLVGSQLPCEPEPDV
AVLTSMLTDPSHITAETAKRRLARGSPPSLASSSAIQLSAPSLKATCTTRHDSPDADLI
EANLLWRQEMGGNITRVESENKVVILDSFEPLQAEEDEREVSVPAEILRRSRKFPRAM
PIWARPDYNPPLLESWKDPDYVPPVVHGCPLPPAKAPPIPPPRRKRTVVLSESTVSSAL
AELATKTFGSSESSAVDSGTATASPDQPSDDGDAGSDVESYSSMPPLEGEPGDPDLSD

GSWSTVSEEASEDVVCCSMSYTWTGALITPCAAEETKLPINALSNSLLRHHNLVYAT
TSRSASLRQKKVTFDRLQVLDDHYRDVLKEMKAKASTVKAKLLSVEEACKLTPPHS
ARSKFGYGAKDVRNLSSKAVNHIRSVWKDLLEDTETPIDTTIMAKNEVFCVQPEKGG
RKPARLIVFPDLGVRVCEKMALYDVVSTLPQAVMGSSYGFQYSPGQRVEFLVNAWK
AKKCPMGFAYDTRCFDSTVTENDIRVEESIYQCCDLAPEARQAIRSLTERLYIGGPLT
NSKGQNCGYRRCRASGVLTTSCGNTLTCYLKAAAACRAAKLQDCTMLVCGDDLVV
ICESAGTQEDEASLRAFTEAMTRYSAPPGDPPKPEYDLELITSCSSNVSVAHDASGKR
VYYLTRDPTTPLARAAWETARHTPVNSWLGNIIMYAPTLWARMILMTHFFSILLAQE
QLEKALDCQIYGACYSIEPLDLPQIIQRLHGLSAFSLHSYSPGEINRVASCLRKLGVPPL
RVWRHRARSVRARLLSQGGRAATCGKYLFNWAVRTKLKLTPIPAASQLDLSSWFVA
GYSGGDIYHSLSRARPRWFMWCLLLLSVGVGIYLLPNR

SEQ ID NO:16: Amino acid sequence of the NS5A protein of HCV adaptive replicon VII, where amino acid change is highlighted in bold

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRRLARGSPPSLA
SSSAIQLSAPSLKATCTTRHDSPDADLIEANLLWRQEMGGNITRVESENKVVILDSFEP
LQAEEDEREVSVPAEILRRSRKFPRAMPIWARPDYNPPLLESWKDPDYVPPVVHGCP
LPPAKAPPIPPPRRKRTVVLSESTVSSALAELATKTFGSSESSAVDSGTATASPDQPSD
DGDAGSDVESYSSMPPLEGEPGDPDLSDGSWSTVSEEASEDVVCC

SEQ ID NO:17: Amino acid sequence of the polyprotein of HCV adaptive replicon II, where amino acid changes are highlighted in bold

MAPITAYSQQTRGLLGCIITSLTGRDRNQVEGEVQVVSTATQSFLATCVNGVCWTVY
HGAGSKTLAGPKGPITQMYTNVDQDLVGWQAPPGARSLTPCTCGSSDLYLVTRHAD
VIPVRRRGDSRGSLLSPRPVSYLKGSSGGPLLCPSGHAVGIFRAAVCTRGVAKAVDFV
PVESMETTMRSPVFTDNSSPPAVPQTFQVAHLHAPTGSGKSTKVPAAYAAQGYKVL
VLNPSVAATLGFGAYMSKAHGIDPNIRTGVRTITTGAPITYSTYGKFLADGGCSGGAY
DIIICDECHSTDSTTILGIGTVLDQAETAGARLVVLATATPPGSVTVPHPNIEEVALSST
GEIPFYGKAIPIETIKGGRHLIFCHSKKKCDELAAKLSGLGLNAVAYYRGLDVSVIPTS
GDVIVVATDALMTGFTGDFDSVIDCNTCVTQTVDFSLDPTFTIETTTVPQDAVSRSQR
RGRTGRGRMGIYRFVTPGERPSGMFDSSVLCECYDAGCAWYELTPAETSVRLRAYL
NTPGLPVCQDHLEFWESVFTGLTHIDAHFLSQTKQAGDNFPYLVAYQATVCARAQA
PPPSWDQMWECLIRLKPTLHGPTPLLYRLGAVQNEVTTTHPITKYIMACMSADLEVV
TSTWVLVGGVLAALAAYCLTTGSVVIVGRIILSGKPAIIPDREVLYREFDEMEECASH
LPYIEQGMQLAEQFKQKAIGLLQTATKQAEAAAPVVESKWRTLEAFWAKHMWNFIS
GIQYLAGLSTLPGNPAIASLMAFTASITSPLTTQHTLLFNILGGWVAAQLAPPSAASAF
VGAGIAGAAVGSIGLGKVLVDILAGYGAGVAGALVAFKVMSGEMPSTEDLVNLLPA
ILSPGALVVGVVCAAILRRHVGPGEGAVQWMNRLIAFASRGNHVSPTHYVPESDAA
ARVTQILSGLTITQLLKRLHQWINEDCSTPCSGSWLRDVWDWICTVLTDFKTWLQSK
LLPRLPGVPFFSCQRGYKGVWRGDGIMQTTCPCGAQITGHVKNGSMRIVGPRTCSNT
WHGTFPINAYTTGPCTPSPAPNYSRALWRVAAEEYVEVTRVGDFHYVTGMTTDNVK
CPCQVPAPEFFTEVDGVRLHRYAPACKPLLREEVTFLVGLNQYLVGSQLPCEPEPDV
AVLTSMLTDPSHITAETAKRGLARGSPPSLASSSASQLSAPSLKATCTTRHDSPDADLI
EANLLWRQEMGGNITRVESENKVVILDSFEPLQAEEDEREVSVPAEILRRSRKFPRAM
PIWARPDYNPPLLESWKDPDYVPPVVHGCPLPPAKAPPIPPPRRKRTVVLSESTVSSAL
AELATKTFGSSESSAVDSGTATASPDQPSDDGDAGSDVESYSSMPPLEGEPGDPDLSD

GSWSTVSEEASEDVVCCSMSYTWTGALITPCAAEETKLPINALSNSLLRHHNLVYAT
TSRSASLRQKKVTFDRLQVLDDHYRDVLKEMKAKASTVKAKLLSVEEACKLTPPHS
ARSKFGYGAKDVRNLSSKAVNHIRSVWKDLLEDTETPIDTTIMAKNEVFCVQPEKGG
RKPARLIVFPDLGVRVCEKMALYDVVSTLPQAVMGSSYGFQYSPGQRVEFLVNAWK
AKKCPMGFAYDTRCFDSTVTENDIRVEESIYQCCDLAPEARQAIRSLTERLYIGGPLT
NSKGQNCGYRRCRASGVLTTSCGNTLTCYLKAAAACRAAKLQDCTMLVCGDDLVV
ICESAGTQEDEASLRAFTEAMTRYSAPPGDPPKPEYDLELITSCSSNVSVAHDASGKR
VYYLTRDPTTPLARAAWETARHTPVNSWLGNIIMYAPTLWARMILMTHFFSILLAQE
QLEKALDCQIYGACYSIEPLDLPQIIQRLHGLSAFSLHSYSPGEINRVASCLRKLGVPPL
RVWRHRARSVRARLLSQGGRAATCGKYLFNWAVRTKLKLTPIPAASQLDLSSWFVA
GYSGGDIYHSLSRARPRWFMWCLLLLSVGVGIYLLPNR

SEQ ID NO:18: Amino acid sequence of the NS5A protein of HCV adaptive replicon II, where amino acid change is highlighted in bold

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRGLARGSPPSLA
SSSASQLSAPSLKATCTTRHDSPDADLIEANLLWRQEMGGNITRVESENKVVILDSFE
PLQAEEDEREVSVPAEILRRSRKFPRAMPIWARPDYNPPLLESWKDPDYVPPVVHGCP
LPPAKAPPIPPPRRKRTVVLSESTVSSALAELATKTFGSSESSAVDSGTATASPDQPSD
DGDAGSDVESYSSMPPLEGEPGDPDLSDGSWSTVSEEASEDVVCC

SEQ ID NO:19: Amino acid sequence of the NS5A protein of HCV adaptive replicon V, where amino acid, change is highlighted in bold

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRRLARGSPPSLS
SSSASQLSAPSLKATCTTRHDSPDADLIEANLLWRQEMGGNITRVESENKVVILDSFE
PLQAEEDEREVSVPAEILRRSRKFPRAMPIWARPDYNPPLLESWKDPDYVPPVVHGCP
LPPAKAPPIPPPRRKRTVVLSESTVSSALAELATKTFGSSESSAVDSGTATASPDQPSD
DGDAGSDVESYSSMPPLEGEPGDPDLSDGSWSTVSEEASEDVVCC

SEQ ID NO:20: Amino acid sequence of the NS5A protein of HCV adaptive replicon IV, where amino acid change is highlighted in bold

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRRLARGSPPCLA
SSSASQLSAPSLKATCTTRHDSPDADLIEANLLWRQEMGGNITRVESENKVVILDSFE
PLQAEEDEREVSVPAEILRRSRKFPRAMPIWARPDYNPPLLESWKDPDYVPPVVHGCP
LPPAKAPPIPPPRRKRTVVLSESTVSSALAELATKTFGSSESSAVDSGTATASPDQPSD
DGDAGSDVESYSSMPPLEGEPGDPDLSDGSWSTVSEEASEDVVCC

SEQ ID NO:21: Amino acid sequence of the NS5A protein of HCV adaptive replicon III, where amino acid change is highlighted in bold

SGSWLRDVWDWICTVLTDFKTWLQSKLLPRLPGVPFFSCQRGYKGVWRGDGIMQTT
CPCGAQITGHVKNGSMRIVGPRTCSNTWHGTFPINAYTTGPCTPSPAPNYSRALWRV
AAEEYVEVTRVGDFHYVTGMTTDNVKCPCQVPAPEFFTEVDGVRLHRYAPACKPLL
REEVTFLVGLNQYLVGSQLPCEPEPDVAVLTSMLTDPSHITAETAKRRLARGSPPPLA
SSSASQLSAPSLKATCTTRHDSPDADLIEANLLWRQEMGGNITRVESENKVVILDSFE
PLQAEEDEREVSVPAEILRRSRKFPRAMPIWARPDYNPPLLESWKDPDYVPPVVHGCP
LPPAKAPPIPPPRRKRTVVLSESTVSSALAELATKTFGSSESSAVDSGTATASPDQPSD
DGDAGSDVESYSSMPPLEGEPGDPDLSDGSWSTVSEEASEDVVCC

SEQ ID NO:22: Nucleotide sequence of DNA clone of HCV adaptive replicon HCVrep/NS2-5B (see Figure 9)

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCAGACCACAACGGTTTCCCTCTAGCGGGATCAATTCCGCCCCTC
TCCCTCCCCCCCCCCTAACGTTACTGGCCGAAGCCGCTTGGAATAAGGCCGGTGT
GCGTTTGTCTATATGTTATTTTCCACCATATTGCCGTCTTTTGGCAATGTGAGGGC
CCGGAAACCTGGCCCTGTCTTCTTGACGAGCATTCCTAGGGGTCTTTCCCCTCTCG
CCAAAGGAATGCAAGGTCTGTTGAATGTCGTGAAGGAAGCAGTTCCTCTGGAAG
CTTCTTGAAGACAAACAACGTCTGTAGCGACCCTTTGCAGGCAGCGGAACCCCCC
ACCTGGCGACAGGTGCCTCTGCGGCCAAAAGCCACGTGTATAAGATACACCTGC
AAAGGCGGCACAACCCCAGTGCCACGTTGTGAGTTGGATAGTTGTGGAAAGAGT
CAAATGGCTCTCCTCAAGCGTATTCAACAAGGGGCTGAAGGATGCCCAGAAGGT
ACCCCATTGTATGGGATCTGATCTGGGGCCTCGGTGCACATGCTTTACATGTGTTT
AGTCGAGGTTAAAAAACGTCTAGGCCCCCCGAACCACGGGGACGTGGTTTTCCTT
TGAAAAACACGATAATACCATGGACCGGGAGATGGCAGCATCGTGCGGAGGCGC
GGTTTTCGTAGGTCTGATACTCTTGACCTTGTCACCGCACTATAAGCTGTTCCTCG
CTAGGCTCATATGGTGGTTACAATATTTATCACCAGGGCCGAGGCACACTTGCA
AGTGTGGATCCCCCCCCTCAACGTTCGGGGGGCCGCGATGCCGTCATCCTCCTC
ACGTGCGCGATCCACCCAGAGCTAATCTTTACCATCACCAAAATCTTGCTCGCCA
TACTCGGTCCACTCATGGTGCTCCAGGCTGGTATAACCAAAGTGCCGTACTTCGT
GCGCGCACACGGGCTCATTCGTGCATGCATGCTGGTGCGGAAGGTTGCTGGGGGT
CATTATGTCCAAATGGCTCTCATGAAGTTGGCCGCACTGACAGGTACGTACGTTT
ATGACCATCTCACCCCACTGCGGGACTGGGCCCACGCGGGCCTACGAGACCTTGC
GGTGGCAGTTGAGCCCGTCGTCTTCTCTGATATGGAGACCAAGGTTATCACCTGG
GGGGCAGACACCGCGGCGTGTGGGGACATCATCTTGGGCCTGCCCGTCTCCGCCC
GCAGGGGGAGGGAGATACATCTGGGACCGGCAGACAGCCTTGAAGGGCAGGGG
TGGCGACTCCTCGCGCCTATTACGGCCTACTCCCAACAGACGCGAGGCCTACTTG
GCTGCATCATCACTAGCCTCACAGGCCGGGACAGGAACCAGGTCGAGGGGGAGG
TCCAAGTGGTCTCCACCGCAACACAATCTTTCCTGGCGACCTGCGTCAATGGCGT
GTGTTGGACTGTCTATCATGGTGCCGGCTCAAAGACCCTTGCCGGCCCAAAGGGC
CCAATCACCCAAATGTACACCAATGTGGACCAGGACCTCGTCGGCTGGCAAGCG
CCCCCCGGGGCGCGTTCCTTGACACCATGCACCTGCGGCAGCTCGGACCTTTACT
TGGTCACGAGGCATGCCGATGTCATTCCGGTGCGCCGGCGGGGCGACAGCAGGG
GGAGCCTACTCTCCCCCAGGCCCGTCTCCTACTTGAAGGGCTCTTCGGGCGGTCC
ACTGCTCTGCCCCTCGGGGCACGCTGTGGGCATCTTTCGGGCTGCCGTGTGCACC

CGAGGGGTTGCGAAGGCGGTGGACTTTGTACCCGTCGAGTCTATGGAAACCACTA
TGCGGTCCCCGGTCTTCACGGACAACTCGTCCCTCCGGCCGTACCGCAGACATT
CCAGGTGGCCCATCTACACGCCCCTACTGGTAGCGGCAAGAGCACTAAGGTGCC
GGCTGCGTATGCAGCCCAAGGGTATAAGGTGCTTGTCCTGAACCCGTCCGTCGCC
GCCACCCTAGGTTTCGGGGCGTATATGTCTAAGGCACATGGTATCGACCCTAACA
TCAGAACCGGGGTAAGGACCATCACCACGGGTGCCCCCATCACGTACTCCACCTA
TGGCAAGTTTCTTGCCGACGGTGGTTGCTCTGGGGGCGCCTATGACATCATAATA
TGTGATGAGTGCCACTCAACTGACTCGACCACTATCCTGGGCATCGGCACAGTCC
TGGACCAAGCGGAGACGGCTGGAGCGCGACTCGTCGTGCTCGCCACCGCTACGC
CTCCGGGATCGGTCACCGTGCCACATCCAAACATCGAGGAGGTGGCTCTGTCCAG
CACTGGAGAAATCCCCTTTTATGGCAAAGCCATCCCCATCGAGACCATCAAGGGG
GGGAGGCACCTCATTTTCTGCCATTCCAAGAAGAAATGTGATGAGCTCGCCGCGA
AGCTGTCCGGCCTCGGACTCAATGCTGTAGCATATTACCGGGGCCTTGATGTATC
CGTCATACCAACTAGCGGAGACGTCATTGTCGTAGCAACGGACGCTCTAATGACG
GGCTTTACCGGCGATTTCGACTCAGTGATCGACTGCAATACATGTGTCACCCAGA
CAGTCGACTTCAGCCTGGACCCGACCTTCACCATTGAGACGACGACCGTGCCAC
AAGACGCGGTGTCACGCTCGCAGCGGCGAGGCAGGACTGGTAGGGGCAGGATGG
GCATTTACAGGTTTGTGACTCCAGGAGAACGGCCCTCGGGCATGTTCGATTCCTC
GGTTCTGTGCGAGTGCTATGACGCGGGCTGTGCTTGGTACGAGCTCACGCCCGCC
GAGACCTCAGTTAGGTTGCGGGCTTACCTAAACACACCAGGGTTGCCCGTCTGCC
AGGACCATCTGGAGTTCTGGGAGAGCGTCTTTACAGGCCTCACCCACATAGACGC
CCATTTCTTGTCCCAGACTAAGCAGGCAGGAGACAACTTCCCCTACCTGGTAGCA
TACCAGGCTACGGTGTGCGCCAGGGCTCAGGCTCCACCTCCATCGTGGGACCAAA
TGTGGAAGTGTCTCATACGGCTAAAGCCTACGCTGCACGGGCCAACGCCCCTGCT
GTATAGGCTGGGAGCCGTTCAAAACGAGGTTACTACCACACACCCCATAACCAA
ATACATCATGGCATGCATGTCGGCTGACCTGGAGGTCGTCACGAGCACCTGGGTG
CTGGTAGGCGGAGTCCTAGCAGCTCTGGCCGCGTATTGCCTGACAACAGGCAGCG
TGGTCATTGTGGGCAGGATCATCTTGTCCGGAAAGCCGGCCATCATTCCCGACAG
GGAAGTCCTTTACCGGGAGTTCGATGAGATGGAAGAGTGCGCCTCACACCTCCCT
TACATCGAACAGGGAATGCAGCTCGCCGAACAATTCAAACAGAAGGCAATCGGG
TTGCTGCAAACAGCCACCAAGCAAGCGGAGGCTGCTGCTCCCGTGGTGGAATCC
AAGTGGCGGACCCTCGAAGCCTTCTGGGCGAAGCATATGTGGAATTTCATCAGCG
GGATACAATATTTAGCAGGCTTGTCCACTCTGCCTGGCAACCCCGCGATAGCATC
ACTGATGGCATTCACAGCCTCTATCACCAGCCCGCTCACCACCCAACATACCCTC
CTGTTTAACATCCTGGGGGGATGGGTGGCCGCCCAACTTGCTCCTCCCAGCGCT
GCTTCTGCTTTCGTAGGCGCCGGCATCGCTGGAGCGGCTGTTGGCAGCATAGGCC
TTGGGAAGGTGCTTGTGGATATTTTGGCAGGTTATGGAGCAGGGGTGGCAGGCGC
GCTCGTGGCCTTTAAGGTCATGAGCGGCGAGATGCCCTCCACCGAGGACCTGGTT
AACCTACTCCCTGCTATCCTCTCCCCTGGCGCCCTAGTCGTCGGGGTCGTGTGCGC
AGCGATACTGCGTCGGCACGTGGGCCCAGGGGAGGGGGCTGTGCAGTGGATGAA
CCGGCTGATAGCGTTCGCTTCGCGGGGTAACCACGTCTCCCCCACGCACTATGTG
CCTGAGAGCGACGCTGCAGCACGTGTCACTCAGATCCTCTCTAGTCTTACCATCA
CTCAGCTGCTGAAGAGGCTTCACCAGTGGATCAACGAGGACTGCTCCACGCCATG
CTCCGGCTCGTGGCTAAGAGATGTTTGGGATTGGATATGCACGGTGTTGACTGAT
TTCAAGACCTGGCTCCAGTCCAAGCTCCTGCCGCGATTGCCGGGAGTCCCCTTCTT
CTCATGTCAACGTGGGTACAAGGGAGTCTGGCGGGGCGACGGCATCATGCAAAC
CACCTGCCCATGTGGAGCACAGATCACCGGACATGTGAAAAACGGTTCCATGAG
GATCGTGGGGCCTAGGACCTGTAGTAACACGTGGCATGGAACATTCCCCATTAAC
GCGTACACCACGGGCCCCTGCACGCCCTCCCCGGCGCCAAATTATTCTAGGGCGC
TGTGGCGGGTGGCTGCTGAGGAGTACGTGGAGGTTACGCGGGTGGGGGATTTCC
ACTACGTGACGGGCATGACCACTGACAACGTAAAGTGCCCGTGTCAGGTTCC
GGCCCCCGAATTCTTCACAGAAGTGGATGGGGTGCGGTTGCACAGGTACGCTCCA
GCGTGCAAACCCCTCCTACGGGAGGAGGTCACATTCCTGGTCGGGCTCAATCAAT

ACCTGGTTGGGTCACAGCTCCCATGCGAGCCCGAACCGGACGTAGCAGTGCTCAC
TTCCATGCTCACCGACCCCTCCCACATTACGGCGGAGACGGCTAAGCGTAGGCTG
GCCAGGGGATCTCCCCCCTCCTTGGCCAGCTCATCAGCTATCCAGCTGTCTGCGC
CTTCCTTGAAGGCAACATGCACTACCCGTCATGACTCCCCGGACGCTGACCTCAT
CGAGGCCAACCTCCTGTGGCGGCAGGAGATGGGCGGGAACATCACCCGCGTGGA
GTCAGAAAATAAGGTAGTAATTTGGACTCTTTCGAGCCGCTCCAAGCGGAGGAG
GATGAGAGGGAAGTATCCGTTCCGGCGGAGATCCTGCGGAGGTCCAGGAAATTC
CCTCGAGCGATGCCCATATGGCACGCCCGGATTACAACCCTCCACTGTTAGAGT
CCTGGAAGGACCCGGACTACGTCCCTCCAGTGGTACACGGGTGTCCATTGCCGCC
TGCCAAGGCCCCTCCGATACCACCTCCACGGAGGAAGAGGACGGTTGTCCTGTCA
GAATCTACCGTGTCTTCTGCCTTGGCGGAGCTCGCCACAAAGACCTTCGGCAGCT
CCGAATCGTCGGCCGTCGACAGCGGCACGGCAACGGCCTCTCCTGACCAGCCCTC
CGACGACGGCGACGCGGGATCCGACGTTGAGTCGTACTCCTCCATGCCCCCCCTT
GAGGGGGAGCCGGGGGATCCCGATCTCAGCGACGGGTCTTGGTCTACCGTAAGC
GAGGAGGCTAGTGAGGACGTCGTCTGCTGCTCGATGTCCTACACATGGACAGGC
GCCCTGATCACGCCATGCGCTGCGGAGGAAACCAAGCTGCCCATCAATGCACTG
AGCAACTCTTTGCTCCGTCACCACAACTTGGTCTATGCTACAACATCTCGCAGCG
CAAGCCTGCGGCAGAAGAAGGTCACCTTTGACAGACTGCAGGTCCTGGACGACC
ACTACCGGGACGTGCTCAAGGAGATGAAGGCGAAGGCGTCCACAGTTAAGGCTA
AACTTCTATCCGTGGAGGAAGCCTGTAAGCTGACGCCCCCACATTCGGCCAGATC
TAAATTTGGCTATGGGGCAAAGGACGTCCGGAACCTATCCAGCAAGGCCGTTAA
CCACATCCGCTCCGTGTGGAAGGACTTGCTGGAAGACACTGAGACACCAATTGAC
ACCACCATCATGGCAAAAAATGAGGTTTTCTGCGTCCAACCAGAGAAGGGGGGC
CGCAAGCCAGCTCGCCTTATCGTATTCCCAGATTTGGGGGTTCGTGTGTGCGAGA
AAATGGCCCTTTACGATGTGGTCTCCACCCTCCCTCAGGCCGTGATGGGCTCTTCA
TACGGATTCCAATACTCTCCTGGACAGCGGGTCGAGTTCCTGGTGAATGCCTGGA
AAGCGAAGAAATGCCCTATGGGCTTCGCATATGACACCCGCTGTTTTGACTCAAC
GGTCACTGAGAATGACATCCGTGTTGAGGAGTCAATCTACCAATGTTGTGACTTG
GCCCCCGAAGCCAGACAGGCCATAAGGTCGCTCACAGAGCGGCTTTACATCGGG
GGCCCCCTGACTAATTCTAAAGGGCAGAACTGCGGCTATCGCCGGTGCCGCGCGA
GCGGTGTACTGACGACCAGCTGCGGTAATACCCTCACATGTTACTTGAAGGCCGC
TGCGGCCTGTCGAGCTGCGAAGCTCCAGGACTGCACGATGCTCGTATGCGGAGAC
GACCTTGTCGTTATCTGTGAAAGCGCGGGGACCCAAGAGGACGAGGCGAGCCTA
CGGGCCTTCACGGAGGCTATGACTAGATACTCTGCCCCCCCTGGGGACCCGCCCA
AACCAGAATACGACTTGGAGTTGATAACATCATGCTCCTCCAATGTGTCAGTCGC
GCACGATGCATCTGGCAAAAGGGTGTACTATCTCACCCGTGACCCCACCACCCCC
CTTGCGCGGGCTGCGTGGGAGACAGCTAGACACACTCCAGTCAATTCCTGGCTAG
GCAACATCATCATGTATGCGCCCACCTTGTGGGCAAGGATGATCCTGATGACTCA
TTTCTTCTCCATCCTTCTAGCTCAGGAACAACTTGAAAAAGCCCTAGATTGTCAGA
TCTACGGGGCCTGTTACTCCATTGAGCCACTTGACCTACCTCAGATCATTCAACG
ACTCCATGGCCTTAGCGCATTTTCACTCCATAGTTACTCTCCAGGTGAGATCAATA
GGGTGGCTTCATGCCTCAGGAAACTTGGGGTACCGCCCTTGCGAGTCTGGAGACA
TCGGGCCAGAAGTGTCCGCGCTAGGCTACTGTCCCAGGGGGGGAGGGCTGCCAC
TTGTGGCAAGTACCTCTTCAACTGGGCAGTAAGGACCAAGCTCAAACTCACTCCA
ATCCCGGCTGCGTCCCAGTTGGATTTATCCAGCTGGTTCGTTGCTGGTTACAGCGG
GGGAGACATATATCACAGCCTGTCTCGTGCCCGACCCCGCTGGTTCATGTGGTGC
CTACTCCTACTTTCTGTAGGGGTAGGCATCTATCTACTCCCCAACCGATGAACGG
GGACCTAAACACTCCAGGCCAATAGGCCATCCTGTTTTTTTCCCTTTTTTTTTTTCT
TTTTTTTTTTTTTTTTTTTTTTTTTTTTTTTCTCCTTTTTTTTTCCTCTTTTTTTCCTT
TTCTTTCCTTTGGTGGCTCCATCTTAGCCCTAGTCACGGCTAGCTGTGAAAGGTCC
GTGAGCCGCTTGACTGCAGAGAGTGCTGATACTGGCCTCTCTGCAGATCAAGT

SEQ ID NO:23: Nucleotide sequence of full-length HCV cDNA clone containing the mutation that results in Ser to Ile at position 1179 of SEQ ID NO:3, and where the 5' NTR. is fused to the neomycin phosphotransferase gene and the EMCV IRES is inserted upstream of the HCV open reading frame (see Figure 9)

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAAGGGCGC
GCCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGT
GTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGAT
CCGGCTACCTGCCCATTCGACCACCAAGCGAAACATCGCATCGAGCGAGCACGT
ACTCGGATGGAAGCCGGTCTTGTCGATCAGGATGATCTGGACGAAGAGCATCAG
GGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTA
TCAGGACATAGCGTTGGCTACCCGTGATATTGCTGAAGAGCTTGGCGGCGAATGG
GCTGACCGCTTCCTCGTGCTTTACGGTATCGCCGCTCCCGATTCGCAGCGCATCGC
CTTCTATCGCCTTCTTGACGAGTTCTTCTGAGTTTAAACAGACCACAACGGTTTCC
CTCTAGCGGGATCAATTCCGCCCCTCTCCCTCCCCCCCCCCTAACGTTACTGGCCG
AAGCCGCTTGGAATAAGGCCGGTGTGCGTTTGTCTATATGTTATTTTCCACCATAT
TGCCGTCTTTTGGCAATGTGAGGGCCCGGAAACCTGGCCCTGTCTTCTTGACGAG
CATTCCTAGGGGTCTTTCCCCTCTCGCCAAAGGAATGCAAGGTCTGTTGAATGTC
GTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAA
AGCCACGTGTATAAGATACACCTGCAAAGGCGGCACAACCCCAGTGCCACGTTG
TGAGTTGGATAGTTGTGGAAAGAGTCAAATGGCTCTCCTCAAGCGTATTCAACAA
GGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCC
GAACCACGGGGACGTGGTTTTCCTTTGAAAAACACGATAATAATGAGCACGAAT
CCTAAACCTCAAAGAAAAACCAAACGTAACACCAACCGCCGCCCACAGGACGTC
AAGTTCCCGGGCGGTGGTCAGATCGTCGGTGGAGTTTACCTGTTGCCGCGCAGGG
GCCCCAGGTTGGGTGTGCGCGCGACTAGGAAGACTTCCGAGCGGTCGCAACCTC
GTGGAAGGCGACAACCTATCCCCAAGGCTCGCCAGCCCGAGGGTAGGGCCTGGG
CTCAGCCCGGGTACCCCTGGCCCCTCTATGGCAATGAGGGCTTGGGGTGGGCAGG
ATGGCTCCTGTCACCCCGTGGCTCTCGGCCTAGTTGGGGCCCCACGGACCCCCGG
CGTAGGTCGCGCAATTTGGGTAAGGTCATCGATACCCTCACGTGCGGCTTCGCCG
ATCTCATGGGGTACATTCCGCTCGTCGGCGCCCCCCTAGGGGGCGCTGCCAGGGC
CCTGGCGCATGGCGTCCGGGTTCTGGAGGACGGCGTGAACTATGCAACAGGGAA
TCTGCCCGGTTGCTCCTTTTCTATCTTCCTTTTGGCTTTGCTGTCCTGTTTGACCAT
CCCAGCTTCCGCTTATGAAGTGCGCAACGTATCCGGAGTGTACCATGTCACGAAC
GACTGCTCCAACGCAAGCATTGTGTATGAGGCAGCGGACATGATCATGCATACCC
CCGGGTGCGTGCCCTGCGTTCGGGAGAACAACTCCTCCCGCTGCTGGGTAGCGCT
CACTCCCACGCTCGCGGCCAGGAACGCTAGCGTCCCCACTACGACGATACGACGC
CATGTCGATTTGCTCGTTGGGGCGGCTGCTCTCTGCTCCGCTATGTACGTGGGAG

ATCTCTGCGGATCTGTTTCCTCGTCGCCCAGCTGTTCACCTTCTCGCCTCGCCGG
CACGAGACAGTACAGGACTGCAATTGCTCAATATATCCCGGCCACGTGACAGGTC
ACCGTATGGCTTGGGATATGATGATGAACTGGTCACCTACAGCAGCCCTAGTGGT
ATCGCAGTTACTCCGGATCCCACAAGCTGTCGTGGATATGGTGGCGGGGGCCCAT
TGGGGAGTCCTAGCGGGCCTTGCCTACTATTCCATGGTGGGGAACTGGGCTAAGG
TTCTGATTGTGATGCTACTCTTTGCCGGCGTTGACGGGGGAACCTATGTGACAGG
GGGGACGATGGCCAAAAACACCCTCGGGATTACGTCCCTCTTTTCACCCGGGTCA
TCCCAGAAAATCCAGCTTGTAAACACCAACGGCAGCTGGCACATCAACAGGACT
GCCCTGAACTGCAATGACTCCCTCAACACTGGGTTCCTTGCTGCGCTGTTCTACGT
GCACAAGTTCAACTCATCTGGATGCCCAGAGCGCATGGCCAGCTGCAGCCCCATC
GACGCGTTCGCTCAGGGGTGGGGGCCCATCACTTACAATGAGTCACACAGCTCGG
ACCAGAGGCCTTATTGTTGGCACTACGCACCCCGGCCGTGCGGTATCGTACCCGC
GGCGCAGGTGTGTGGTCCAGTGTACTGCTTCACCCCAAGCCCTGTCGTGGTGGG
ACGACCGACCGGTTCGGCGTCCCTACGTACAGTTGGGGGGAGAATGAGACGGAC
GTGCTGCTTCTTAACAACACGCGGCCGCCGCAAGGCAACTGGTTTGGCTGTACAT
GGATGAATAGCACTGGGTTCACCAAGACGTGCGGGGGCCCCCCGTGTAACATCG
GGGGGATCGGCAATAAAACCTTGACCTGCCCCACGGACTGCTTCCGGAAGCACC
CCGAGGCCACTTACACCAAGTGTGGTTCGGGGCCTTGGTTGACACCCAGATGCTT
GGTCCACTACCCATACAGGCTTTGGCACTACCCCTGCACTGTCAACTTTACCATCT
TCAAGGTTAGGATGTACGTGGGGGGAGTGGAGCACAGGCTCGAAGCCGCATGCA
ATTGGACTCGAGGAGAGCGTTGTAACCTGGAGGACAGGGACAGATCAGAGCTTA
GCCCGCTGCTGCTGTCTACAACGGAGTGGCAGGTATTGCCCTGTTCCTTCACCAC
CCTACCGGCTCTGTCCACTGGTTTGATCCATCTCCATCAGAACGTCGTGGACGTAC
AATACCTGTACGGTATAGGGTCGGCGGTTGTCTCCTTTGCAATCAAATGGGAGTA
TGTCCTGTTGCTCTTCCTTCTTCTGGCGGACGCGCGCGTCTGTGCCTGCTTGTGGA
TGATGCTGCTGATAGCTCAAGCTGAGGCCGCCCTAGAGAACCTGGTGGTCCTCAA
CGCGGCATCCGTGGCCGGGGCGCATGGCATTCTCTCCTTCCTCGTGTTCTTCTGTG
CTGCCTGGTACATCAAGGGCAGGCTGGTCCCTGGGGCGGCATATGCCCTCTACGG
CGTATGGCCGCTACTCCTGCTCCTGCTGGCGTTACCACCACGAGCATACGCCATG
GACCGGGAGATGGCAGCATCGTGCGGAGGCGCGGTTTTCGTAGGTCTGATACTCT
TGACCTTGTCACCGCACTATAAGCTGTTCCTCGCTAGGCTCATATGGTGGTTACAA
TATTTTATCACCAGGGCCGAGGCACACTTGCAAGTGTGGATCCCCCCCCTCAACG
TTCGGGGGGGCCGCGATGCCGTCATCCTCCTCACGTGCGCGATCCACCCAGAGCT
AATCTTTACCATCACCAAAATCTTGCTCGCCATACTCGGTCCACTCATGGTGCTCC
AGGCTGGTATAACCAAAGTGCCGTACTTCGTGCGCGCACACGGGCTCATTCGTGC
ATGCATGCTGGTGCGGAAGGTTGCTGGGGGTCATTATGTCCAAATGGCTCTCATG
AAGTTGGCCGCACTGACAGGTACGTACGTTTATGACCATCTCACCCCACTGCGGG
ACTGGGCCCACGCGGGCCTACGAGACCTTGCGGTGGCAGTTGAGCCCGTCGTCTT
CTCTGATATGGAGACCAAGGTTATCACCTGGGGGGCAGACACCGCGGCGTGTGG
GGACATCATCTTGGGCCTGCCCGTCTCCGCCCGCAGGGGGAGGGAGATACATCTG
GGACCGGCAGACAGCCTTGAAGGGCAGGGGTGGCGACTCCTCGCGCCTATTACG
GCCTACTCCCAACAGACGCGAGGCCTACTTGGCTGCATCATCACTAGCCTCACAG
GCCGGGACAGGAACCAGGTCGAGGGGGAGGTCCAAGTGGTCTCCACCGCAACAC
AATCTTTCCTGGCGACCTGCGTCAATGGCGTGTGTTGGACTGTCTATCATGGTGCC
GGCTCAAAGACCCTTGCCGGCCCAAAGGGCCCAATCACCCAAATGTACACCAAT
GTGGACCAGGACCTCGTCGGCTGGCAAGCGCCCCCCGGGGCGCGTTCCTTGACAC
CATGCACCTGCGGCAGCTCGGACCTTTACTTGGTCACGAGGCATGCCGATGTCAT
TCCGGTGCGCCGGCGGGGCGACAGCAGGGGGAGCCTACTCTCCCCCAGGCCCGT
CTCCTACTTGAAGGGCTCTTCGGGCGGTCCACTGCTCTGCCCCTCGGGGCACGCT
GTGGGCATCTTTCGGGCTGCCGTGTGCACCCGAGGGGTTGCGAAGGCGGTGGACT
TTGTACCCGTCGAGTCTATGGAAACCACTATGCGGTCCCCGGTCTTCACGGACAA
CTCGTCCCCTCCGGCCGTACCGCAGACATTCCAGGTGGCCCATCTACACGCCCCT
ACTGGTAGCGGCAAGAGCACTAAGGTGCCGGCTGCGTATGCAGCCCAAGGGTAT

AAGGTGCTTGTCCTGAACCCGTCCGTCGCCGCCACCCTAGGTTTCGGGGCGTATA
TGTCTAAGGCACATGGTATCGACCCTAACATCAGAACCGGGGTAAGGACCATCA
CCACGGGTGCCCCCATCACGTACTCCACCTATGGCAAGTTTCTTGCCGACGGTGG
TTGCTCTGGGGGCGCCTATGACATCATAATATGTGATGAGTGCCACTCAACTGAC
TCGACCACTATCCTGGGCATCGGCACAGTCCTGGACCAAGCGGAGACGGCTGGA
GCGCGACTCGTCGTGCTCGCCACCGCTACGCCTCCGGGATCGGTCACCGTGCCAC
ATCCAAACATCGAGGAGGTGGCTCTGTCCAGCACTGGAGAAATCCCCTTTTATGG
CAAAGCCATCCCCATCGAGACCATCAAGGGGGGGAGGCACCTCATTTCTGCCAT
TCCAAGAAGAAATGTGATGAGCTCGCCGCGAAGCTGTCCGGCCTCGGACTCAAT
GCTGTAGCATATTACCGGGGCCTTGATGTATCCGTCATACCAACTAGCGGAGACG
TCATTGTCGTAGCAACGGACGCTCTAATGACGGGCTTTACCGGCGATTTCGACTC
AGTGATCGACTGCAATACATGTGTCACCCAGACAGTCGACTTCAGCCTGGACCCG
ACCTTCACCATTGAGACGACGACCGTGCCACAAGACGCGGTGTCACGCTCGCAGC
GGCGAGGCAGGACTGGTAGGGGCAGGATGGGCATTTACAGGTTTGTGACTCCAG
GAGAACGGCCCTCGGGCATGTTCGATTCCTCGGTTCTGTGCGAGTGCTATGACGC
GGGCTGTGCTTGGTACGAGCTCACGCCCGCCGAGACCTCAGTTAGGTTGCGGGCT
TACCTAAACACACCAGGGTTGCCCGTCTGCCAGGACCATCTGGAGTTCTGGGAGA
GCGTCTTTACAGGCCTCACCCACATAGACGCCCATTTCTTGTCCCAGACTAAGCA
GGCAGGAGACAACTTCCCCTACCTGGTAGCATACCAGGCTACGGTGTGCGCCAG
GGCTCAGGCTCCACCTCCATCGTGGGACCAAATGTGGAAGTGTCTCATACGGCTA
AAGCCTACGCTGCACGGGCCAACGCCCCTGCTGTATAGGCTGGGAGCCGTTCAAA
ACGAGGTTACTACCACACACCCCATAACCAAATACATCATGGCATGCATGTCGGC
TGACCTGGAGGTCGTCACGAGCACCTGGGTGCTGGTAGGCGGAGTCCTAGCAGCT
CTGGCCGCGTATTGCCTGACAACAGGCAGCGTGGTCATTGTGGGCAGGATCATCT
TGTCCGGAAAGCCGGCCATCATTCCCGACAGGGAAGTCCTTTACCGGGAGTTCGA
TGAGATGGAAGAGTGCGCCTCACACCTCCCTTACATCGAACAGGGAATGCAGCTC
GCCGAACAATTCAAACAGAAGGCAATCGGGTTGCTGCAAACAGCCACCAAGCAA
GCGGAGGCTGCTGCTCCCGTGGTGGAATCCAAGTGGCGGACCCTCGAAGCCTTCT
GGGCGAAGCATATGTGGAATTTCATCAGCGGGATACAATATTTAGCAGGCTTGTC
CACTCTGCCTGGCAACCCCGCGATAGCATCACTGATGGCATTCACAGCCTCTATC
ACCAGCCCGCTCACCACCCAACATACCCTCCTGTTTAACATCCTGGGGGGATGGG
TGGCCGCCCAACTTGCTCCTCCCAGCGCTGCTTCTGCTTTCGTAGGCGCCGGCATC
GCTGGAGCGGCTGTTGGCAGCATAGGCCTTGGGAAGGTGCTTGTGGATATTTTGG
CAGGTTATGGAGCAGGGGTGGCAGGCGCGCTCGTGGCCTTTAAGGTCATGAGCG
GCGAGATGCCCTCCACCGAGGACCTGGTTAACCTACTCCCTGCTATCCTCTCCCCT
GGCGCCCTAGTCGTCGGGGTCGTGTGCGCAGCGATACTGCGTCGGCACGTGGGCC
CAGGGGAGGGGGCTGTGCAGTGGATGAACCGGCTGATAGCGTTCGCTTCGCGGG
GTAACCACGTCTCCCCCACGCACTATGTGCCTGAGAGCGACGCTGCAGCACGTGT
CACTCAGATCCTCTCTAGTCTTACCATCACTCAGCTGCTGAAGAGGCTTCACCAGT
GGATCAACGAGGACTGCTCCACGCCATGCTCCGGCTCGTGGCTAAGAGATGTTTG
GGATTGGATATGCACGGTGTTGACTGATTTCAAGACCTGGCTCCAGTCCAAGCTC
CTGCCGCGATTGCCGGGAGTCCCCTTCTTCTCATGTCAACGTGGGTACAAGGGAG
TCTGGCGGGGCGACGGCATCATGCAAACCACCTGCCCATGTGGAGCACAGATCA
CCGGACATGTGAAAAACGGTTCCATGAGGATCGTGGGGCCTAGGACCTGTAGTA
ACACGTGGCATGGAACATTCCCCATTAACGCGTACACCACGGGCCCCTGCACGCC
CTCCCCGGCGCCAAATTATTCTAGGGCGCTGTGGCGGGTGGCTGCTGAGGAGTAC
GTGGAGGTTACGCGGGTGGGGGATTTCCACTACGTGACGGGCATGACCACTGAC
AACGTAAAGTGCCCGTGTCAGGTTCCGGCCCCCGAATTCTTCACAGAAGTGGATG
GGGTGCGGTTGCACAGGTACGCTCCAGCGTGCAAACCCCTCCTACGGGAGGAGG
TCACATTCCTGGTCGGGCTCAATCAATACCTGGTTGGGTCACAGCTCCCATGCGA
GCCCGAACCGGACGTAGCAGTGCTCACTTCCATGCTCACCGACCCCTCCCACATT
ACGGCGGAGACGGCTAAGCGTAGGCTGGCCAGGGGATCTCCCCCCTCCTTGGCC
AGCTCATCAGCTATCCAGCTGTCTGCGCCTTCCTTGAAGGCAACATGCACTACCC

GTCATGACTCCCCGGACGCTGACCTCATCGAGGCCAACCTCCTGTGGCGGCAGGA
GATGGGCGGGAACATCACCCGCGTGGAGTCAGAAAATAAGGTAGTAATTTTGGA
CTCTTTCGAGCCGCTCCAAGCGGAGGAGGATGAGAGGGAAGTATCCGTTCCGGC
GGAGATCCTGCGGAGGTCCAGGAAATTCCCTCGAGCGATGCCCATATGGGCACG
CCCGGATTACAACCCTCCACTGTTAGAGTCCTGGAAGGACCCGGACTACGTCCCT
CCAGTGGTACACGGGTGTCCATTGCCGCCTGCCAAGGCCCCTCCGATACCACCTC
CACGGAGGAAGAGGACGGTTGTCCTGTCAGAATCTACCGTGTCTTCTGCCTTGGC
GGAGCTCGCCACAAAGACCTTCGGCAGCTCCGAATCGTCGGCCGTCGACAGCGG
CACGGCAACGGCCTCTCCTGACCAGCCCTCCGACGACGGCGACGCGGGATCCGA
CGTTGAGTCGTACTCCTCCATGCCCCCCCTTGAGGGGGAGCCGGGGGATCCCGAT
CTCAGCGACGGGTCTTGGTCTACCGTAAGCGAGGAGGCTAGTGAGGACGTCGTCT
GCTGCTCGATGTCCTACACATGGACAGGCGCCCTGATCACGCCATGCGCTGCGGA
GGAAACCAAGCTGCCCATCAATGCACTGAGCAACTCTTTGCTCCGTCACCACAAC
TTGGTCTATGCTACAACATCTCGCAGCGCAAGCCTGCGGCAGAAGAAGGTCACCT
TTGACAGACTGCAGGTCCTGGACGACCACTACCGGGACGTGCTCAAGGAGATGA
AGGCGAAGGCGTCCACAGTTAAGGCTAAACTTCTATCCGTGGAGGAAGCCTGTA
AGCTGACGCCCCCACATTCGGCCAGATCTAAATTTGGCTATGGGGCAAAGGACGT
CCGGAACCTATCCAGCAAGGCCGTTAACCACATCCGCTCCGTGTGGAAGGACTTG
CTGGAAGACACTGAGACACCAATTGACACCACCATCATGGCAAAAAATGAGGTT
TTCTGCGTCCAACCAGAGAAGGGGGGCCGCAAGCCAGCTCGCCTTATCGTATTCC
CAGATTTGGGGGTTCGTGTGTGCGAGAAAATGGCCCTTTACGATGTGGTCTCCAC
CCTCCCTCAGGCCGTGATGGGCTCTTCATACGGATTCCAATACTCTCCTGGACAG
CGGGTCGAGTTCCTGGTGAATGCCTGGAAAGCGAAGAAATGCCCTATGGGCTTCG
CATATGACACCCGCTGTTTTGACTCAACGGTCACTGAGAATGACATCCGTGTTGA
GGAGTCAATCTACCAATGTTGTGACTTGGCCCCCGAAGCCAGACAGGCCATAAG
GTCGCTCACAGAGCGGCTTTACATCGGGGGCCCCCTGACTAATTCTAAAGGGCAG
AACTGCGGCTATCGCCGGTGCCGCGCGAGCGGTGTACTGACGACCAGCTGCGGT
AATACCCTCACATGTTACTTGAAGGCCGCTGCGGCCTGTCGAGCTGCGAAGCTCC
AGGACTGCACGATGCTCGTATGCGGAGACGACCTTGTCGTTATCTGTGAAAGCGC
GGGGACCCAAGAGGACGAGGCGAGCCTACGGGCCTTCACGGAGGCTATGACTAG
ATACTCTGCCCCCCCTGGGGACCCGCCCAAACCAGAATACGACTTGGAGTTGATA
ACATCATGCTCCTCCAATGTGTCAGTCGCGCACGATGCATCTGGCAAAAGGGTGT
ACTATCTCACCCGTGACCCCACCACCCCCCTTGCGCGGGCTGCGTGGGAGACAGC
TAGACACACTCCAGTCAATTCCTGGCTAGGCAACATCATCATGTATGCGCCCACC
TTGTGGGCAAGGATGATCCTGATGACTCATTTCTTCTCCATCCTTCTAGCTCAGGA
ACAACTTGAAAAAGCCCTAGATTGTCAGATCTACGGGGCCTGTTACTCCATTGAG
CCACTTGACCTACCTCAGATCATTCAACGACTCCATGGCCTTAGCGCATTTTCACT
CCATAGTTACTCTCCAGGTGAGATCAATAGGGTGGCTTCATGCCTCAGGAAACTT
GGGGTACCGCCCTTGCGAGTCTGGAGACATCGGGCCAGAAGTGTCCGCGCTAGG
CTACTGTCCCAGGGGGGGAGGGCTGCCACTTGTGGCAAGTACCTCTTCAACTGGG
CAGTAAGGACCAAGCTCAAACTCACTCCAATCCCGGCTGCGTCCCAGTTGGATTT
ATCCAGCTGGTTCGTTGCTGGTTACAGCGGGGGAGACATATATCACAGCCTGTCT
CGTGCCCGACCCCGCTGGTTCATGTGGTGCCTACTCCTACTTTCTGTAGGGGTAGG
CATCTATCTACTCCCCAACCGATGAACGGGGACCTAAACACTCCAGGCCAATAGG
CCATCCTGTTTTTTTCCCTTTTTTTTTTTCTTTTTTTTTTTTTTTTTTTTTTTTTTTT
TTTTCTCCTTTTTTTTTCCTCTTTTTTTCCTTTTCTTTCCTTTGGTGGCTCCATCTTAG
CCCTAGTCACGGCTAGCTGTGAAAGGTCCGTGAGCCGCTTGACTGCAGAGAGTGC
TGATACTGGCCTCTCTGCAGATCAAGT

SEQ ID NO:24: Nucleotide sequence of full-length HCV cDNA clone containing the mutation that results in Ser to Ile at position 1179 of SEQ ID NO:3 (see Figure 9)

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCATGAGCACGAATCCTAAACCTCAAAGAAAAACCAAACGTAAC
ACCAACCGCCGCCCACAGGACGTCAAGTTCCCGGGCGGTGGTCAGATCGTCGGT
GGAGTTTACCTGTTGCCGCGCAGGGGCCCCAGGTTGGGTGTGCGCGCGACTAGGA
AGACTTCCGAGCGGTCGCAACCTCGTGGAAGGCGACAACCTATCCCCAAGGCTC
GCCAGCCCGAGGGTAGGGCCTGGGCTCAGCCCGGGTACCCCTGGCCCCTCTATGG
CAATGAGGGCTTGGGGTGGGCAGGATGGCTCCTGTCACCCCGTGGCTCTCGGCCT
AGTTGGGGCCCCACGGACCCCCGGCGTAGGTCGCGCAATTTGGGTAAGGTCATCG
ATACCCTCACGTGCGGCTTCGCCGATCTCATGGGGTACATTCCGCTCGTCGGCGC
CCCCCTAGGGGGCGCTGCCAGGGCCCTGGCGCATGGCGTCCGGGTTCTGGAGGA
CGGCGTGAACTATGCAACAGGGAATCTGCCCGGTTGCTCCTTTTCTATCTTCCTTT
TGGCTTTGCTGTCCTGTTTGACCATCCCAGCTTCCGCTTATGAAGTGCGCAACGTA
TCCGGAGTGTACCATGTCACGAACGACTGCTCCAACGCAAGCATTGTGTATGAGG
CAGCGGACATGATCATGCATACCCCCGGGTGCGTGCCCTGCGTTCGGGAGAACA
ACTCCTCCCGCTGCTGGGTAGCGCTCACTCCCACGCTCGCGGCCAGGAACGCTAG
CGTCCCCACTACGACGATACGACGCCATGTCGATTTGCTCGTTGGGGCGGCTGCT
CTCTGCTCCGCTATGTACGTGGGAGATCTCTGCGGATCTGTTTCCTCGTCGCCCA
GCTGTTCACCTTCTCGCCTCGCCGGCACGAGACAGTACAGGACTGCAATTGCTCA
ATATATCCCGGCCACGTGACAGGTCACCGTATGGCTTGGGATATGATGATGAACT
GGTCACCTACAGCAGCCCTAGTGGTATCGCAGTTACTCCGGATCCCACAAGCTGT
CGTGGATATGGTGGCGGGGGCCCATTGGGGAGTCCTAGCGGGCCTTGCCTACTAT
TCCATGGTGGGGAACTGGGCTAAGGTTCTGATTGTGATGCTACTCTTTGCCGGCG
TTGACGGGGGAACCTATGTGACAGGGGGGACGATGGCCAAAAACACCCTCGGGA
TTACGTCCCTCTTTTCACCCGGGTCATCCCAGAAAATCCAGCTTGTAAACACCAA
CGGCAGCTGGCACATCAACAGGACTGCCCTGAACTGCAATGACTCCCTCAACACT
GGGTTCCTTGCTGCGCTGTTCTACGTGCACAAGTTCAACTCATCTGGATGCCCAG
AGCGCATGGCCAGCTGCAGCCCCATCGACGCGTTCGCTCAGGGGTGGGGGCCCA
TCACTTACAATGAGTCACACAGCTCGGACCAGAGGCCTTATTGTTGGCACTACGC
ACCCCGGCCGTGCGGTATCGTACCCGCGGCGCAGGTGTGTGGTCCAGTGTACTGC
TTCACCCCAAGCCCTGTCGTGGTGGGGACGACCGACCGGTTCGGCGTCCCTACGT
ACAGTTGGGGGGAGAATGAGACGGACGTGCTGCTTCTTAACAACACGCGGCCGC
CGCAAGGCAACTGGTTTGGCTGTACATGGATGAATAGCACTGGGTTCACCAAGAC
GTGCGGGGGCCCCCCGTGTAACATCGGGGGGATCGGCAATAAAACCTTGACCTG
CCCCACGGACTGCTTCCGGAAGCACCCCGAGGCCACTTACACCAAGTGTGGTTCG
GGGCCTTGGTTGACACCCAGATGCTTGGTCCACTACCCATACAGGCTTTGGCACT
ACCCCTGCACTGTCAACTTTACCATCTTCAAGGTTAGGATGTACGTGGGGGGAGT
GGAGCACAGGCTCGAAGCCGCATGCAATTGGACTCGAGGAGAGCGTTGTAACCT
GGAGGACAGGGACAGATCAGAGCTTAGCCCGCTGCTGCTGTCTACAACGGAGTG
GCAGGTATTGCCCTGTTCCTTCACCACCCTACCGGCTCTGTCCACTGGTTTGATCC
ATCTCCATCAGAACGTCGTGGACGTACAATACCTGTACGGTATAGGGTCGGCGGT
TGTCTCCTTTGCAATCAAATGGGAGTATGTCCTGTTGCTCTTCCTTCTTCTGGCGG
ACGCGCGCGTCTGTGCCTGCTTGTGGATGATGCTGCTGATAGCTCAAGCTGAGGC
CGCCCTAGAGAACCTGGTGGTCCTCAACGCGGCATCCGTGGCCGGGGCGCATGG
CATTCTCTCCTTCCTCGTGTTCTTCTGTGCTGCCTGGTACATCAAGGGCAGGCTGG
TCCCTGGGGCGGCATATGCCCTCTACGGCGTATGGCCGCTACTCCTGCTCCTGCTG
GCGTTACCACCACGAGCATACGCCATGGACCGGGAGATGGCAGCATCGTGCGGA
GGCGCGGTTTTCGTAGGTCTGATACTCTTGACCTTGTCACCGCACTATAAGCTGTT
CCTCGCTAGGCTCATATGGTGGTTACAATATTTATCACCAGGGCCGAGGCACAC

TTGCAAGTGTGGATCCCCCCCTCAACGTTCGGGGGGCCGCGATGCCGTCATCC
TCCTCACGTGCGCGATCCACCCAGAGCTAATCTTTACCATCACCAAAATCTTGCTC
GCCATACTCGGTCCACTCATGGTGCTCCAGGCTGGTATAACCAAAGTGCCGTACT
TCGTGCGCGCACACGGGCTCATTCGTGCATGCATGCTGGTGCGGAAGGTTGCTGG
GGGTCATTATGTCCAAATGGCTCTCATGAAGTTGGCCGCACTGACAGGTACGTAC
GTTTATGACCATCTCACCCCACTGCGGGACTGGGCCCACGCGGCCTACGAGACC
TTGCGGTGGCAGTTGAGCCCGTCGTCTTCTCTGATATGGAGACCAAGGTTATCAC
CTGGGGGGCAGACACCGCGGCGTGTGGGGACATCATCTTGGGCCTGCCCGTCTCC
GCCCGCAGGGGGAGGGAGATACATCTGGGACCGGCAGACAGCCTTGAAGGGCAG
GGGTGGCGACTCCTCGCGCCTATTACGGCCTACTCCCAACAGACGCGAGGCCTAC
TTGGCTGCATCATCACTAGCCTCACAGGCCGGGACAGGAACCAGGTCGAGGGGG
AGGTCCAAGTGGTCTCCACCGCAACACAATCTTTCCTGGCGACCTGCGTCAATGG
CGTGTGTTGGACTGTCTATCATGGTGCCGGCTCAAAGACCCTTGCCGGCCCAAAG
GGCCCAATCACCCAAATGTACACCAATGTGGACCAGGACCTCGTCGGCTGGCAA
GCGCCCCCCGGGGCGCGTTCCTTGACACCATGCACCTGCGGCAGCTCGGACCTTT
ACTTGGTCACGAGGCATGCCGATGTCATTCCGGTGCGCCGGCGGGGCGACAGCA
GGGGGAGCCTACTCTCCCCCAGGCCCGTCTCCTACTTGAAGGGCTCTTCGGGCGG
TCCACTGCTCTGCCCCTCGGGGCACGCTGTGGGCATCTTTCGGGCTGCCGTGTGC
ACCCGAGGGGTTGCGAAGGCGGTGGACTTTGTACCCGTCGAGTCTATGGAAACC
ACTATGCGGTCCCCGGTCTTCACGGACAACTCGTCCCCTCCGGCCGTACCGCAGA
CATTCCAGGTGGCCCATCTACACGCCCCTACTGGTAGCGGCAAGAGCACTAAGGT
GCCGGCTGCGTATGCAGCCCAAGGGTATAAGGTGCTTGTCCTGAACCCGTCCGTC
GCCGCCACCCTAGGTTTCGGGGCGTATATGTCTAAGGCACATGGTATCGACCCTA
ACATCAGAACCGGGGTAAGGACCATCACCACGGGTGCCCCCATCACGTACTCCA
CCTATGGCAAGTTTCTTGCCGACGGTGGTTGCTCTGGGGGCGCCTATGACATCAT
AATATGTGATGAGTGCCACTCAACTGACTCGACCACTATCCTGGGCATCGGCACA
GTCCTGGACCAAGCGGAGACGGCTGGAGCGCGACTCGTCGTGCTCGCCACCGCT
ACGCCTCCGGGATCGGTCACCGTGCCACATCCAAACATCGAGGAGGTGGCTCTGT
CCAGCACTGGAGAAATCCCCTTTTATGGCAAAGCCATCCCCATCGAGACCATCAA
GGGGGGGAGGCACCTCATTTTCTGCCATTCCAAGAAGAAATGTGATGAGCTCGCC
GCGAAGCTGTCCGGCCTCGGACTCAATGCTGTAGCATATTACCGGGGCCTTGATG
TATCCGTCATACCAACTAGCGGAGACGTCATTGTCGTAGCAACGGACGCTCTAAT
GACGGGCTTTACCGGCGATTTCGACTCAGTGATCGACTGCAATACATGTGTCACC
CAGACAGTCGACTTCAGCCTGGACCCGACCTTCACCATTGAGACGACGACCGTGC
CACAAGACGCGGTGTCACGCTCGCAGCGGCGAGGCAGGACTGGTAGGGGCAGGA
TGGGCATTTACAGGTTTGTGACTCCAGGAGAACGGCCCTCGGGCATGTTCGATTC
CTCGGTTCTGTGCGAGTGCTATGACGCGGGCTGTGCTTGGTACGAGCTCACGCCC
GCCGAGACCTCAGTTAGGTTGCGGGCTTACCTAAACACACCAGGGTTGCCCGTCT
GCCAGGACCATCTGGAGTTCTGGGAGAGCGTCTTTACAGGCCTCACCCACATAGA
CGCCCATTTCTTGTCCCAGACTAAGCAGGCAGGAGACAACTTCCCCTACCTGGTA
GCATACCAGGCTACGGTGTGCGCCAGGGCTCAGGCTCCACCTCCATCGTGGACC
AAATGTGGAAGTGTCTCATACGGCTAAAGCCTACGCTGCACGGGCCAACGCCCCT
GCTGTATAGGCTGGGAGCCGTTCAAAACGAGGTTACTACCACACACCCCATAACC
AAATACATCATGGCATGCATGTCGGCTGACCTGGAGGTCGTCACGAGCACCTGGG
TGCTGGTAGGCGGAGTCCTAGCAGCTCTGGCCGCGTATTGCCTGACAACAGGCAG
CGTGGTCATTGTGGGCAGGATCATCTTGTCCGGAAAGCCGGCCATCATTCCCGAC
AGGGAAGTCCTTTACCGGGAGTTCGATGAGATGGAAGAGTGCGCCTCACACCTCC
CTTACATCGAACAGGGAATGCAGCTCGCCGAACAATTCAAACAGAAGGCAATCG
GGTTGCTGCAAACAGCCACCAAGCAAGCGGAGGCTGCTGCTCCCGTGGTGGAAT
CCAAGTGGCGGACCCTCGAAGCCTTCTGGGCGAAGCATATGTGGAATTTCATCAG
CGGGATACAATATTTAGCAGGCTTGTCCACTCTGCCTGGCAACCCCGCGATAGCA
TCACTGATGGCATTCACAGCCTCTATCACCAGCCCGCTCACCACCCAACATACCC
TCCTGTTTAACATCCTGGGGGGATGGGTGGCCGCCCAACTTGCTCCTCCCAGCGC

TGCTTCTGCTTTCGTAGGCGCCGGCATCGCTGGAGCGGCTGTTGGCAGCATAGGC
CTTGGGAAGGTGCTTGTGGATATTTTGGCAGGTTATGGAGCAGGGGTGGCAGGCG
CGCTCGTGGCCTTTAAGGTCATGAGCGGCGAGATGCCCTCCACCGAGGACCTGGT
TAACCTACTCCCTGCTATCCTCTCCCCTGGCGCCCTAGTCGTCGGGGTCGTGTGCG
CAGCGATACTGCGTCGGCACGTGGGCCCAGGGGAGGGGGCTGTGCAGTGGATGA
ACCGGCTGATAGCGTTCGCTTCGCGGGGTAACCACGTCTCCCCCACGCACTATGT
GCCTGAGAGCGACGCTGCAGCACGTGTCACTCAGATCCTCTCTAGTCTTACCATC
ACTCAGCTGCTGAAGAGGCTTCACCAGTGGATCAACGAGGACTGCTCCACGCCAT
GCTCCGGCTCGTGGCTAAGAGATGTTTGGGATTGGATATGCACGGTGTTGACTGA
TTTCAAGACCTGGCTCCAGTCCAAGCTCCTGCCGCGATTGCCGGGAGTCCCCTTCT
TCTCATGTCAACGTGGGTACAAGGGAGTCTGGCGGGGCGACGGCATCATGCAAA
CCACCTGCCCATGTGGAGCACAGATCACCGGACATGTGAAAAACGGTTCCATGA
GGATCGTGGGGCCTAGGACCTGTAGTAACACGTGGCATGGAACATTCCCCATTAA
CGCGTACACCACGGGCCCCTGCACGCCCTCCCCGGCGCCAAATTATTCTAGGGCG
CTGTGGCGGGTGGCTGCTGAGGAGTACGTGGAGGTTACGCGGGTGGGGGATTTC
CACTACGTGACGGGCATGACCACTGACAACGTAAAGTGCCCGTGTCAGGTTCCGG
CCCCCGAATTCTTCACAGAAGTGGATGGGGTGCGGTTGCACAGGTACGCTCCAGC
GTGCAAACCCCTCCTACGGGAGGAGGTCACATTCCTGGTCGGGCTCAATCAATAC
CTGGTTGGGTCACAGCTCCCATGCGAGCCCGAACCGGACGTAGCAGTGCTCACTT
CCATGCTCACCGACCCCTCCCACATTACGGCGGAGACGGCTAAGCGTAGGCTGGC
CAGGGGATCTCCCCCCTCCTTGGCCAGCTCATCAGCTATCCAGCTGTCTGCGCCTT
CCTTGAAGGCAACATGCACTACCCGTCATGACTCCCCGGACGCTGACCTCATCGA
GGCCAACCTCCTGTGGCGGCAGGAGATGGGCGGGAACATCACCCGCGTGGAGTC
AGAAAATAAGGTAGTAATTTTGGACTCTTTCGAGCCGCTCCAAGCGGAGGAGGA
TGAGAGGGAAGTATCCGTTCCGGCGGAGATCCTGCGGAGGTCCAGGAAATTCCC
TCGAGCGATGCCCATATGGGCACGCCCGGATTACAACCCTCCACTGTTAGAGTCC
TGGAAGGACCCGGACTACGTCCCTCCAGTGGTACACGGGTGTCCATTGCCGCCTG
CCAAGGCCCCTCCGATACCACCTCCACGGAGGAAGAGGACGGTTGTCCTGTCAG
AATCTACCGTGTCTTCTGCCTTGGCGGAGCTCGCCACAAAGACCTTCGGCAGCTC
CGAATCGTCGGCCGTCGACAGCGGCACGGCAACGGCCTCTCCTGACCAGCCCTCC
GACGACGGCGACGCGGGATCCGACGTTGAGTCGTACTCCTCCATGCCCCCCCTTG
AGGGGGAGCCGGGGGATCCCGATCTCAGCGACGGGTCTTGGTCTACCGTAAGCG
AGGAGGCTAGTGAGGACGTCGTCTGCTGCTCGATGTCCTACACATGGACAGGCGC
CCTGATCACGCCATGCGCTGCGGAGGAAACCAAGCTGCCCATCAATGCACTGAG
CAACTCTTTGCTCCGTCACCACAACTTGGTCTATGCTACAACATCTCGCAGCGCA
AGCCTGCGGCAGAAGAAGGTCACCTTTGACAGACTGCAGGTCCTGGACGACCAC
TACCGGGACGTGCTCAAGGAGATGAAGGCGAAGGCGTCCACAGTTAAGGCTAAA
CTTCTATCCGTGGAGGAAGCCTGTAAGCTGACGCCCCCACATTCGGCCAGATCTA
AATTTGGCTATGGGGCAAAGGACGTCCGGAACCTATCCAGCAAGGCCGTTAACC
ACATCCGCTCCGTGTGGAAGGACTTGCTGGAAGACACTGAGACACCAATTGACA
CCACCATCATGGCAAAAAATGAGGTTTTCTGCGTCCAACCAGAGAAGGGGGGCC
GCAAGCCAGCTCGCCTTATCGTATTCCCAGATTTGGGGGTTCGTGTGTGCGAGAA
AATGGCCCTTTACGATGTGGTCTCCACCCTCCCTCAGGCCGTGATGGGCTCTTCAT
ACGGATTCCAATACTCTCCTGGACAGCGGGTCGAGTTCCTGGTGAATGCCTGGAA
AGCGAAGAAATGCCCTATGGGCTTCGCATATGACACCCGCTGTTTTGACTCAACG
GTCACTGAGAATGACATCCGTGTTGAGGAGTCAATCTACCAATGTTGTGACTTGG
CCCCCGAAGCCAGACAGGCCATAAGGTCGCTCACAGAGCGGCTTTACATCGGGG
GCCCCCTGACTAATTCTAAAGGGCAGAACTGCGGCTATCGCCGGTGCCGCGCGAG
CGGTGTACTGACGACCAGCTGCGGTAATACCCTCACATGTTACTTGAAGGCCGCT
GCGGCCTGTCGAGCTGCGAAGCTCCAGGACTGCACGATGCTCGTATGCGGAGAC
GACCTTGTCGTTATCTGTGAAAGCGCGGGGACCCAAGAGGACGAGGCGAGCCTA
CGGGCCTTCACGGAGGCTATGACTAGATACTCTGCCCCCCCTGGGGACCCGCCCA
AACCAGAATACGACTTGGAGTTGATAACATCATGCTCCTCCAATGTGTCAGTCGC

GCACGATGCATCTGGCAAAAGGGTGTACTATCTCACCCGTGACCCCACCACCCCC
CTTGCGCGGGCTGCGTGGGAGACAGCTAGACACACTCCAGTCAATTCCTGGCTAG
GCAACATCATCATGTATGCGCCCACCTTGTGGGCAAGGATGATCCTGATGACTCA
TTTCTTCTCCATCCTTCTAGCTCAGGAACAACTTGAAAAAGCCCTAGATTGTCAGA
TCTACGGGGCCTGTTACTCCATTGAGCCACTTGACCTACCTCAGATCATTCAACG
ACTCCATGGCCTTAGCGCATTTTCACTCCATAGTTACTCTCCAGGTGAGATCAATA
GGGTGGCTTCATGCCTCAGGAAACTTGGGGTACCGCCCTTGCGAGTCTGGAGACA
TCGGGCCAGAAGTGTCCGCGCTAGGCTACTGTCCCAGGGGGGGAGGGCTGCCAC
TTGTGGCAAGTACCTCTTCAACTGGGCAGTAAGGACCAAGCTCAAACTCACTCCA
ATCCCGGCTGCGTCCCAGTTGGATTTATCCAGCTGGTTCGTTGCTGGTTACAGCGG
GGGAGACATATATCACAGCCTGTCTCGTGCCCGACCCCGCTGGTTCATGTGGTGC
CTACTCCTACTTTCTGTAGGGGTAGGCATCTATCTACTCCCCAACCGATGAACGG
GGACCTAAACACTCCAGGCCAATAGGCCATCCTGTTTTTTTCCCTTTTTTTTTTTCT
TTTTTTTTTTTTTTTTTTTTTTTTTTTTTTTTTTCTCCTTTTTTTTTCCTCTTTTTTTCCTT
TTCTTTCCTTTGGTGGCTCCATCTTAGCCCTAGTCACGGCTAGCTGTGAAAGGTCC
GTGAGCCGCTTGACTGCAGAGAGTGCTGATACTGGCCTCTCTGCAGATCAAGT

SEQ ID NO:25: Nucleotide sequence of DNA clone of HCV adaptive replicon 5'NTR-EMCV/HCVrepVII

GCCAGCCCCCGATTGGGGGCGACACTCCACCATAGATCACTCCCCTGTGAGGAAC
TACTGTCTTCACGCAGAAAGCGTCTAGCCATGGCGTTAGTATGAGTGTCGTGCAG
CCTCCAGGACCCCCCCTCCCGGGAGAGCCATAGTGGTCTGCGGAACCGGTGAGTA
CACCGGAATTGCCAGGACGACCGGGTCCTTTCTTGGATCAACCCGCTCAATGCCT
GGAGATTTGGGCGTGCCCCCGCGAGACTGCTAGCCGAGTAGTGTTGGGTCGCGA
AAGGCCTTGTGGTACTGCCTGATAGGGTGCTTGCGAGTGCCCCGGGAGGTCTCGT
AGACCGTGCACCAGACCACAACGGTTTCCCTCTAGCGGATCAATTCCGCCCCTC
TCCCTCCCCCCCCCCTAACGTTACTGGCCGAAGCCGCTTGGAATAAGGCCGGTGT
GCGTTTGTCTATATGTTATTTTCCACCATATTGCCGTCTTTTGGCAATGTGAGGGC
CCGGAAACCTGGCCCTGTCTTCTTGACGAGCATTCCTAGGGGTCTTTCCCCTCTCG
CCAAAGGAATGCAAGGTCTGTTGAATGTCGTGAAGGAAGCAGTTCCTCTGGAAG
CTTCTTGAAGACAAACAACGTCTGTAGCGACCCTTTGCAGGCAGCGGAACCCCCC
ACCTGGCGACAGGTGCCTCTGCGGCCAAAAGCCACGTGTATAAGATACACCTGC
AAAGGCGGCACAACCCCAGTGCCACGTTGTGAGTTGGATAGTTGTGGAAAGAGT
CAAATGGCTCTCCTCAAGCGTATTCAACAAGGGGCTGAAGGATGCCCAGAAGGT
ACCCCATTGTATGGGATCTGATCTGGGGCCTCGGTGCACATGCTTTACATGTGTTT
AGTCGAGGTTAAAAAACGTCTAGGCCCCCCGAACCACGGGGACGTGGTTTTCCTT
TGAAAAACACGATAATACCATGGCGCCTATTACGGCCTACTCCCAACAGACGCG
AGGCCTACTTGGCTGCATCATCACTAGCCTCACAGGCCGGGACAGGAACCAGGTC
GAGGGGGAGGTCCAAGTGGTCTCCACCGCAACACAATCTTTCCTGGCGACCTGCG
TCAATGGCGTGTGTTGGACTGTCTATCATGGTGCCGGCTCAAAGACCCTTGCCGG
CCCAAAGGGCCCAATCACCCAAATGTACACCAATGTGGACCAGGACCTCGTCGG
CTGGCAAGCGCCCCCCGGGGCGCGTTCCTTGACACCATGCACCTGCGGCAGCTCG
GACCTTTACTTGGTCACGAGGCATGCCGATGTCATTCCGGTGCGCCGGCGGGGCG
ACAGCAGGGGGAGCCTACTCTCCCCCAGGCCCGTCTCCTACTTGAAGGGCTCTTC
GGGCGGTCCACTGCTCTGCCCCTCGGGGCACGCTGTGGGCATCTTTCGGGCTGCC
GTGTGCACCCGAGGGGTTGCGAAGGCGGTGGACTTTGTACCCGTCGAGTCTATGG
AAACCACTATGCGGTCCCCGGTCTTCACGGACAACTCGTCCCCTCCGGCCGTACC
GCAGACATTCCAGGTGGCCCATCTACACGCCCCTACTGGTAGCGGCAAGAGCACT
AAGGTGCCGGCTGCGTATGCAGCCCAAGGGTATAAGGTGCTTGTCCTGAACCCGT
CCGTCGCCGCCACCCTAGGTTTCGGGGCGTATATGTCTAAGGCACATGGTATCGA
CCCTAACATCAGAACCGGGGTAAGGACCATCACCACGGGTGCCCCCATCACGTA
CTCCACCTATGGCAAGTTTCTTGCCGACGGTGGTTGCTCTGGGGGCGCCTATGAC

ATCATAATATGTGATGAGTGCCACTCAACTGACTCGACCACTATCCTGGGCATCG
GCACAGTCCTGGACCAAGCGGAGACGGCTGGAGCGCGACTCGTCGTGCTCGCCA
CCGCTACGCCTCCGGGATCGGTCACCGTGCCACATCCAAACATCGAGGAGGTGGC
TCTGTCCAGCACTGGAGAAATCCCCTTTATGGCAAAGCCATCCCCATCGAGACC
ATCAAGGGGGGGAGGCACCTCATTTCTGCCATTCCAAGAAGAAATGTGATGAG
CTCGCCGCGAAGCTGTCCGGCCTCGGACTCAATGCTGTAGCATATTACCGGGGCC
TTGATGTATCCGTCATACCAACTAGCGGAGACGTCATTGTCGTAGCAACGGACGC
TCTAATGACGGGCTTTACCGGCGATTTCGACTCAGTGATCGACTGCAATACATGT
GTCACCCAGACAGTCGACTTCAGCCTGGACCCGACCTTCACCATTGAGACGACGA
CCGTGCCACAAGACGCGGTGTCACGCTCGCAGCGGCGAGGCAGGACTGGTAGGG
GCAGGATGGGCATTTACAGGTTTGTGACTCCAGGAGAACGGCCCTCGGGCATGTT
CGATTCCTCGGTTCTGTGCGAGTGCTATGACGCGGGCTGTGCTTGGTACGAGCTC
ACGCCCGCCGAGACCTCAGTTAGGTTGCGGGCTTACCTAAACACACCAGGGTTGC
CCGTCTGCCAGGACCATCTGGAGTTCTGGGAGAGCGTCTTTACAGGCCTCACCCA
CATAGACGCCCATTTCTTGTCCCAGACTAAGCAGGCAGGAGACAACTTCCCCTAC
CTGGTAGCATACCAGGCTACGGTGTGCGCCAGGGCTCAGGCTCCACCTCCATCGT
GGGACCAAATGTGGAAGTGTCTCATACGGCTAAAGCCTACGCTGCACGGGCCAA
CGCCCCTGCTGTATAGGCTGGGAGCCGTTCAAAACGAGGTTACTACCACACACCC
CATAACCAAATACATCATGGCATGCATGTCGGCTGACCTGGAGGTCGTCACGAGC
ACCTGGGTGCTGGTAGGCGGAGTCCTAGCAGCTCTGGCCGCGTATTGCCTGACAA
CAGGCAGCGTGGTCATTGTGGGCAGGATCATCTTGTCCGGAAAGCCGGCCATCAT
TCCCGACAGGGAAGTCCTTTACCGGGAGTTCGATGAGATGGAAGAGTGCGCCTC
ACACCTCCCTTACATCGAACAGGGAATGCAGCTCGCCGAACAATTCAAACAGAA
GGCAATCGGGTTGCTGCAAACAGCCACCAAGCAAGCGGAGGCTGCTGCTCCCGT
GGTGGAATCCAAGTGGCGGACCCTCGAAGCCTTCTGGGCGAAGCATATGTGGAA
TTTCATCAGCGGGATACAATATTTAGCAGGCTTGTCCACTCTGCCTGGCAACCCC
GCGATAGCATCACTGATGGCATTCACAGCCTCTATCACCAGCCCGCTCACCACCC
AACATACCCTCCTGTTTAACATCCTGGGGGGATGGGTGGCCGCCCAACTTGCTCC
TCCCAGCGCTGCTTCTGCTTTCGTAGGCGCCGGCATCGCTGGAGCGGCTGTTGGC
AGCATAGGCCTTGGGAAGGTGCTTGTGGATATTTTGGCAGGTTATGGAGCAGGGG
TGGCAGGCGCGCTCGTGGCCTTTAAGGTCATGAGCGGCGAGATGCCCTCCACCGA
GGACCTGGTTAACCTACTCCCTGCTATCCTCTCCCCTGGCGCCCTAGTCGTCGGGG
TCGTGTGCGCAGCGATACTGCGTCGGCACGTGGGCCCAGGGGAGGGGGCTGTGC
AGTGGATGAACCGGCTGATAGCGTTCGCTTCGCGGGGTAACCACGTCTCCCCCAC
GCACTATGTGCCTGAGAGCGACGCTGCAGCACGTGTCACTCAGATCCTCTCTAGT
CTTACCATCACTCAGCTGCTGAAGAGGCTTCACCAGTGGATCAACGAGGACTGCT
CCACGCCATGCTCCGGCTCGTGGCTAAGAGATGTTTGGGATTGGATATGCACGGT
GTTGACTGATTTCAAGACCTGGCTCCAGTCCAAGCTCCTGCCGCGATTGCCGGGA
GTCCCCTTCTTCTCATGTCAACGTGGGTACAAGGGAGTCTGGCGGGGCGACGGCA
TCATGCAAACCACCTGCCCATGTGGAGCACAGATCACCGGACATGTGAAAAACG
GTTCCATGAGGATCGTGGGGCCTAGGACCTGTAGTAACACGTGGCATGGAACATT
CCCCATTAACGCGTACACCACGGGCCCCTGCACGCCCTCCCCGGCGCCAAATTAT
TCTAGGGCGCTGTGGCGGGTGGCTGCTGAGGAGTACGTGGAGGTTACGCGGGTG
GGGGATTTCCACTACGTGACGGGCATGACCACTGACAACGTAAAGTGCCCGTGTC
AGGTTCCGGCCCCCGAATTCTTCACAGAAGTGGATGGGGTGCGGTTGCACAGGTA
CGCTCCAGCGTGCAAACCCCTCCTACGGGAGGAGGTCACATTCCTGGTCGGGCTC
AATCAATACCTGGTTGGGTCACAGCTCCCATGCGAGCCCGAACCGGACGTAGCA
GTGCTCACTTCCATGCTCACCGACCCCTCCCACATTACGGCGGAGACGGCTAAGC
GTAGGCTGGCCAGGGGATCTCCCCCCTCCTTGGCCAGCTCATCAGCTATCCAGCT
GTCTGCGCCTTCCTTGAAGGCAACATGCACTACCCGTCATGACTCCCCGGACGCT
GACCTCATCGAGGCCAACCTCCTGTGGCGGCAGGAGATGGGCGGGAACATCACC
CGCGTGGAGTCAGAAAATAAGGTAGTAATTTTGGACTCTTTCGAGCCGCTCCAAG
CGGAGGAGGATGAGAGGGAAGTATCCGTTCCGGCGGAGATCCTGCGGAGGTCCA

GGAAATTCCCTCGAGCGATGCCCATATGGGCACGCCCGGATTACAACCCTCCACT
GTTAGAGTCCTGGAAGGACCCGGACTACGTCCCTCCAGTGGTACACGGGTGTCCA
TTGCCGCCTGCCAAGGCCCCTCCGATACCACCTCCACGGAGGAAGAGGACGGTTG
TCCTGTCAGAATCTACCGTGTCTTCTGCCTTGGCGGAGCTCGCCACAAAGACCTTC
GGCAGCTCCGAATCGTCGGCCGTCGACAGCGGCACGGCAACGGCCTCTCCTGACC
AGCCCTCCGACGACGGCGACGCGGGATCCGACGTTGAGTCGTACTCCTCCATGCC
CCCCCTTGAGGGGGAGCCGGGGGATCCCGATCTCAGCGACGGGTCTTGGTCTACC
GTAAGCGAGGAGGCTAGTGAGGACGTCGTCTGCTGCTCGATGTCCTACACATGGA
CAGGCGCCCTGATCACGCCATGCGCTGCGGAGGAAACCAAGCTGCCCATCAATG
CACTGAGCAACTCTTTGCTCCGTCACCACAACTTGGTCTATGCTACAACATCTCGC
AGCGCAAGCCTGCGGCAGAAGAAGGTCACCTTTGACAGACTGCAGGTCCTGGAC
GACCACTACCGGGACGTGCTCAAGGAGATGAAGGCGAAGGCGTCCACAGTTAAG
GCTAAACTTCTATCCGTGGAGGAAGCCTGTAAGCTGACGCCCCCACATTCGGCCA
GATCTAAATTTGGCTATGGGGCAAAGGACGTCCGGAACCTATCCAGCAAGGCCG
TTAACCACATCCGCTCCGTGTGGAAGGACTTGCTGGAAGACACTGAGACACCAAT
TGACACCACCATCATGGCAAAAAATGAGGTTTTCTGCGTCCAACCAGAGAAGGG
GGGCCGCAAGCCAGCTCGCCTTATCGTATTCCCAGATTTGGGGGTTCGTGTGTGC
GAGAAAATGGCCCTTTACGATGTGGTCTCCACCCTCCCTCAGGCCGTGATGGGCT
CTTCATACGGATTCCAATACTCTCCTGGACAGCGGGTCGAGTTCCTGGTGAATGC
CTGGAAAGCGAAGAAATGCCCTATGGGCTTCGCATATGACACCCGCTGTTTTGAC
TCAACGGTCACTGAGAATGACATCCGTGTTGAGGAGTCAATCTACCAATGTTGTG
ACTTGGCCCCCGAAGCCAGACAGGCCATAAGGTCGCTCACAGAGCGGCTTTACAT
CGGGGGCCCCCTGACTAATTCTAAAGGGCAGAACTGCGGCTATCGCCGGTGCCGC
GCGAGCGGTGTACTGACGACCAGCTGCGGTAATACCCTCACATGTTACTTGAAGG
CCGCTGCGGCCTGTCGAGCTGCGAAGCTCCAGGACTGCACGATGCTCGTATGCGG
AGACGACCTTGTCGTTATCTGTGAAAGCGCGGGGACCCAAGAGGACGAGGCGAG
CCTACGGGCCTTCACGGAGGCTATGACTAGATACTCTGCCCCCCCTGGGGACCCG
CCCAAACCAGAATACGACTTGGAGTTGATAACATCATGCTCCTCCAATGTGTCAG
TCGCGCACGATGCATCTGGCAAAAGGGTGTACTATCTCACCCGTGACCCCACCAC
CCCCCTTGCGCGGGCTGCGTGGGAGACAGCTAGACACACTCCAGTCAATTCCTGG
CTAGGCAACATCATCATGTATGCGCCCACCTTGTGGGCAAGGATGATCCTGATGA
CTCATTTCTTCTCCATCCTTCTAGCTCAGGAACAACTTGAAAAAGCCCTAGATTGT
CAGATCTACGGGGCCTGTTACTCCATTGAGCCACTTGACCTACCTCAGATCATTC
AACGACTCCATGGCCTTAGCGCATTTTCACTCCATAGTTACTCTCCAGGTGAGATC
AATAGGGTGGCTTCATGCCTCAGGAAACTTGGGGTACCGCCCTTGCGAGTCTGGA
GACATCGGGCCAGAAGTGTCCGCGCTAGGCTACTGTCCCAGGGGGGGAGGGCTG
CCACTTGTGGCAAGTACCTCTTCAACTGGGCAGTAAGGACCAAGCTCAAACTCAC
TCCAATCCCGGCTGCGTCCCAGTTGGATTTATCCAGCTGGTTCGTTGCTGGTTACA
GCGGGGGAGACATATATCACAGCCTGTCTCGTGCCCGACCCCGCTGGTTCATGTG
GTGCCTACTCCTACTTTCTGTAGGGGTAGGCATCTATCTACTCCCCAACCGATGAA
CGGGGACCTAAACACTCCAGGCCAATAGGCCATCCTGTTTTTTCCCTTTTTTTTT
TTCTTTTTTTTTTTTTTTTTTTTTTTTTTTTTTTTTCTCCTTTTTTTTTCCTCTTTTTTT
CCTTTTCTTTCCTTTGGTGGCTCCATCTTAGCCCTAGTCACGGCTAGCTGTGAAAG
GTCCGTGAGCCGCTTGACTGCAGAGAGTGCTGATACTGGCCTCTCTGCAGATCAA
GT

## Claims

1. A polynucleotide comprising a non-naturally occurring HCV sequence that is capable of productive replication in a host cell, or is capable of being transcribed into a non-naturally occurring HCV sequence that is capable of productive replication in a host cell, wherein the HCV sequence comprises, from 5' to 3' on the positive-sense nucleic acid, a functional 5' non-translated region (5' NTR); one or more protein coding regions, including at least one polyprotein coding region that is capable of replicating HCV RNA; and a functional HCV 3' non-translated region (3' NTR);
wherein the polyprotein coding region comprises an NS5A gene comprising an adaptive mutation (i) encoding an amino acid sequence change selected from the group consisting of Ser (1179) to Ile, Arg (1164) to Gly, Ala (1174) to Ser, Ser (1172) to Cys, Ser (1172) to Pro, of SEQ ID NO:3 or (ii) comprising a deletion of nucleotides corresponding to nucleotides 5345 to 5485 of SEQ ID NO:6.

2. The polynucleotide of claim 1, having a transfection efficiency into mammalian cells of greater than 0.01%.

3. The polynucleotide of claim 1, having a transfection efficiency into mammalian cells of greater than 0.1%.

4. The polynucleotide of claim 1, having a transfection efficiency into mammalian cells of greater than 1%.

5. The polynucleotide of claim 1, having a transfection efficiency into mammalian cells of about 6%.

6. The polynucleotide of claim 1, wherein the polynucleotide is capable of replication in a non-hepatic cell.

7. The polynucleotide of claim 6, wherein the non-hepatic cell is a HeLa cell.

8. The polynucleotide of claim 1, wherein the HCV is impaired in its ability to cause disease, establish chronic infections, trigger autoimmune responses, and transform cells.

9. The polynucleotide of claim 1, wherein the polynucleotide comprises at least one IRES selected from the group consisting of a viral IRES, a cellular IRES, and an artificial IRES.

10. The polynucleotide of claim 9, wherein the HCV polyprotein coding region encodes all HCV structural and nonstructural proteins.

11. The polynucleotide of claim 9, wherein the polyprotein coding region is incapable of making infectious HCV particles.

12. The polynucleotide of claim 11, wherein the polyprotein coding region comprises a mutation and/or a deletion in the structural protein coding region.

13. The polynucleotide of claim 10 or claim 12, further comprising a foreign gene operably linked to a first IRES and the HCV polyprotein coding region operably linked to a second IRES.

14. The polynucleotide of claim 13, wherein the foreign gene is a gene encoding a selectable marker or a reporter gene.

15. The polynucleotide of claim 14, wherein:
(a) the first IRES is an HCV IRES;
(b) the foreign gene is a neo gene; and
(c) the second IRES is a EMCV IRES.

16. The polynucleotide of claim 15, wherein the HCV sequence is a genotype 1 HCV sequence.

17. The polynucleotide of claim 16, wherein the HCV sequence is subtype 1b.

18. The polynucleotide of claim 15, comprising SEQ ID NO:5 or SEQ ID NO:6.

19. The polynucleotide of any of claims 1 to 18 wherein the polynucleotide is double-stranded DNA.

20. A vector comprising the polynucleotide of claim 19 operably associated with a promoter.

21. A cell comprising the vector of claim 20.

22. A host cell comprising the polynucleotide of any of claims 1 to 18, wherein the host cell is a mammalian cell.

23. The host cell of claim 22 wherein the host cell is a human cell.

24. The host cell of claim 23 wherein the host cell is selected from the group consisting of a liver cell, a T-cell, a B-cell and a HeLa cell.

25. The host cell of claim 24 wherein the host cell is a HeLa cell.

## Patentansprüche

1. Polynucleotid, das eine nicht natürlich vorkommende HCV-Sequenz umfasst, die zu produktiver Replikation in einer Wirtszelle in der Lage ist, oder das in eine nicht natürlich vorkommende HCV-Sequenz transkribierbar ist, die zu produktiver Replikation in einer Wirtszelle in der Lage ist, wobei die HCV-Sequenz 5'→3' auf der positivsträngigen Nucleinsäure eine funktionelle nichttranslatierte 5'-Region (5'-NTR); eine oder mehrere für ein Protein kodierende Regionen, die zumindest eine für ein Polyprotein kodierende Region umfassen, die zur Replikation von HCV-RNA in der Lage ist; und eine funktionelle nichttranslatierte 3'-HCV-Region (3'-NTR) umfasst; wobei die für ein Polyprotein kodierende Region ein NS5A-Gen umfasst, das eine adaptive Mutation umfasst, die (i) für eine aus der aus Ser (1179) zu Ile, Arg (1164) zu Gly, Ala (1174) zu Ser, Ser (1172) zu Cys und Ser (1172) zu Pro von Seq.-ID Nr. 3 bestehenden Gruppe ausgewählte Aminosäuresequenzänderung kodiert, oder (ii) eine Deletion von Nucleotiden umfasst, die den Nucleotiden 5345 bis 5485 von Seq.-ID Nr. 6 entsprechen.

2. Polynucleotid nach Anspruch 1, das eine Transfektionseffizienz in Säugetierzellen von mehr als 0,01 % aufweist.

3. Polynucleotid nach Anspruch 1, das eine Transfektionseffizienz in Säugetierzellen von mehr als 0,1 % aufweist.

4. Polynucleotid nach Anspruch 1, das eine Transfektionseffizienz in Säugetierzellen von mehr als 1 % aufweist.

5. Polynucleotid nach Anspruch 1, das eine Transfektionseffizienz in Säugetierzellen von etwa 6 % aufweist.

6. Polynucleotid nach Anspruch 1, wobei das Polynucleotid zur Replikation in einer nichthepatischen Zelle in der Lage ist.

7. Polynucleotid nach Anspruch 6, wobei die nichthepatische Zelle eine HeLa-Zelle ist.

8. Polynucleotid nach Anspruch 1, wobei das HCV in seiner Fähigkeit geschwächt ist, eine Krankheit zu verursachen, zu chronischen Infektionen zu führen, Autoimmunreaktionen auszulösen und Zellen zu transformieren.

9. Polynucleotid nach Anspruch 1, wobei das Polynucleotid zumindest eine IRES umfasst, die aus der aus einer viralen IRES, einer zellulären IRES und einer künstlichen IRES bestehenden Gruppe ausgewählt ist.

10. Polynucleotid nach Anspruch 9, wobei die für ein HCV-Polyprotein kodierende Region für alle HCV-Strukturproteine und -Nichtstrukturproteine kodiert.

11. Polynucleotid nach Anspruch 9, wobei die für ein Polyprotein kodierende Region nicht in der Lage ist, infektiöse HCV-Partikel zu produzieren.

12. Polynucleotid nach Anspruch 11, wobei die für ein Polyprotein kodierende Region eine Mutation und/oder Deletion in der für Strukturproteine kodierenden Region umfasst.

13. Polynucleotid nach Anspruch 10 oder Anspruch 13, das weiters ein fremdes Gen operabel an eine erste IRES gebunden und die für ein HCV-Polyprotein kodierende Region operabel an eine zweite IRES gebunden umfasst.

14. Polynucleotid nach Anspruch 13, wobei das fremde Gen ein Gen ist, das für einen selektierbaren Marker oder ein Reportergen kodiert.

15. Polynucleotid nach Anspruch 14, wobei:
(a) die erste IRES eine HCV-IRES ist,
(b) das fremde Gen ein neo-Gen ist und
(c) die zweite IRES eine EMCV-IRES ist.

16. Polynucleotid nach Anspruch 15, wobei die HCV-Sequenz eine HCV-Sequenz vom Genotyp 1 ist.

17. Polynucleotid nach Anspruch 16, wobei die HCV-Sequenz vom Subtyp 1 b ist.

18. Polynucleotid nach Anspruch 15, das Seq.-ID Nr. 5 oder Seq.-ID Nr. 6 umfasst.

19. Polynucleotid nach einem der Ansprüche 1 bis 18, wobei das Polynucleotid doppelsträngige DNA ist.

20. Vektor, der ein Polynucleotid nach Anspruch 19 operabel mit einem Promotor assoziiert umfasst.

21. Zelle, die einen Vektor nach Anspruch 20 umfasst.

22. Wirtszelle, die ein Polynucleotid nach einem der Ansprüche 1 bis 18 umfasst, wobei die Wirtszelle eine Säugetierzelle ist.

23. Wirtszelle nach Anspruch 22, wobei die Wirtszelle eine menschliche Zelle ist.

24. Wirtszelle nach Anspruch 23, wobei die Wirtszelle aus der aus einer Leberzelle, einer T-Zelle, einer B-Zelle und einer HeLa-Zelle bestehenden Gruppe ausgewählt ist.

25. Wirtszelle nach Anspruch 24, wobei die Wirtszelle eine HeLa-Zelle ist.

## Revendications

1. Polynucléotide comprenant une séquence HCV se produisant non-naturellement qui convient pour une réplication productive dans une cellule hôte, ou qui peut être transcrite en une séquence HCV se produisant non-naturellement qui convient pour la réplication productive dans une cellule hôte, où la séquence HCV comprend, de 5' à 3' sur l'acide nucléique dans le sens positif, une région fonctionnelle 5' non-translatée (5' NTR); une ou plusieurs régions de codage de protéine, incluant au moins une région de codage de polyprotéine qui est apte à répliquer HCV ARN; et une région fonctionnelle HCV 3' non-translatée (3' NTR); où la région de codage de polyprotéine comprend un gène NS5A comprenant une mutation adaptive (i) codant pour un changement de séquence d'acides aminés sélectionnée dans le groupe consistant en Ser (1179) à Ile, Arg (1164) à Gly, Ala (1174) à Ser, Ser (1172) à Pro, en SEQ ID NO:3 ou (ii) comprenant une délétion de nucléotides correspondant aux nucléotides 5345 à 5485 de la SEQ ID NO:6.

2. Polynucléotide selon la revendication 1, ayant une efficience de transfection dans des cellules mammaliennes supérieure à 0,01%.

3. Polynucléotide selon la revendication 1, ayant une efficience de transfection dans des cellules mammaliennes supérieure à 0,1%.

4. Polynucléotide selon la revendication 1, ayant une efficience de transfection dans des cellules mammaliennes supérieure à 1%.

5. Polynucléotide selon la revendication 1, ayant une efficience de transfection dans des cellules mammaliennes d'environ 6%.

6. Polynucléotide selon la revendication 1, où le polynucléotide convient pour la réplication dans une cellule non-hépatique.

7. Polynucléotide selon la revendication 6, où la cellule non-hépatique est une cellule HeLa.

8. Polynucléotide selon la revendication 1, où le HCV est détérioré dans son aptitude à provoquer un trouble, établir des infections chroniques, déclencher des réponses auto-immunes et transformer des cellules.

9. Polynucléotide selon la revendication 1, où le polynucléotide comprend au moins une IRES sélectionnée dans le groupe consistant en une IRES virale, une IRES cellulaire et une IRES artificielle.

10. Polynucléotide selon la revendication 9, où la région de codage de la polyprotéine HCV code toutes les protéines HCV structurelles et non structurelles.

11. Polynucléotide selon la revendication 9, où la région de codage de polyprotéine est inapte à réaliser des particules HCV infectieuses.

12. Polynucléotide selon la revendication 11, où la région de codage de polyprotéine comprend une mutation et/ou une délétion dans la région de codage de protéine structurelle.

13. Polynucléotide selon la revendication 10 ou la revendication 12, comprenant en outre un gène étranger fonctionnellement lié à une première IRES, et la région de codage de polyprotéine HCV est fonctionnellement liée à une deuxième IRES.

14. Polynucléotide selon la revendication 13, où le gène étranger est un gène codant pour un marqueur ou un gène reporter pouvant être sélectionné.

15. Polynucléotide selon la revendication 14, où:
(a) la première IRES est une HCV IRES;
(b) le gène étranger est un néo gène; et
(c) la deuxième IRES est une EMCV IRES.

16. Polynucléotide selon la revendication 15, où la séquence HCV est une séquence HCV de génotype 1.

17. Polynucléotide selon la revendication 16, où la séquence HCV est un sous-type 1b.

18. Polynucléotide selon la revendication 15, comprenant la SEQ ID NO:5 ou la SEQ ID NO:6.

19. Polynucléotide selon l'une quelconque des revendications 1 à 18, où le polynucléotide est un ADN à brin double.

20. Vecteur comprenant le polynucléotide de la revendication 19, fonctionnellement associé à un promoteur.

21. Cellule comprenant le vecteur de la revendication 20.

22. Cellule hôte comprenant le polynucléotide selon l'une quelconque des revendications 1 à 18, où la cellule hôte est une cellule mammalienne.

23. Cellule hôte selon la revendication 22, où la cellule hôte est une cellule humaine.

24. Cellule hôte selon la revendication 23, où la cellule hôte est sélectionnée dans le groupe consistant en une cellule du foie, une cellule T, une cellule B et une cellule HeLa.

25. Cellule hôte selon la revendication 24, où la cellule hôte est une cellule HeLa.
